(19) Europäisches Patentamt
European Patent Office
Office européen des brevets

(11) EP 4 209 225 A1

(12) EUROPEAN PATENT APPLICATION
published in accordance with Art. 153(4) EPC

(43) Date of publication:
**12.07.2023 Bulletin 2023/28**

(21) Application number: **21863593.6**

(22) Date of filing: **31.08.2021**

(51) International Patent Classification (IPC):
***A61K 36/634*** (2006.01) ***A61K 36/25*** (2006.01)
***A61P 31/12*** (2006.01) ***A61P 29/00*** (2006.01)
***A61P 37/04*** (2006.01)

(52) Cooperative Patent Classification (CPC):
**A61K 36/25; A61K 36/634; A61P 29/00; A61P 31/12; A61P 37/04**

(86) International application number:
**PCT/CN2021/115584**

(87) International publication number:
**WO 2022/048529 (10.03.2022 Gazette 2022/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.09.2020 CN 202010923837**
**14.09.2020 CN 202010959266**

(71) Applicant: **Dalian Fusheng Natural Medicine Development Co., Ltd.**
**Dalian, Liaoning 116600 (CN)**

(72) Inventors:
- **WANG, Shuo**
  **Dalian, Liaoning 116600 (CN)**
- **FU, Li**
  **Dalian, Liaoning 116600 (CN)**
- **FENG, Xue**
  **Dalian, Liaoning 116600 (CN)**
- **LIU, Yang**
  **Dalian, Liaoning 116600 (CN)**
- **LIN, Rongxin**
  **Dalian, Liaoning 116600 (CN)**
- **HOU, Jirui**
  **Dalian, Liaoning 116600 (CN)**
- **LU, Mingming**
  **Dalian, Liaoning 116600 (CN)**
- **FU, Wenfei**
  **Dalian, Liaoning 116600 (CN)**
- **LU, Qi**
  **Dalian, Liaoning 116600 (CN)**
- **ZHOU, Qingfeng**
  **Dalian, Liaoning 116600 (CN)**

(74) Representative: **Murgitroyd & Company**
**Murgitroyd House**
**165-169 Scotland Street**
**Glasgow G5 8PL (GB)**

# (54) FORSYTHIAE FRUCTUS COMPONENT AND OPTIONAL GINSENG COMPONENT, AND USE THEREOF

(57) The present invention discloses a composition of forsythia suspensa component and an optional panax ginseng component that can be used safely, efficiently and stably to achieve the antiviral, antipyretic, anti-inflammatory and/or immunity boosting effects, including forsythin, forsythin derivatives, and the composition of forsythin and phillygenin for the prevention and treatment of diseases associated with human cytomegalovirus infection, such as simple human cytomegalovirus infection or patients infected by human cytomegalovirus during the prevention and treatment of cardiovascular and cerebrovascular diseases, tumors, burns or AIDS or during the process of organ transplantation or during the perinatal period. Wherein, the process for preparing the composition of forsythia suspensa and panax ginseng components is simple, suitable for industrial production and easy to popularize.

EP 4 209 225 A1

## Description

### Technical Field

The present invention belongs to the field of medicinal chemistry, health food products and medicine. To be specific, the present invention relates to a composition of forsythia suspensa extract (preferably also including panax ginseng extract), the preparation method, and its application in achieving the antiviral, antipyretic, anti-inflammatory and immunity boosting effects, including the application of forsythin, forsythin derivatives and the composition of forsythin and phillygenin in the manufacturing of health products and drugs for the prevention and treatment of diseases associated with human cytomegalovirus infection.

### Background Technologies

Human cytomegalovirus (HCMV) is also known as a cellular inclusion body virus because the infected cells become swollen and contain large intranuclear inclusion bodies. HCMV belongs to the beta-herpesvirus subfamily. With a diameter of approximately 200 nm, HCMV is one of the largest animal viruses. It has a core of 64 nm in diameter containing viral DNA, which is enclosed by a icosahydron with a diameter of 110 nm. The entire particle is enclosed by a capsule consisting of at least 25-30 proteins or glycoproteins encoded by the virus particles. The genome of HCMV is approximately 235-240 kb long with a molecular weight of 150-160 $\times$ $10^3$ kDa. HCMV is a linear double-stranded DNA virus. It is composed of two components or segments, the unique long (LTL) and unique short (US) regions. As the two components have different directions or are inversed at the junctions, the DNA molecules have four different isomers. HCMV is widely distributed so that animals can also be infected, which may cause infections of various systems, mainly the genitourinary system and central nervous system and liver diseases, and the severity ranges from mild asymptomatic infection to severe defects or even life-threatening.

The HCMV gene contains three genetic fragments encoding the infected cell-specific polypeptides, i.e., the immediate early (IE) protein, early (E) protein and late (L) protein. The three proteins have immunogenicity and are time-phase dependent. The transcription and translation of HCMV genes are regulated by themselves and the host cells. The expression of immediate early genes and early genes are regulated by the promoters. The detection of these two genes is mostly seen in acute infections. The transcription and translation of the late genes are regulated by IE genes, E genes and proteins. The detection of late genes indicates that HCMV is in the latent period and the viral assembly is mature, which is the consequence of HCMV infection. There are three morphological transformation regions in HCMV genome, namely the mtrI, mtr II and mtr III, which can induce cell transformation. MtrI is located on the E fragment of Hind III at the left end of the U genome of AD169 strain. It is 588 bp long and can transform the NIH 3T3 and primary mouse embryonic cells. Mtr II is located on the EM fragment of XbaI/Bam HI of the DNA of HCMV Towne strain. It is 980 bp long and can transform the NIH 3T3 and rat-2 cells. It has its corresponding regions on the AD169 and TdCMV Tanaka of HCMV strains and contains two isolated promoters, P1 and P2. Mtr II is present in all the transformed cells and the resulted tumor cells. Mtr III is located in the XbaI/BamHI fragment and encodes the major immediate early protein with a molecular weight of 72 kD, which is involved in the transactivation and self-regulation of the virus. Mtr III does not exist in the transformed cells.

At the cellular level, HCMV infections are classified into three types: 1) toxigenic infection; 2) latent infection; 3) cell transformation or latent carcinogenesis. In in vitro studies, after HCMVs were inoculated into human fibroblasts, cell strains containing HCMV antigens were formed, which had the characteristics of transformed cells. The specific fragments of HCMV-DNA were extracted and used to transfect cells, and some of the cells could be transformed by integrating the DNA into the chromosomal DNA of the cells, and the transformed cells were inoculated into athymic nude mice to produce tumors. In a study by Liu Chaoqi et al., the HPV DNA-immortalized human cervical epithelial cells constructed by the transfection using the HCMV IE were inoculated to the nude mice. The results showed that HCMVs were integrated into the chromosomes of the cells of the animals, acted synergistically with HPV16 to promote the malignant transformation of cervical epithelial cells, and formed tumors in the nude mice, indicating the close relationship between HCMV and cervical cancer. In a study conducted by Lu Deyin et al., ultraviolet inactivated HCMVs were inoculated into the uterine cervix of mice three times a week for 8 consecutive weeks. The results showed that the incidence rate of cervical precancerous lesions of the HCMV group was 27.8% (23/83), and that of cervical cancer was 20.5% (17/83).

CMV has the typical morphology of herpesvirus (HSV), and its DNA structure also resembles HSV, but it is 5% larger than the size of HSV Human cytomegalovirus (HCMV) is highly species-specific to the host or cultured cells. HCMV can only infect humans and proliferate in human fibroblasts. In cell culture, HCMV proliferates slowly and requires a long replication cycle. It generally takes 30 to 40 days to develop the cytopathic effect (CPE) during the initial isolation and culture. The lesions are characterized by the appearance of round and enlarged cells, enlarged nuclei, and a large eosinophilic inclusion body surrounded by a "halo" in the nuclei. The immune function of the cells in the human body

plays an important role in the onset and development of CMV infections. Cellular immune deficiency may lead to severe and long-term CMV infection, and further suppress the cellular immune function, such as decreases in the viability of killer T cells and the function of NK cells. After primary CMV infection, the body can produce specific antibodies and killer T lymphocytes, activating the NM cells. The antibody has a limited capacity to prevent the virus replication of CMV, and has a certain resistance to the reinfection by the same virus strain. However, it cannot resist the reactivation of endogenous latent viruses or exogenous infections caused by other different CMV strains. While the species specific killing T lymphocytes and antibody-dependent cytotoxic cells can exert the greatest antiviral effect.

HCMV infection is very common in the population, which can cause a variety of diseases. In adults, immunodeficient or immunosuppressed patients are mostly affected by the HCMV infections. Especially patients receiving immunosuppressive agents after organ or bone marrow transplantation and those with malignant tumors after receiving radiotherapy and chemotherapy are highly susceptible to HCMV infection. The incidence of HCMV infection in AIDS patients is also very high. Due to the high prevalence of HCMV infection and the potential serious consequences, great attention has been paid to HCMV infections all over the world, which is of great significance in exploring the epidemiology, diagnostic techniques, treatment and prevention of HCMV infections as well as the related basic research on the virology. After receiving chemotherapy or radiotherapy, the immune function of tumor patients is much lower than that of normal individuals, and it is easy for the latent human cytomegalovirus (HCMV) to be reactivated and cause serious infections. It is an important factor for infections that affect the survival rate and the quality of life of tumor patients.

CMV infection is a congenital or acquired infection caused by cytomegalovirus (CMV). CMV is a double-stranded DNA virus belonging to the subgroup of the herpes virus family. Its morphology is similar to that of other herpes viruses and has obvious species specificity to the host cells or cultured tissue cells. Human CMVs can only be isolated and cultured in human embryonic fibroblasts.

HCMV infection is widespread in the human population. The infection rate among adults in China is more than 95%, and the infection in adults mostly affects patients with immune deficiency or immunosuppression. Especially patients receiving immunosuppressive agents after organ or bone marrow transplantation and those with malignant tumors after receiving radiotherapy and chemotherapy are highly susceptible to HCMV infection. Most of the infected patients will present with no clinical symptoms, but the virus invasion into multiple organs and systems can cause severe diseases under certain conditions. HCMVs may invade the lung, liver, kidney, salivary gland, mammary gland and other glands, as well as multinucleated white blood cells and lymphocytes, and can be discharged from saliva, milk, blood, urine, semen, and uterine secretions for a long time or intermittently. Multiple routes, such as oral cavity, genital tract, placenta, blood transfusion or organ transplantation, usually transmit the virus.

1. Congenital infection: in pregnant women with HCMV infection, the virus can invade the fetus through the placenta and cause congenital infection, and in rare cases, which may result in premature delivery, abortion, stillbirth or death after birth. The neonates may develop jaundice, hepatosplenomegaly, thrombocytopenic purpura or hemolytic anemia. Most of the surviving neonates will have the sequelea of permanent mental retardation, neuromuscular dyskinesia, deafness and chorioretinitis.
2. Perinatal infection: when HCMV is discharged from the urinary tract and cervix of the puerpera, the infants can be infected through the birth canal during delivery. Most infants will develop subclinical infections with slight or no clinical symptoms, whereas some will have slight respiratory tract obstruction or liver injury.
3. Infections in children and adults: the virus can be transmitted through the routes of breast-sucking, kissing, sexual contact and blood transfusion, and the symptoms caused by the infections are usually subclinical. In some cases, it may lead to heterophil antibody negative infectious mononucleosis. The latent viruses in monocytes and lymphocytes can be activated due to factors including pregnancy, immunosuppressive therapy, organ transplantation and tumors, resulting in mononucleosis, hepatitis, interstitial pneumonia, retinitis, encephalitis, etc.
4. Cell transformation and carcinogenic potential: UV-inactivated HCMVs can transform the embryonic fibroblasts of rodents. In some tumors, such as cervical cancer, colon cancer, prostate cancer and Kaposis sarcoma, the detection rate of HCMV DNA was found high and the titer of HCMV antibody was also higher compared to normal individuals. In addition, the viral particles were also detected in the cell lines constructed from these tumors, suggesting that HCMV, like other herpes viruses, has carcinogenic potential. After receiving chemotherapy or radiotherapy, the immune function of tumor patients is much lower than that of normal individuals, and it is easy for the latent human cytomegalovirus (HCMV) to be reactivated and cause serious infections. It is an important factor for the infection that affects the survival rate and the quality of life of tumor patients.

Most patients with HCMV infection are in the latent infection state. So, even if HCMV is replicated in the body, it will cause asymptomatic infections in most cases. Currently, no anti-HCMV drugs are available that are both effective and safe. Therefore, the treatment for HCMV infection is still limited to symptomatic treatment. Ganciclovir can only be used with caution for symptomatic infections due to its toxic side effects, such as myelosuppression. Ganciclovir DHPG can prevent the spreading of HCMV infection. If it is co-administered with high titer anti-HCMV immunoglobulin, the

mortality of complications of HCMV pneumonia can be reduced in patients undergoing bone marrow transplantation. For HCMV-infected patients with resistance to ganciclovir DHPG, foscarnet sodium can be used. This drug can persistently reduce the spreading of HCMV infection, but with poor effect compared with ganciclovir. The anti-HCMV live vaccines developed in other countries can induce generations of antibodies, but whether the carcinogenic potential of the vaccine can be ruled out remains unknown.

Forsythia suspensa is a commonly used traditional Chinese medicine in clinical settings with wide application and huge consumption. It has the functions of clearing heat, detoxifying, subduing swellings, dissolving lumps, and scavenging free radicals. In clinical settings, it is often prepared into formulas with other Chinese herbal medicines and used to treat various viral infection-related diseases. After painstaking explorations, the inventors prepared and extracted the active monomeric compounds forsythin and phillygenin, and synthesized and prepared forsythiin derivatives through chemical synthesis. Based on a series of pharmacological activity studies carried out by the inventors, it was found that forsythiin, forsythiin derivatives and the composition of forsythiin and phillygenin are effective in treating human cytomegalovirus infection, which provided new approaches for the prevention and treatment for this kind of disease.

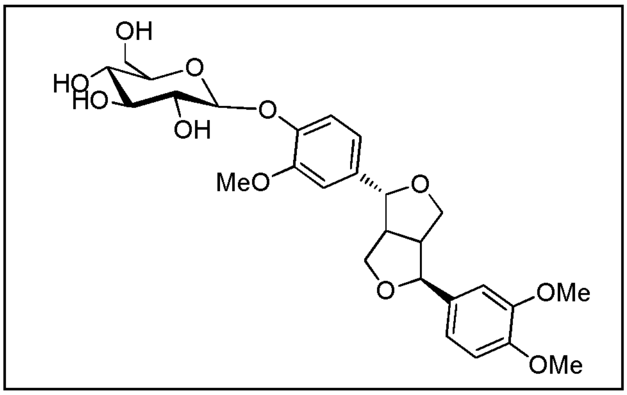

Forsythin

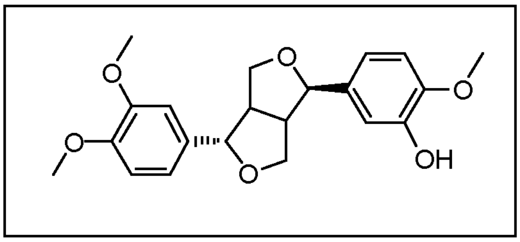

(+)- Phillygenin

Panax ginseng belongs to the genus Panax of the Araliaceae family. It has been widely used in China, Korea and Japan for over 2000 years. It is mainly indicated for the prevention of diseases and prolonging life expectancy. According to the records in the Shennong's Classic of Materia Medica, panax ginseng has the following therapeutic effects, including nourishing the five viscera, calming the nerves, settling the mind, stopping the palpitation due to fright, eliminating the evil, improving the eyesight and intelligence, conducive to controlling body weight and prolonging life expectancy. Modern medical studies have shown that panax ginseng's main functions and effects include regulating the central nervous system, exerting anti-cancer and anti-tumor effects, regulating immune functions, exerting anti-diabetic effects, enhancing liver function, improving cardiovascular and cerebrovascular disorders, exerting anti-arteriosclerosis effects, regulating blood pressure, improving climacteric disorders, exerting anti-osteoporosis effects, and exerting anti-fatigue, anti-oxidation as well as anti-aging effects, etc.

As the main effective components of panax ginseng, ginsenoside Rg3 and ginsenoside Rg5 have good safety profiles and have been developed as anti-tumor oral preparations for clinical application. In-depth studies have been conducted on their dosage forms of injection.

Most existing studies investigate the individual pharmacological effects of panax ginseng medicinal material, total ginsenoside, forsythia suspensa medicinal material or phillygenin, and no study has used the combination of panax ginseng and forsythia suspensa to study their antiviral, antipyretic, anti-inflammatory and immunity boosting pharmacological effects. Based on a large amount of modern and scientific studies, the inventors of the present invention extracted the effective components from the panax ginseng and forsythia suspensa medicina materials by adopting state-of-art

separation and purification technologies. In addition, the inventors carried out studies on the application of the extracts of panax ginseng and forsythia suspensa and the corresponding drug products in achieving the antiviral, antipyretic, anti-inflammatory, and immunity boosting effects, and applied them in the manufacturing of drug products and health foods, showing broad market prospects.

In addition, based on a large amount of modern and scientific studies, the inventors of the present invention extracted the effective components of forsythin and the composition of forsythin and phillygenin from forsythia suspensa and its leaves, and synthesized and prepared the forsythin derivatives through further studies. These products can provide health products and medicines with high efficiency and low toxicity for the susceptible population and patients of human cytomegalovirus infection.

### Description of the Invention

The present invention aims to provide a novel composition of forsythia suspensa extract (preferably also including panax ginseng extract), the preparation method, and its application in providing the antiviral, antipyretic, anti-inflammatory and immunity boosting effects, including especially the novel application of forsythin, forsythin derivatives and the composition of forsythin and phillygenin (forsythin/phillygeninin) for the prevention and treatment of diseases associated with human cytomegalovirus infection.

The first aspect of the present invention provides a composition to achieve the antiviral, antipyretic, anti-inflammatory and/or immunity boosting effects, which includes the forsythia suspensa components.

In the first aspect of the present invention, the forsythia suspensa components consist of preferably forsythin, forsythin derivatives and the composition of forsythin and phillygenin. Wherein, the content of the said forsythin, forsythin derivatives and the composition of forsythin and phillygenin is ≥ 1%, preferably ≥ 30%, further preferably ≥ 60%, still further preferably ≥ 80%, and still further preferably ≥ 98%.

The said forsythin derivative is preferably a phillygenin glucuronic acid derivative. In particular, the said forsythin derivatives include but are not limited to 33-hydroxy phillygenin-8-O-β-D-glucuronide, 9-hydroxy phillygenin-8-O-β-D-glucuronide, 33, 34-methylenedioxy phillygenin-8-O-β-D-glucuronide, (2R,3R,4R,5S)-methyl 6-(5-((1R,4S)-4-(3,4-dimethoxyphenyl) hexahydrofuro[3,4-c] furan-1-yl)-2-methoxyphenoxy)-3,4,5 -trihydroxyte trahydro-2H-pyran-2-carboxylate, sodium (2R,3R,4R,5S)-6-(5- ((1R,4S) -4- (3,4-dimethoxyphenyl) hexahydrofuro [3,4-c]furan-1-yl) -2-methoxyphenoxy)-3,4,5-trihydroxytetrahydro -2H - pyran-2-carboxylate, potassium (2R,3R,4R,5S)-6-(5-((1R,4S)-4- (3,4-dimethoxyphenyl) hexahydrofuro[3,4-c]furan-1-yl)-2-methoxyphenoxy)-3,4,5-trihydroxytetrahydro- 2H- pyran-2-carboxylate or (2R,3R, 4R,5S)-6-(5-((1R,4S)-4-(3,4-dimethoxyphenyl) hexahydrofuro[3,4-c]furan-1-yl)-2-methoxyphenoxy)-3,4,5 -trihydroxytetrahydro-2H-pyran-2-carboxylic acid; preferably 33-hydroxy phillygenin-8-O-β-D-glucuronide, 9-hydroxy phillygenin-8-O-β-D-glucuronide, 33, 34-methylenedioxy phillygenin-8-O-β-D-glucuronide, (2R,3R,4R,5 S)-methyl 6-(5-((1R,4S)-4-(3,4-dimethoxyphenyl) hexahydrofuro [3,4-c] furan-1-yl)-2-methoxyphenoxy)-3,4,5 -trihydroxyte trahydro-2H-pyran-2-carboxylate, sodium (2R,3R,4R,5S)-6-(5- ((1R,4S) -4- (3,4-dimethoxyphenyl) hexahydrofuro [3,4-c]furan-1-yl) -2-methoxyphenoxy)-3,4,5-trihydroxytetrahydro -2H - pyran-2-carboxylate and potassium (2R,3R,4R,5S)-6-(5-((1R,4S)-4- (3,4-dimethoxyphenyl) hexahydrofuro[3,4-c]furan-1-yl)-2-methoxyphenoxy)-3,4,5 -trihydroxytetrahydro- 2H- pyran-2-carboxylate.

In particular, the composition of forsythin and phillygenin consists of forsythin and phillygeninin. Preferably, wherein the weight ratio of forsythin and phillygenin in the composition of forsythin and phillygenin is 2-99.99: 0.01-98, preferably 80-99: 1-20, further preferably 90-98: 2-10, such as 98:2 or 90:10.

In the said composition of the first aspect of the present invention, it is also preferable to include a panax ginseng component, i.e., the composition of the first aspect of the present invention is preferably a composition of panax ginseng and forsythia suspensa. Wherein, the weight ratio of panax ginseng component to forsythia suspensa component in the composition of panax ginseng and forsythia suspensa is 2-98:2-98, preferably 80:20 or 20:80, further preferably 90:6 or 6:90, and still further preferably 98:2 or 2:98.

In particular, the content of ginsenoside Rg3 in the panax ginseng component in the composition of panax ginseng and forsythia suspensa is ≥ 0.03%, preferably ≥ 10%, further preferably ≥ 50%, still further preferably ≥ 80%, still further preferably ≥ 90%, and still further preferably ≥ 99%. The content of forsythin in the forsythia suspensa component is ≥ 0.15%, preferably ≥ 15%, further preferably ≥ 60%, still further preferably ≥ 80%, still further preferably ≥ 90% and still further preferably ≥ 99%.

The composition of the first aspect of the present invention provides a composition for achieving the antiviral, antipyretic, anti-inflammatory and/or immunity boosting effects, which includes panax ginseng and forsythin. More preferably, wherein, the ginsenoside is total ginsenoside, panaxadiol saponins, ginsenoside Rg3 or 20 (R)-ginsenoside Rg3.

Further preferably, this composition still further includes phillygenin.

Preferably, the composition of the first aspect of the present invention consists of the panax ginseng extract and the forsythia suspensa extract. Preferably, wherein the extract is an alcoholic extract or water extract, for example, the composition of the first aspect of the present invention may consist of an alcoholic extract of panax ginseng and an

alcoholic extract of forsythia suspensa. The composition of the first aspect of the present invention may also consist of a water extract of panax ginseng and a water extract of forsythia suspensa. The alcohol can be methanol or ethanol or an aqueous solution thereof, preferably ethanol or an aqueous solution thereof. In the aqueous solution of alcohol, the concentration of alcohol may be 50% to 100% (V/V), preferably 60% to 95% (V/V), such as 70 to 90% (V/V). Wherein, the weight ratio of panax ginseng and forsythia suspensa used for the extraction is preferably 2-50: 50-98. In the specific embodiment of the present invention, the extracting step may include soaking the panax ginseng and/or forsythia suspensa in alcohol or water and then heating and retaining the filtrate and optionally carrying out or repeating a plurality of times (e.g., 2-5 times) the step of heating the filtered residue with alcohol or water and retaining the filtrate, and then drying the filtrate.

In the composition of the first aspect of the present invention, it is also preferable to further include one or more of the following components, including panax notoginseng, aloe vera, glycyrrhiza uralensis, fallopia multiflora, ginkgo biloba, black sesame extract, zingiber officinale extract, grape seed extract, pomegranate seed extract, plant essential oil, arbutin, vitamin C and derivatives thereof or vitamin E and derivatives thereof. The second aspect of the present invention provides the preparation for achieving the antiviral, antipyretic, anti-inflammatory and/or immunity-boosting effects, which comprise the composition of the first aspect of the present invention and a carrier that is acceptable in the industries of pharmaceutics, health products or foods. Preferably, the present invention provides medicines or health products for achieving the antiviral, antipyretic, anti-inflammatory and/or immunity boosting effects, which include the panax ginseng component and the forsythia suspensa component. Wherein, the weight ratio of panax ginseng component to forsythia suspensa component is 2-98:2-98, preferably 80:20 or 20:80, further preferably 90:6 or 6:90, and still further preferably 98:2 or 2:98. In particular, the said medicines or health products consist of the components of the composition of panax ginseng and forsythia suspensa and a pharmaceutically acceptable carrier.

Wherein, the ratio of the total weight of the panax ginseng component to the forsythia suspensa component to the weight of the pharmaceutically acceptable carrier in the composition of panax ginseng and forsythia suspensa is 1:1 to 1:100, preferably 1:10, further preferably 1: 5, still further preferably 1: 2, and still further preferably 1:1.

In particular, the ratio of the weight of the said composition of panax ginseng and forsythia suspensa to the total weight of the said medicine or health product is 0.01-50:100, preferably 0.1-50:100, and further preferably 1-50:100.

In particular, the said panax ginseng component and the forsythia suspensa component can also be added with pharmaceutically acceptable carriers and/or food additives.

The said drugs may also include forsythin, forsythin derivatives, the composition of forsythin and phillygenin, and a pharmaceutically acceptable carrier, preferably consisting of forsythin, forsythin derivatives and the composition of forsythin and phillygenin and a pharmaceutically acceptable carrier. In addition, the said health products may also include forsythin, forsythin derivatives and the composition of forsythin and phillygenin and a carrier or food additive acceptable in the industry of health products or foods, preferably composed of forsythin, forsythin derivatives and the composition of forsythin and phillygenin and a carrier or food additive acceptable in the industry of health products or foods.

Preferably, the weight ratio of forsythin, forsythin derivatives and the composition of forsythin and phillygenin to the total weight of the health product or drug is 0.01-50:100.

Pharmaceutically acceptable carriers are generally accepted by health professionals as suitable for this purpose and as inactive ingredients in pharmaceutical agents. The compilation of pharmaceutically acceptable carriers can be found in the Handbook of Pharmaceutical Excipients, 2nd edition, edited by A. Wade and P. J. Weller, and published by the American Pharmaceutical Association, Washington and The Pharmaceutical Press, London, 1994. Food additives are non-nutritive substances that are consciously added to foods in small amounts to improve the appearance, flavor, texture or storage properties of foods. Relevant food additives can be found in China's national food safety standard, the Standards for Use of Food Additives. Food additives are non-nutritive substances that are consciously added to foods in small amounts to improve the appearance, flavor, texture or storage properties of foods. Relevant food additives can be found in China's national food safety standard, the Standards for Use of Food Additives.

In particular, the said carrier comprises excipients, such as starch and water; lubricants, such as magnesium stearate; disintegrants, such as microcrystalline cellulose; filling agents, such as lactose; binding agents, such as pregelatinized starch and dextrin; sweeteners; antioxidants; preservatives; flavoring agents, colorants and spices.

In particular, the said drug may exist in the dosage forms of tablets, capsules, pills, powder, granules, syrup, solution, emulsion, injection, spray, aerosol, gel, cream, cataplasm, adhesive plaster or emplastrum.

In particular, the said health food products may exist in the forms of tablets, capsules, pills, powder, granules, syrup, solution, emulsion, spray, aerosol, gel, cream, cataplasm, adhesive plaster or emplastrums, or in the forms of foods, such as dairy products, confectionery, beverages, biscuits, tea leaves and related products, wine and the like.

In particular, based on panax ginseng component and forsythia suspensa component, the said medicines or health products may also further include one or more of the following components, including panax notoginseng, aloe vera, glycyrrhiza uralensis, fallopia multiflora, ginkgo biloba, black sesame extract, zingiber officinale extract, grape seed extract, pomegranate seed extract, plant essential oil, arbutin, vitamin C and derivatives thereof or vitamin E and derivatives thereof.

Also in particular, based on forsythin, forsythin derivatives and the composition of forsythin and phillygenin, the said drug or health product may also include the extract of evodia spp. fruit, zingiber officinale, amomum cardamomum, alpinia katsumadai hayata, alpinia officinarum, bamboo shavings, phragmitis rhizoma, pinellia ternata, syzygium aromaticum, inula japonica inflorescence, eriobotrya japonica leaf, diospyros kaki calyx, canavalia gladiata seed, red ochre, agastache rugosa, aquilaria sinensis, perilla frutescens, vitamin C and its derivatives or vitamin E and its derivatives.

Wherein, in the said composition of panax ginseng and forsythia suspensa, the components of panax ginseng and forsythia suspensa exist in the form of Chinese medicinal materials, or the composition of panax ginseng-forsythia suspensa extracts prepared by solvent heating extraction method, or the composition of the composition of panax ginseng-forsythia suspensa-cyclodextrin comprising the composition of panax ginseng and forsythia suspensa combined with cyclodextrin or its derivative, or the composition of ginsenoside-forsythin-cyclodextrin comprising the ginsenoside-forsythin and ginsenoside-forsythin combined with cyclodextrin or its derivatives.

In particular, the said panax ginseng-forsythia extract composition selects panax ginseng-forsythia suspensa alcohol extract and panax ginseng-forsythia suspensa water extract. The said ginsenoside consists of total ginsenoside, panaxadiol saponins and 20 (R)-panax ginsenoside Rg3; and the said forsythins include and forsythin and phillygenin.

In particular, the said composition of panax ginseng-forsythia suspensa-cyclodextrin is characterized by the selection of a mixture prepared by mixing the composition of panax ginseng-forsythia suspensa extracts with α-, β- or γ-cyclodextrin or its derivatives, or a complex prepared by physical or chemical treatment of the composition of panax ginseng-forsythia suspensa extract with α-, β- or γ-cyclodextrin or its derivatives.

In particular, the said composition of ginsenoside-forsythin-cyclodextrin is characterized by the selection of a mixture prepared by mixing ginsenoside, forsythin and cyclodextrin with α-, β- or γ-cyclodextrin or its derivatives, or a complex prepared by physical or chemical treatment of ginsenoside, forsythin and α-, β- or γ-cyclodextrin or its derivatives.

Wherein, the weight ratio of the traditional Chinese medicine components to the cyclodextrins or the derivatives thereof in the said complex is 1:1-100.

In particular, the said cyclodextrin is α-, β- or γ-cyclodextrin; the said cyclodextrin derivatives are hydroxyethyl□-cyclodextrin, 2,6-dimethyl□-cyclodextrin, 2,3,6-trimethyl□-cyclodextrin, 2,6-diethyl□-cyclodextrin, 2,3,6-triethyl□-cyclodextrin, maltosyl□-cyclodextrin or sulfobutylether β-cyclodextrin, p-toluenesulfonyl chloride (p-TsCl) substituted β-cyclodextrin, 6-position substituted β-CD p-toluenesulfonate (β-cyclodextrin-6-OTs), 2-oxohydroxypropyl-β-cyclodextrin, 2-position monosubstituted p-toluenesulfonate (2-β-cyclodextrin-2-OTs), β-cyclodextrin p-toluenesulfonate (tosyl-β-CD), PCL-(Tos) 7-β-CD, and the star-shaped macromolecule of β-cyclodextrin.

The third aspect of the present invention provides the method for preparing the composition of the first aspect of the present invention, comprising a step of mixing ginsenoside and forsythin and optionally phillygenin, or comprising the step of extracting panax ginseng and forsythia suspensa and optionally Chinese medicinal materials by heating with a solvent. The former can be a mixing method for substantially pure compounds.

In the specific embodiment of the present invention, the said panax ginseng-forsythia suspensa alcohol extract is prepared according to the following steps:

1) Mix the medicinal materials of panax ginseng and forsythia suspensa with the extracting solvent and the process by soaking;
2) Heat and extract the mixture of the soaked panax ginseng, forsythia suspensa and extracting solvent, filter and collect the extraction solution;
3) Collect and combine the extraction solution, concentrate it, prepare it into an extractum and dry it.

In particular, the duration of the soaking treatment process described in step 1) is 30-60 min, preferably 30 min.

In particular, the weight ratio of the medicinal material panax ginseng and the extracting solvent is 1:8-12, preferably 1:10.

In particular, the extracting solvent is an ethanol solution with a volume percent concentration of ≥ 50%, preferably a 50% to 75% ethanol solution.

Wherein, the number of times of heating extraction described in step 2) is 2-3 times, preferably 3 times.

In particular, in each heating and extracting process, the weight ratio of the said panax ginseng and forsythia suspensa medicinal materials and the extracting solvent is 1: 8-12, preferably 1: 8; the temperature during each heating extraction is 60-80°C, preferably 70°C; and the heating extraction duration is 0.5-2 h, preferably 1 h. Wherein step 3), in the said concentration process, the temperature is 65-85°C, preferably 80°C, the relative density of the concentrated extract is 1.05-1.15, preferably 1.1, and the temperature in the drying treatment process is 70-90°C, preferably 85°C.

Wherein, the said panax ginseng-forsythia suspensa water extract is prepared according to the following steps:

1) Mix the medicinal materials of panax ginseng and forsythia suspensa with water and process by soaking;
2) Decoct and extract the mixture of the soaked panax ginseng, forsythia suspensa and extracting solvent, filter and collect the extraction solution;

3) Collect and combine the extraction solution, concentrate it, prepare it into an extractum and dry it.

In particular, the duration of the soaking treatment process described in step 1) is 30-60 min, preferably 30 min.
In particular, the weight ratio of the medicinal materials panax ginseng and extracting solvent is 1:8-12, preferably 1:10.
Wherein, the number of times of the said decocting extraction in step 2) is 2-3 times, preferably 3 times.
In particular, in each decocting and extracting process, the weight ratio of the said panax ginseng medicinal materials and extracting solvent is 1: 8-12, preferably 1: 8; the temperature during each decocting extraction is 90-100°C, preferably 90-95°C; and the heating extraction duration is 0.5-2 h, preferably 1 h.
Wherein step 3), in the said concentration process, the temperature is 70-95°C, preferably 80°C, the relative density of the concentrated extract is 1.05-1.15, preferably 1.1, and the temperature in the drying treatment process is 70-95°C, preferably 85°C.
Wherein, the said total ginsenoside is prepared according to the following steps:

1) Mix the medicinal material of panax ginseng with water, and soak the material;
2) Heat and extract the mixture of the soaked panax ginseng and extracting solvent, filter and collect the extraction solution;
3) Use the macroporous resin column to separate the collected panax ginseng extract, collect and combine the eluent to obtain the panax ginseng eluent from the resin column;
4) Concentrate and dry the eluent of panax ginseng eluent from the resin column to obtain the total ginsenoside ginseng root.

In particular, the duration of the soaking treatment process described in step 1) is 30-60 min, preferably 30 min.
In particular, the weight ratio of the medicinal material panax ginseng and the extracting solvent is 1:8-12, preferably 1:10.
In particular, the extracting solvent is an ethanol solution with a volume percent concentration of ≥ 50%, preferably a 50% to 75% ethanol solution.
Wherein, the number of times of heating extraction described in step 2) is 2-3 times, preferably 3 times.
In particular, in each heating and extracting process, the weight ratio of the said panax ginseng medicinal materials and the extracting solvent is 1: 8-12, preferably 1: 8; the temperature during each heating extraction is 60-80°C, preferably 70°C; and the heating extraction duration is 0.5-2 h, preferably 1 h.
In particular, the method also comprises concentrating the ginseng extract collected in step 2) to prepare the panax ginseng concentrated solution and then carrying out the said separation treatment using the macroporous resin column.
In particular, the concentrated panax ginseng solution obtained after the concentration treatment contains 1-3.5 g/mL of crude drug panax ginseng, i.e., equivalent to 1-3.5 g crude drug/ml, preferably 2.5 g crude drug/mL.
Wherein, the said chromatographic treatment using the macroporous resin column in step 3) comprises the following sequential steps: 3A) injecting panax ginseng extract solution into the macroporous resin column, then using water as the eluent for elution; 3B) using ethanol solution with volume percentage concentration of 30% to 50% as the eluent for elution and collecting ethanol eluent to obtain the panax ginseng eluent from resin column.
In particular, during chromatography using the macroporous resin column, the ratio of the weight of panax ginseng in the panax ginseng extract solution to the volume of macroporous resin is 1: 0.8-2.5, preferably 1: 1. In particular, during the separation treatment using the macroporous resin column, the ratio of the column diameter of the macroporous resin column to the height of the resin column is 1: 5-10, preferably 1: 5-7, and further preferably 1: 5.5-5.9.
Wherein, one of X-5, AB-8, NK-2, NKA-2, NK-9, D3520, D101 and WLD is selected for the said macroporous adsorption resin in step 3), preferably selecting D101.
In particular, the ratio of the amount of water used in step 3A) to the column volume of the macroporous resin column is 2-4:1, preferably 4:1; the ratio of the amount of ethanol solution with a volume percentage concentration of 30%-50% to the volume of the macroporous resin column in step 3B) is 2-8:1, preferably 4-8:1, further preferably 8:1.
In particular, the volume percentage concentration of the eluent ethanol solution used in step 3B) is 50%. Wherein step 4), in the concentration process, the temperature is 65-90°C, preferably 80°C; and the temperature in the drying treatment process is 75-95°C, preferably 85°C.
In particular, the content of the said total ginsenosides is 20% to 50% (the test is conducted in accordance with the method specified in Annex B of the national standard: GB/T19506-2009).
Wherein, the said panaxadiol is prepared according to the following steps:

1) Mix the medicinal material of panax ginseng with water, and soak the material;
2) Heat and extract the mixture of the soaked panax ginseng and extracting solvent, filter and collect the extraction

solution;

3) Use the macroporous resin column to perform the first chromatographic separation treatment of the collected panax ginseng extract. Collect and combine the eluent to obtain the first panax ginseng eluent from the resin column;

4) Use the macroporous resin column to perform the second chromatographic separation treatment of the first panax ginseng eluent from the resin column. Collect and combine the eluent to obtain the second panax ginseng eluent from the resin column;

5) Concentrate and dry the second eluent of panax ginseng eluent from the resin column to obtain the panaxadiol.

In particular, the duration of the soaking treatment process described in step 1) is 30-60 min, preferably 30 min.

In particular, the weight ratio of the medicinal material panax ginseng and the extracting solvent is 1:8-12, preferably 1:10.

In particular, the extracting solvent is an ethanol solution with a volume percent concentration of ≥ 50%, preferably a 50% to 75% ethanol solution.

Wherein, the number of times of heating extraction described in step 2) is 2-3 times, preferably 3 times.

In particular, in each heating and extracting process, the weight ratio of the said panax ginseng medicinal materials and the extracting solvent is 1:8-12, preferably 1: 8; the temperature during each heating extraction is 60-80°C, preferably 70°C; and the heating extraction duration is 0.5-2 h, preferably 1 h.

In particular, the method also comprises the step 2), that is, concentrating the panax ginseng extract collected in step 2) to prepare the panax ginseng concentrated solution and then perform the first macroporous resin column chromatography separation treatment.

In particular, the concentrated panax ginseng solution obtained after the concentration treatment contains 1-3.5 g/mL of crude drug panax ginseng, i.e., equivalent to 1-3.5 g crude drug/ml, preferably 2.5 g crude drug/mL.

Wherein, the said first chromatographic treatment using the macroporous resin column in step 3) comprises the following sequential steps: 3A) injecting panax ginseng extract solution into the macroporous resin column, then using water as the eluent for elution; 3B) using ethanol solution with volume percentage concentration of 30% to 50% as the eluent for elution and collecting ethanol eluent to obtain the first panax ginseng eluent from the macroporous resin column.

In particular, during chromatography using the macroporous resin column, the ratio of the weight of panax ginseng in the panax ginseng extract solution to the volume of macroporous resin is 1: 0.8-2.5, preferably 1: 1. In particular, during the separation treatment using the macroporous resin column, the ratio of the column diameter of the macroporous resin column to the height of the resin column is 1: 5-10, preferably 1: 5-7, and further preferably 1: 5.5-5.9.

Wherein, in the said macroporous adsorption resin in step 3), one of X-5, AB-8, NK-2, NKA-2, NK-9, D3520, D101 and WLD is selected, preferably selecting D101.

In particular, the ratio of the amount of water used in step 3A) to the column volume of the macroporous resin column is 2-4:1, preferably 4:1; the ratio of the amount of ethanol solution with a volume percentage concentration of 30%-50% to the volume of the macroporous resin column in step 3B) is 2-8:1, preferably 4-8:1, further preferably 8:1.

In particular, the volume percentage concentration of the eluent ethanol solution used in step 3B) is 50%.

In particular, the method also comprises the step 3), that is, concentrating the first panax ginseng eluent from the macroporous resin column to prepare the first panax ginseng concentrated solution by the resin column and then perform the said second separation treatment using the macroporous resin column chromatography.

In particular, the concentrated panax ginseng solution obtained after the concentration treatment contains 3.5-6 g/mL of crude drug panax ginseng, i.e., equivalent to 3.5-6 g crude drug/ml, preferably 5.0 g crude drug/mL.

In particular, the said second chromatography using the macroporous resin column in step 4) comprises the following sequential steps: 4A) injecting the first eluent of panax ginseng from the resin column into the macroporous resin column, followed by eluting using ethanol solution with a volume percentage concentration of 50% to 60% as the eluent; 4B) using ethanol solution with a volume percentage concentration of 70% to 80% as the eluent to elute and collecting the ethanol eluent to obtain the second eluent of panax ginseng from the resin column.

In particular, during the second chromatography using the macroporous resin column in step 4), the ratio of the weight of panax ginseng in the first eluent of panax ginseng from the resin column to the volume of macroporous resin is 1:0.8-2.5, preferably 1:1.

In particular, during the separation treatment using the macroporous resin column, the ratio of the column diameter of the macroporous resin column to the height of the resin column is 1:5-10, preferably 1:5-7, and further preferably 1: 5.5-5.9.

Wherein, one of HPD-200, D203, XAD-4 and HPD-100 is selected for the said macroporous adsorption resin in step 4), preferably selecting HPD-100.

In particular, the ratio of the amount of the ethanol solution with a volume percentage concentration of 50% to 60% to the column volume of the macroporous resin column used in step 4A) is 2-4:1, preferably 4:1; the ratio of the amount of ethanol solution with a mass percentage concentration of 70%-80% to the volume of the macroporous resin

column in step 3B) is 2-8:1, preferably 4-8:1, further preferably 8:1.

Wherein step 5), in the said concentration process, the temperature is 65-95°C, preferably 80°C; and the temperature in the drying treatment process is 70-95°C, preferably 85°C.

In particular, the content of the said panaxadiol is 30% to 70% (the test is conducted in accordance with the method specified in Annex B of the national standard: GB/T19506-2009).

Wherein the content of the said 20 (R)-ginsenoside Rg3 is 1% to 98%, preferably 30% to 80%, further preferably 60%.

In particular, the content of the said 20 (R)-ginsenoside Rg3 is ≥ 1%, preferably ≥ 30%, further preferably ≥ 60%, still further preferably ≥ 80%, and still further preferably ≥ 98%.

Wherein, the content of the 20 (R)-ginsenoside Rg3 derivative is 1% to 98%, preferably 30% to 80%, further preferably 60%.

In particular, the content of the said 20 (R)-ginsenoside Rg3 is ≥ 1%, preferably ≥ 30%, further preferably ≥ 60%, still further preferably ≥ 80%, and still further preferably ≥ 98%, and still further preferably ≥ 98%. Wherein, the said forsythin is extracted and prepared according to the following method:

1) Heat the forsythia suspensa leaves or forsythia suspense and reflux with the solvent for 2-3 times, with a duration of 2-4 h each time;
2) Concentrate the extract, allow it to stand and precipitate to obtain the crude mixture of phillygenin and forsythin;
3) Dissolve the crude mixture of phillygenin and forsythin with the solvent, allow it to stand and crystallize to obtain the mixture of phillygenin and forsythin.
4) Recrystallize of the mixture of phillygenin and forsythin with the solvent to obtain the forsythin extract.

In particular, the said solvents in steps 1), 3) and 4) are methanol, ethanol, acetone, aqueous methanol or aqueous ethanol.

In particular, the said aqueous methanol has a mass percentage concentration of 70% to 95%; and the said aqueous ethanol has a mass percentage concentration of 70% to 95%.

Wherein, the standing temperature in step 2) is room temperature, preferably 10-35°C, further preferably 20-25°C; the standing duration is 1-48 h; the volume ratio of the concentrated extract solution to the original extract solution in step 2) is 0.1-0.5:1.

In particular, the said temperature of the recrystallization treatment in step 4) is room temperature, preferably 10-35°C, further preferably 20-25°C.

The fourth aspect of the present invention provides the method for preparing the preparation of the third aspect of the present invention, which comprises the step of mixing the composition of the first aspect of the present invention with a carrier acceptable in the industries of pharmaceutics, health products or foods.

Preferably, the method of the fourth aspect of the present invention comprises the step of mixing the composition of the first aspect of the present invention with a cyclodextrin or a derivative thereof (e.g., α-, β-or γ-cyclodextrin or a derivative thereof). It is also preferable that the method of the fourth aspect of the present invention comprises the step of physically or chemically treating the composition of the first aspect of the present invention with a cyclodextrin or a derivative thereof (e.g., α-, β-or γ-cyclodextrin or a derivative thereof) to form a complex.

The fifth aspect of the present invention provides the application of the composition of the first aspect of the present invention in the preparation of a medicine or health foods to achieve the antiviral, antipyretic, anti-inflammatory and/or immunity-boosting effects. Accordingly, the present invention provides the method for achieving the antiviral, antipyretic, anti-inflammatory and/or immunity-boosting effects, which comprises administering an effective amount of the composition of the first aspect of the present invention to an individual who need the said composition. Preferably, the individual may be a mammal, preferably a human. Preferably, the fifth aspect of the present invention provides the application of forsythin, forsythin derivatives and the composition of forsythin and phillygenin in the preparation of health products or medicines with anti-cytomegalovirus therapeutic effects; correspondingly, it also provides the application of forsythin, forsythin derivatives and the composition of forsythin and phillygenin in the preparation of health products or medicines for the prevention and treatment of disorders caused by cytomegalovirus infection. The said health product or medicine contains forsythin, forsythin derivatives and the composition of forsythin and phillygenin as the active ingredients for the anti-cytomegalovirus effect or for the prevention and treatment of human cytomegalovirus infection related disorders or diseases.

Wherein, the cytomegalovirus is human cytomegalovirus and accordingly, the said condition is a disorder caused by infection with human cytomegalovirus. The said condition comprises a simple human cytomegalovirus infection condition, such as simple human cytomegalovirus infection and patients infected by human cytomegalovirus during the treatment of cardiovascular and cerebrovascular diseases, tumors, burns or AIDS and during the process of organ transplantation or during the perinatal period.

Wherein, the said composition of forsythin and phillygenin is composed of the forsythin and phillygenin monomers, or the composition of phillygenin and forsythin extracts prepared by the solvent heating extraction method, or the composition of phillygenin-forsythin-cyclodextrinare prepared with phillygenin and forsythin and cyclodextrin or its derivatives.

In particular, the said composition of phillygenin-forsythin-cyclodextrin is a mixture by selecting a mixture prepared by mixing phillygenin, forsythin and α-, β- or γ-cyclodextrin or its derivatives, or a complex prepared by physical or chemical treatment of phillygenin, forsythin and α-, β- or γ-cyclodextrin or its derivatives.

In particular, the ratio of the weight of phillygenin and forsythin to the weight of cyclodextrin or a derivative of cyclodextrin in the composition of phillygenin-forsythin-cyclodextrin is 1:1-50.

In particular, the said cyclodextrin is α-, β- or γ-cyclodextrin; the said cyclodextrin derivatives are hydroxyethyl -cyclodextrin, 2,6-dimethyl -cyclodextrin, 2,3,6-trimethyl -cyclodextrin, 2,6-diethyl -cyclodextrin, 2,3,6-triethyl -cyclodextrin, maltosyl -cyclodextrin or sulfobutylether β-cyclodextrin, p-toluenesulfonyl chloride (p-TsCl) substituted β-cyclodextrin, 6-position substituted β-CD p-toluenesulfonate (β-cyclodextrin-6-OTs), 2-oxohydroxypropyl-β-cyclodextrin, 2-position monosubstituted p-toluenesulfonate (2-β-cyclodextrin-2-OTs), β-cyclodextrin p-toluenesulfonate (tosyl-β-CD) and PCL-(Tos) 7-β-CD, the star-shaped macromolecule of β-cyclodextrin.

Unless otherwise specified, the term "effective amount" as used herein refers to the amount of medicine needed to produce a therapeutically or prophylactically effective effect. The "effective amount" can be adjusted and varied, ultimately determined by the healthcare personnel by taking into account the following factors of the general conditions, such as the route of administration and the nature of the formulation, the recipient’s body weight and age, as well as the nature and severity of the disease being treated.

Compared to the prior art, the present invention has the following obvious advantages:

1. The formula of the composition of the panax ginseng and forsythia suspensa of the present invention is scientific and unique with definite therapeutic effect, high safety profile, controllable quality, rapid onset of action, little toxic and side effect, and is suitable for long-term use. Hence, it has good prospects in terms of pharmaceutical use. A series of experimental studies have proved that it can achieve the antiviral, antipyretic, anti-inflammatory and/or immunity boosting effects, with its effects and functions significantly superior to the effect of panax ginseng or forsythia suspensa when used alone. The combination use of panax ginseng and forsythia suspensa in accordance with a specific proportion has a synergistic effect.
2. The serial experimental studies conducted for the present invention demonstrated that forsythin, forsythin derivatives and the composition of forsythin and phillygenin have significant effects in preventing, relieving and treating diseases related to human cytomegalovirus infection, with potent pharmacological effects, quick onset of action, little toxic and side effects, and good safety profile. It is suitable for long-term administration. Therefore, it has good application prospects.
3. The product of the present invention has the following advantages: abundant sources of raw materials, low price, mild process condition, safe clinical use, and simple preparation process. It can be prepared into various dosage forms, with small dosage and convenient use; in particular, the product of the present invention is safe in raw materials, it can be consumed on a daily basis or taken for a long period of time, and it can be produced into dairy products, candy, beverage, biscuit, teas and related products, wine and other food forms, so it has wide applicability scope and is easy to be popularized.
4. The composition of the forsythia composition of the present invention can be formulated quantitatively using monomer components or prepared by extracting to prepare into the composition of panax ginseng and forsythia suspensa, or the composition of panax ginseng and forsythia suspensa is combined with α-, β- or γ-cyclodextrin or its derivatives and formulated into a composition. In addition, the composition of panax ginseng and forsythia suspensa medicine can be combined to prepare compounds with one or more of the following components to achieve the antiviral, antipyretic, anti-inflammatory and/or immunity-boosting effects: dandelion extract, isatis tinctoria root extract, honeysuckle flower extract, anemarrhena extract, scrophularia extract, prunella extract, phragmitis rhizoma extract, lophatherum gracile extract, gardenia jasminoides extract, fritillariae cirrhosae bulbus extract, folium Ilicis latifoliae extract and houttuyniae herba extract).
5. The present invention can use the active ingredient of the single component of forsythin and forsythin derivative and the composition of forsythin and phillygenin or the composition of forsythin and forsythin derivative and the composition of forsythin and phillygenin and other active ingredients (such as the extract of evodia spp. fruit, zingiber officinale, amomum cardamomum, alpinia katsumadai hayata, alpinia officinarum, bamboo shavings, phragmitis rhizoma, pinellia ternata, syzygium aromaticum, inula japonica inflorescence, eriobotrya japonica leaf, diospyros kaki calyx, canavalia gladiata seed, red ochre, agastache rugosa, aquilaria sinensis, perilla frutescens, vitamin C and its derivatives or vitamin E and its derivatives) to prepare a medicine for preventing, relieving and treating human cytomegalovirus infection related conditions. The products use common formulas for preventing and treating human cytomegalovirus infection.

## Detailed Embodiment of the Invention

The beneficial effects of the formulations described in the present invention are further described below using the specific embodiments. These exemplary embodiments are exemplary only and do not constitute any limitation to the scope of the present invention. It should acknowledged by those skilled in the art that the details and forms of the technical proposal of the present invention can be modified or substituted without departing from the formulation ideas and scope of use of the present invention. However, all these modifications and substitutions fall within the scope of protection of the present invention.

The said forsythin derivatives 33-hydroxy-phillygenin glucuronide, 9-hydroxy-phillygenin glucuronide and 33, 34-methylene dioxy-phillygenin glucuronide in the present invention are the same as the forsythin derivatives described in the patent application (application No.: 201510319303.4, priority No.: 201510164294.6). The (2R,3R,4R,5S)-methyl 6-(5-((1R,4S)-4-(3,4-dimethoxyphenyl) hexahydrofuro[3,4-c] furan-1-yl)-2-methoxyphenoxy)-3,4,5 -trihydroxyte trahydro-2H-pyran-2-carboxylate, sodium (2R,3R,4R,5S)-6-(5- ((1R,4S) -4- (3,4-dimethoxyphenyl) hexahydrofuro [3,4-c]furan-1-yl) -2-methoxyphenoxy)-3,4,5-trihydroxytetrahydro -2H - pyran-2-carboxylate and potassium (2R,3R,4R,5S)-6-(5-((1R,4S)-4- (3,4-dimethoxyphenyl) hexahydrofuro[3,4-c]furan-1-yl)-2-methoxyphenoxy)-3,4,5-trihydroxytetrahydro- 2H- pyran-2-carboxylate and forsythione glucuronic acid are the same as those described in the patent application (application No.: 201510320579.4, priority No.: 201410825547.5).

### Exemplary Embodiment 1: Forsythin Tablets

1. Prepare the raw materials according to the following ratios:

| | |
|---|---|
| Forsythin (purity: 86%) | 500 g |
| Starch | 1000 g |
| Talc | 15g |
| Magnesium stearate | 15g |

2. Mix forsythin with starch by the above ratios, mix well and prepare the mixture into granules, add talc and magnesium stearate, mix well, and then compress the mixture into 10,000 tablets.

### Exemplary Embodiment 2: Forsythin Capsules

1. Prepare the raw materials according to the following ratios:

| | |
|---|---|
| Forsythin (purity: 60%) | 200 g |
| Starch | 1000 g |

2. Mix forsythin with starch, mix well and dispense it into 10,000 capsules.

### Exemplary Embodiment 3: Forsythin Capsules

1. Prepare the raw materials according to the following ratios:

| | |
|---|---|
| Forsythin (purity: 98%) | 500 g |
| Starch | 1000 g |

2. Mix forsythin with starch, mix well and dispense it into 10,000 capsules.

### Exemplary Embodiment 4: Forsythin Tablets

1. Prepare the raw materials according to the following ratios:

| | |
|---|---|
| Forsythin (purity: 63%) | 50 g |
| Evodia spp. fruit extract | 200 g |
| Vitamin C | 400 g |
| Starch | 1000 g |
| Talc | 16.5g |
| Magnesium stearate | 16.5g |

2. Mix forsythin, evodia spp. fruit extract, vitamine C and starch, mix well and prepare the mixture into granules, then add talc and magnesium stearate, mix well, and then compress the mixture into 10,000 tablets.

**Exemplary Embodiment 5: Forsythin Capsules**

1. Prepare the raw materials according to the following ratios:

| | |
|---|---|
| Forsythin (purity: 98%) | 300 g |
| Alpinia officinarum extract | 300 g |
| Vitamin C | 600 g |
| Starch | 1000 g |

2. Mix forsythin, alpinia officinarum extract, vitamine C and starch, mix well and dispense it into 10,000 capsules.

**Exemplary Embodiment 6: Forsythin Granules**

1. Prepare the raw materials according to the following ratios:

| | |
|---|---|
| Forsythin (purity: 92%) | 500 g |
| Amomi fructus extract | 30 g |
| Vitamin C | 600 g |
| Sucrose powder | 5000 g |

2. Mix forsythin, amomi fructus extract, vitamine C and sucrose powder, mix well and prepare it into granules and dispense it into 10,000 sachets.

**Exemplary Embodiment 7: Forsythin Oral Solution**

1. Prepare the raw materials according to the following ratios:

| | |
|---|---|
| Forsythin (purity: 98%) | 4g |
| Bamboo shavings extract | 3g |
| Phragmitis rhizoma extract | 3g |
| Glucose syrup | 5g |
| Deionized water | Appropriate amount |

Take forsythin, bamboo shavings extract and phragmitis rhizoma extract, dissolve with ethanol, add glucose syrup, then add deionized water to 100 ml, and mix well to obtain the product.

**Exemplary Embodiment 8: 33-Hydroxy-phillygenin Glucuronide Tablets**

1. Prepare the raw materials according to the following ratios:

| | |
|---|---|
| 33-hydroxy-phillygenin glucuronide (purity 66%) | 500 g |
| Starch | 1000 g |
| Talc | 15g |
| Magnesium stearate | 15g |

2. Mix 33-hydroxy-phillygenin glucuronide and starch well, granulate, add talc and magnesium stearate, and compress to make 10000 tablets.

**Exemplary Embodiment 9: 33-Hydroxy-phillygenin Glucuronide Capsules**

1. Prepare the raw materials according to the following ratios:

| | |
|---|---|
| 33-hydroxy-phillygenin glucuronide (purity 60%) | 200 g |
| Starch | 1000 g |

2. Mix 33-hydroxy-phillygenin glucuronide and starch well, and fill it into capsules to make 10000 capsules.

**Exemplary Embodiment 10: 33-Hydroxy-phillygenin Glucuronide Capsules**

1. Prepare the raw materials according to the following ratios:

| | |
|---|---|
| 33-hydroxy-phillygenin glucuronide (purity 98%) | 500 g |
| Starch | 1000 g |

2. Mix 33-hydroxy-phillygenin glucuronide and starch well, and fill it into capsules to make 10000 capsules.

**Exemplary Embodiment 11: 33-Hydroxy-phillygenin Glucuronide Tablets**

1. Prepare the raw materials according to the following ratios:

| | |
|---|---|
| 33-hydroxy-phillygenin glucuronide (purity 63%) | 50 g |
| Evodia spp. fruit extract | 20 g |
| Vitamin C | 400 g |
| Starch | 1000 g |
| Talc | 14.7g |
| Magnesium stearate | 14.7g |

2. Mix 33-hydroxy-phillygenin glucuronide, evodia spp. fruit extract, vitamin C and starch well, granulate, add talc and magnesium stearate and mix well and compress to make 10000 tablets.

**Exemplary Embodiment 12: 33-Hydroxy-phillygenin Glucuronide Capsules**

1. Prepare the raw materials according to the following ratio:

| | |
|---|---|
| 33-Hydroxy-phillygenin glucuronide (purity 98%) | 300 g |
| Pinellia ternata extract | 30 g |
| Vitamin C | 600 g |
| Starch | 1000 g |

2. Mix 33-hydroxy-phillygenin glucuronide, pinellia ternata extract, vitamin C and starch well, and fill it into capsules to make 10000 capsules.

**Exemplary Embodiment 13: 33-Hydroxy-phillygenin Glucuronide Granules**

1. Prepare the raw materials according to the following ratio:

| | |
|---|---|
| 33-Hydroxy-phillygenin glucuronide (purity 92%) | 500 g |
| Amomum cardamomum extract | 30 g |
| Vitamin C | 600 g |
| Sucrose powder | 5000 g |

2. Mix 33-hydroxy-phillygenin glucuronide, amomum cardamomum extract, vitamin C and sucrose powder well, granulate, and dispense it into 10000 bags.

**Exemplary Embodiment 14: 33-Hydroxy-phillygenin Glucuronide Oral Solution**

1. Prepare the raw materials according to the following ratio:

| | |
|---|---|
| 33-Hydroxy-phillygenin glucuronide (purity 98%) | 4g |
| Evodia spp. fruit extract | 3g |
| Amomum cardamomum extract | 3g |
| Glucose syrup | 5g |
| Deionized water | Appropriate amount |

2. Dissolve 33-hydroxy-phillygenin glucuronide, amomum cardamomum extract and evodia spp. fruit extract in ethanol, add glucose syrup, and finally add deionized water to 100 mL.

**Exemplary Embodiment 15: 9-Hydroxy-phillygenin Glucuronide Tablets**

1. Prepare the raw materials according to the following ratio:

| | |
|---|---|
| 9-Hydroxy-phillygenin glucuronide (purity 66%) | 500 g |
| Starch | 1000 g |
| Talc | 15g |
| Magnesium stearate | 15g |

2. Mix 9-hydroxy-phillygenin glucuronide and starch well, granulate, add talc and magnesium stearate, and compress to make 10000 tablets.

**Exemplary Embodiment 16: 9-Hydroxy-phillygenin Glucuronide Capsules**

1. Prepare the raw materials according to the following ratio:

| | |
|---|---|
| 9-Hydroxy-phillygenin glucuronide (purity 97%) | 200 g |
| Starch | 1000 g |

2. Mix 9-hydroxy-phillygenin glucuronide and starch well, and fill it into capsules to make 10000 capsules.

**Exemplary Embodiment 17: 9-Hydroxy-phillygenin Glucuronide Capsules**

1. Prepare the raw materials according to the following ratio:

| | |
|---|---|
| 9-Hydroxy-phillygenin glucuronide (purity 98%) | 500 g |
| Starch | 1000 g |

2. Mix 9-hydroxy-phillygenin glucuronide and starch well, and fill it into capsules to make 10000 capsules.

**Exemplary Embodiment 18: 9-Hydroxy-phillygenin Glucuronide Tablets**

1. Prepare the raw materials according to the following ratio:

| | |
|---|---|
| 9-Hydroxy-phillygenin glucuronide (purity 63%) | 50 g |
| Evodia spp. fruit extract | 20 g |
| Vitamin C | 400 g |
| Starch | 1000 g |
| Talc | 14.7g |
| Magnesium stearate | 14.7g |

2. Mix 9-hydroxy-phillygenin glucuronide, evodia spp. fruit extract, vitamin C and starch, granulate, add talc and magnesium stearate and mix well, and compress to make 10000 tablets.

**Exemplary Embodiment 19: 9-Hydroxy-phillygenin Glucuronide Capsules**

1. Prepare the raw materials according to the following ratio:

| | |
|---|---|
| 9-Hydroxy-phillygenin glucuronide (purity 98%) | 300 g |
| Pinellia ternata extract | 30 g |
| Vitamin C | 600 g |
| Starch | 1000 g |

2. Mix 9-hydroxy-phillygenin glucuronide, pinellia ternata extract, vitamin C and starch well, and fill it into capsules to make 10000 capsules.

**Exemplary Embodiment 20: 9-Hydroxy-phillygenin Glucuronide Granules**

1. Prepare the raw materials according to the following ratio:

| | |
|---|---|
| 9-Hydroxy-phillygenin glucuronide (purity 92%) | 500 g |
| Amomum cardamomum extract | 30 g |
| Vitamin C | 600 g |
| Sucrose powder | 5000 g |

2. Mix 9-hydroxy-phillygenin glucuronide, amomum cardamomum extract, vitamin C and sucrose powder well, granulate, and dispense it into 10000 bags.

**Exemplary Embodiment 21: 9-Hydroxy-phillygenin Glucuronide Oral Solution**

1. Prepare the raw materials according to the following ratio:

| | |
|---|---|
| 9-Hydroxy-phillygenin glucuronide (purity 98%) | 4g |
| Inula japonica inflorescence extract | 3g |
| Amomum cardamomum extract | 3g |
| Glucose syrup | 5g |
| Deionized water | Appropriate amount |

2. Dissolve 9-hydroxy-phillygenin glucuronide, amomum cardamomum extract and inula japonica inflorescence extract in ethanol, add glucose syrup, and finally add deionized water to 100 mL.

**Exemplary Embodiment 22: 33,34-Methylenedioxy-phillygenin Glucuronide Tablets**

1. Prepare the raw materials according to the following ratio:

| | |
|---|---|
| 33,34-Methylenedioxy-phillygenin glucuronide (purity 66%) | 500 g |
| Starch | 1000 g |
| Talc | 15g |
| Magnesium stearate | 15g |

2. Mix 33,34-methylenedioxy-phillygenin glucuronide and starch well, granulate, add talc and magnesium stearate and mix well, and compress to make 10000 tablets.

**Exemplary Embodiment 23: 33,34-Methylenedioxy-phillygenin Glucuronide Capsules**

1. Prepare the raw materials according to the following ratio:

| | |
|---|---|
| 33,34-Methylenedioxy-phillygenin glucuronide (purity 97%) | 200 g |
| Starch | 1000 g |

2. Mix 33,34-methylenedioxy-phillygenin glucuronide and starch well, and fill it into capsules to make 10000 capsules.

**Exemplary Embodiment 24: 33,34-Methylenedioxy-phillygenin Glucuronide Capsules**

1. Prepare the raw materials according to the following ratio:

| | |
|---|---|
| 33,34-Methylenedioxy-phillygenin glucuronide (purity 98%) | 500 g |
| Starch | 1000 g |

2. Mix 33,34-methylenedioxy-phillygenin glucuronide and starch well, and fill it into capsules to make 10000 capsules.

**Exemplary Embodiment 25: 33,34-Methylenedioxy-phillygenin Glucuronide Tablets**

1. Prepare the raw materials according to the following ratio:

| | |
|---|---|
| 33,34-Methylenedioxy-phillygenin glucuronide (purity 63%) | 50 g |
| Inula japonica inflorescence extract | 20 g |
| Vitamin C | 400 g |
| Starch | 1000 g |
| Talc | 14.7g |

(continued)

| | |
|---|---|
| Magnesium stearate | 14.7g |

2. Mix 33,34-methylenedioxy-phillygenin glucuronide, inula japonica inflorescence extract, vitamin C and starch well, granulate, add talc and magnesium stearate and mix well, and compress to make 10000 tablets.

**Exemplary Embodiment 26: 33,34-Methylenedioxy-phillygenin Glucuronide Capsules**

1. Prepare the raw materials according to the following ratio:

| | |
|---|---|
| 33,34-Methylenedioxy-phillygenin glucuronide (purity 98%) | 300 g |
| Pinelli a ternata extract | 30 g |
| Vitamin C | 600 g |
| Starch | 1000 g |

2. Mix 33,34-methylenedioxy-phillygenin glucuronide, pinellia extract, vitamin C and starch well, and fill it into capsules to make 10000 capsules.

**Exemplary Embodiment 27: 33,34-Methylenedioxy-phillygenin Glucuronide Granules**

1. Prepare the raw materials according to the following ratio:

| | |
|---|---|
| 33,34-Methylenedioxy-phillygenin glucuronide (purity 92%) | 500 g |
| Amomum cardamomum extract | 30 g |
| Vitamin C | 600 g |
| Sucrose powder | 5000 g |

2. Mix 33,34-methylenedioxy-phillygenin glucuronide, amomum cardamomum extract, vitamin C and sucrose powder well, granulate, and dispense it into 10000 bags.

**Exemplary Embodiment 28: 33,34-Methylenedioxy-phillygenin Glucuronide Granules**

1. Prepare the raw materials according to the following ratio:

| | |
|---|---|
| 33,34-Methylenedioxy-phillygenin glucuronide (purity 98%) | 300 g |
| Amomum cardamomum extract | 30 g |
| Vitamin C | 600 g |
| Sucrose powder | 5000 g |
| Make 10000 bags | |

2. Mix 33,34-methylenedioxy-phillygenin glucuronide, amomum cardamomum extract, vitamin C and sucrose powder well, granulate, and dispense it into 10000 bags.

**Exemplary Embodiment 29: 33,34-Methylenedioxy-phillygenin Glucuronide Oral Solution**

1. Prepare the raw materials according to the following ratio:

| | |
|---|---|
| 33,34-Methylenedioxy-phillygenin glucuronide (purity 98%) | 4g |
| Inula japonica inflorescence extract | 3g |
| Amomum cardamomum extract | 3g |

(continued)

| | |
|---|---|
| Glucose syrup | 5g |
| Deionized water | Appropriate amount |

2. Dissolve 33,34-methylenedioxy-phillygenin glucuronide, amomum cardamomum extract and inula japonica inflorescence extract in ethanol, add glucose syrup, and finally add deionized water to 100 mL.

**Exemplary Embodiment 30: Preparation of Forsythin and Phillygenin Composition Tablets**

1. Prepare forsythin/phillygenin composition tablets according to the following ratio:

| | |
|---|---|
| Forsythin/phillygenin composition (weight ratio of 98: 2) | 500 g |
| Starch | 480 g |
| Talc | 10 g |
| Magnesium stearate | 10 g |

2. Mix 490 g of forsythin and 10 g of phillygenin with starch well, granulate, add talc and magnesium stearate, and compress to make 10000 tablets.

**Exemplary Embodiment 31: Preparation of Forsythin/Phillygenin Composition Granules**

1. Prepare forsythin/phillygenin composition granules according to the following ratio:

| | |
|---|---|
| Forsythin/phillygenin composition (weight ratio of 98: 2) | 100 g |
| Microcrystalline cellulose | 10000 g |

2. Mix 98 g of forsythin and 2 g of phillygenin with microcrystalline cellulose well, granulate, and dispense it into 10000 bags.

**Exemplary Embodiment 32: Preparation of Forsythin/Phillygenin Composition Capsules**

1. Prepare forsythin/phillygenin composition capsules according to the following ratio:

| | |
|---|---|
| Forsythin/phillygenin composition (weight ratio of 98: 2) | 250 g |
| Starch | 2500 g |

2. Mix 245g of forsythin and 5g of phillygenin with starch well, and fill it into capsules to make 10000 capsules.

**Exemplary Embodiments 33-36: Preparation of Forsythin/Phillygenin Composition Capsules**

In Exemplary Embodiments 33-36, the forsythin/phillygenin composition is mixed with starch in the weight ratios in the following table and then filled into capsules to make 10000 capsules, respectively.

| Exemplary Embodiment No | Raw material Phillygenin and forsythin composition/g | Excipient Starch/g | Weight ratio of raw material to excipient |
|---|---|---|---|
| Exemplary Embodiment 33 | 500(98:2) | 500 | 1:1 |
| Exemplary Embodiment 34 | 50 (80:20) | 5000 | 1:100 |
| Exemplary Embodiment 35 | 250(90:10) | 2500 | 1:10 |

(continued)

| Exemplary Embodiment No | Raw material Phillygenin and forsythin composition/g | Excipient Starch/g | Weight ratio of raw material to excipient |
|---|---|---|---|
| Exemplary Embodiment 36 | 250(95:5) | 5000 | 1:20 |

**Exemplary Embodiments 37-40: Preparation of Forsythin/Phillygenin Composition Granules**

In Exemplary Embodiments 37-40, the forsythin/phillygenin composition is mixed with microcrystalline cellulose according to the weight ratios in the following table, granulated and packed into 10000 bags.

| Exemplary Embodiment No | Raw material Phillygenin and forsythin composition/g | Excipient Microcrystalline cellulose/g | Weight ratio of raw material to excipient |
|---|---|---|---|
| Exemplary Embodiment 37 | 1000(98:2) | 1000 | 1:1 |
| Exemplary Embodiment 38 | 250 (80:20) | 25000 | 1:100 |
| Exemplary Embodiment 39 | 2500(90:10) | 25000 | 1:10 |
| Exemplary Embodiment 30 | 2500(95:5) | 50000 | 1:20 |

**Exemplary Embodiment 41: Preparation of Forsythin/Phillygenin Composition Tablets**

1. Prepare forsythin/phillygenin composition tablets according to the following ratio:

| | |
|---|---|
| Forsythin/phillygenin composition (weight ratio of 98: 2) | 500 g |
| Starch | 380 g |
| Agastache rugosa extract | 100 g |
| Talc | 10 g |
| Magnesium stearate | 10 g |

2. Mix 490 g of forsythin and 10 g of forsythin with agastache rugosa extract powder well, then mix well with starch, granulate, add talc and magnesium stearate and mix well, and compress to make 10000 tablets.

**Exemplary Embodiment 42: Preparation of Forsythin/Phillygenin Composition Granules**

1. Prepare forsythin/phillygenin composition granules according to the following ratio:

| | |
|---|---|
| Forsythin/phillygenin composition (weight ratio of 98: 2) | 250 g |
| Aquilaria sinensis extract | 250 g |
| Agastache rugosa extract | 250 g |
| Microcrystalline cellulose | 24500 g |

2. Mix 245g of forsythin and 5g of phillygenin with aquilaria sinensis extract and agastache rugosa extract powder, then mix well with microcrystalline cellulose, granulate, and dispense it into 10000 bags.

**Exemplary Embodiment 43: Preparation of Forsythin/Phillygenin Composition Capsules**

1. Prepare forsythin/phillygenin composition capsules according to the following ratio:

| | |
|---|---|
| Forsythin/phillygenin composition (weight ratio of 98: 2) | 250 g |
| Perilla frutescens extract | 250 g |
| Eriobotrya japonica leaf extract | 250 g |
| Syzygium aromaticum extract | 250 g |
| Starch | 1000 g |

2. Mix 245g of forsythin and 5g of phillygenin, perilla frutescens extract, eriobotrya japonica leaf extract and syzygium aromaticum extract powder well, then mix well with starch, and fill it into capsules to make 10000 capsules.

**Exemplary Embodiment 44: Preparation of Forsythin/Phillygenin Composition Tablets**

1. Prepare forsythin/phillygenin composition tablets according to the following ratio:

| | |
|---|---|
| Forsythin/phillygenin composition (weight ratio of 80: 20) | 500 g |
| Starch | 480 g |
| Eriobotrya japonica leaf extract | 500 g |
| Talc | 10 g |
| Magnesium stearate | 10 g |

2. Mix 490 g of forsythin and 10 g of phillygenin with eriobotrya japonica leaf extract powder well, then mix well with starch, granulate, add talc and magnesium stearate and mix well, and compress to make 10000 tablets.

**Exemplary Embodiment 45: Preparation of Forsythin/Phillygenin Composition Granules**

1. Prepare forsythin/phillygenin composition granules according to the following ratio:

| | |
|---|---|
| Forsythin/phillygenin composition (weight ratio of 90: 10) | 1000 g |
| Inula japonica inflorescence extract | 500 g |
| Pinellia ternata extract | 500 g |
| Microcrystalline cellulose | 10000 g |

2. Mix 900 g of forsythin and 100 g of phillygenin with the above extracts (inula japonica inflorescence, pinellia ternata), then mix well with microcrystalline cellulose, granulate, and dispense it into 10000 bags.

**Exemplary Embodiment 46: Preparation of Forsythin/Phillygenin Composition Capsules**

1. Prepare forsythin/phillygenin composition capsules according to the following ratio:

| | |
|---|---|
| Forsythin/phillygenin composition (weight ratio of 94: 6) | 2000 g |
| Inula japonica inflorescence extract | 250 g |
| Katsumade galangal seed extract | 500 g |
| Eriobotrya japonica leaf extract | 250 g |
| Starch | 1000 g |

2. Mix 1880 g of forsythin and 120 g of phillygenin with the above extracts (inula japonica inflorescence, katsumade galangal seed, eriobotrya japonica leaf) well, then mix well with starch, and fill it into capsules to make 10000 capsules.

**Exemplary Embodiment 47: Preparation of Forsythin/Phillygenin Composition Soft Capsules**

1. Prepare forsythin/phillygenin composition soft capsules according to the following ratio:

| | |
|---|---|
| Forsythin/phillygenin composition (weight ratio of 94: 6) | 2000 g |
| β-cyclodextrin | 1500 g |
| PEG-400 | 2000 g |
| PEG-4000 | 26g |
| Propylene glycol | 260 g |

2. Heat 2000 g of PEG-400, 26g of PEG-4000 and 260 g of propylene glycol to 65°C to melt, mix well, and use this as a water-soluble matrix; Mix with the composition and mix well. Take 5000 g of gelatin and 4500 g of water, heat to 70°C, take 1750 g of glycerin and 15 g of methylparaben, stir, evacuate for 1 h, mix with the above gelatin, maintain at 70°C and allow to stand overnight. Prepare with an automatic soft capsule filling machine to obtain 10000 soft capsules.

**Exemplary Embodiment 48: Experimental Study of Forsythin, Forsythin Derivative, Forsythin and Phillygenin Composition against Human Cytomegalovirus in Vitro**

1. Experimental Materials

1.1 Drugs and Reagents:

Forsythin (Batch No.: 20130303), white powder, manufactured by Dalian Fusheng Natural Drugs Development Co., Ltd. The purity was determined to be 99.5% by high performance liquid chromatography (HPLC). The content was confirmed to be 99.5% by calibration with the forsythin reference standard for assay of pharmaceutical and biological products in China.
33-Hydroxy-forsythin glucuronide (forsythin derivative A, batch No.: 20130301), white powder, manufactured by Dalian Fusheng Natural Drugs Development Co., Ltd. The purity was determined to be 98.5% by area normalization with two detectors (ultraviolet detector and evaporative light scattering detector) of high performance liquid chromatography.
9-Hydroxy-forsythin glucuronide (forsythin derivative B, batch number: 20130302), white powder, manufactured by Dalian Fusheng Natural Drugs Development Co., Ltd. The purity was determined to be 99.2% by area normalization with two detectors (ultraviolet detector and evaporative light scattering detector) of high performance liquid chromatography.
33,34-Methylenedioxy-forsythin glucuronide (forsythin derivative C, batch number: 20130301), white powder, manufactured by Dalian Fusheng Natural Drugs Development Co., Ltd. The purity was determined to be 98.7% by area normalization with two detectors (ultraviolet detector and evaporative light scattering detector) of high performance liquid chromatography.
Forsythin/phillygenin composition A (Batch No.: 20130305), white powder, manufactured by Dalian Fusheng Natural Drugs Development Co., Ltd. Upon the content test by high performance liquid chromatography, the ratio of forsythin to phillygenin was 98: 2, and the sum of the contents of forsythin and phillygenin was 98.4%. Forsythin/phillygenin composition B (Batch No.: 20130306), white powder, manufactured by Dalian Fusheng Natural Drugs Development Co., Ltd. Upon the content test by high performance liquid chromatography, the ratio of forsythin to phillygenin was 90: 10, and the sum of the contents of forsythin and phillygenin was 98.1%.
Ganciclovir (GCV) Powder for Injection (trade name: Li ke wei, manufacturer: Hubei Keyi Pharmaceutical Co., Ltd., batch No.: 091102)

1.2 Viruses and Cells

Human embryonic lung fibroblast cell strain (purchased from Sino Best Biological Technology Co., Ltd.). The cells were cultured and subcultured according to the conventional method.
HCMV AD169 strain was purchased from American Type Culture Collection (ATCC).

2. Method

2.1 Viral Virulence Assay:

The cells and viruses were cultured, subcultured and proliferated according to the conventional method, and the $TCD_{50}$ of HCMV AD169 strain was $10^{-4.6}$.

2.2. Drug Toxicity Testing:

MTT and cytopathic effect methods were used to determine the maximum non-toxic concentration ($TD_0$) of each drug and GCV, respectively. The results by the two methods were consistent. The results are shown in Table 1.

2.3. Inhibitory Effect on HCMV

Cells were inoculated in a 96-well plate, allowed to grow into monolayers, and inoculated with a virus challenge of 100 times $TCD_{50}$. Starting from TDo, each drug was diluted stepwise according to 10-fold gradient to prepare 5 concentrations, with 4 wells set for each concentration. In addition, 4 wells of normal cell control and 4 wells of virus control were set. After 10 days of culture, the minimum effective concentration (MEC) of the drug was investigated, and the therapeutic index (TDo/MEC) was calculated. The specific data are shown in the table below.

Table 1 Summary of Therapeutic Index for Drug and Control

| Drug name | Maximum non-toxic concentration $TD_0$ | Minimum effective concentration MEC | Therapeutic index ($TD_0$/MEC) |
|---|---|---|---|
| Ganciclovir (GCV) | 10 $mg.L^{-1}$ | 10 $mg.L^{-1}$ | 1 |
| Forsythin | 40 $mg.L^{-1}$ | 4 $mg.L^{-1}$ | 10 |
| 33-Hydroxy-phillygenin glucuronide | 30 $mg.L^{-1}$ | 0.3 $mg.L^{-1}$ | 100 |
| 9-Hydroxy-phillygenin glucuronide | 40 $mg.L^{-1}$ | 0.4 $mg.L^{-1}$ | 100 |
| 33,34-Methylenedioxy-phillygenin glucuronide | 40 $mg.L^{-1}$ | 0.4 $mg.L^{-1}$ | 100 |
| Forsythin/phillygenin composition A | 30 $mg.L^{-1}$ | 3 $mg.L^{-1}$ | 10 |
| Forsythin/phillygenin composition B | 30 $mg.L^{-1}$ | 3 $mg.L^{-1}$ | 10 |

3 Discussions

The inhibitory effects of forsythin, forsythin derivative, forsythin and phillygenin composition on human cytomegalovirus (HCMV) in vitro were investigated using the cytopathic effect method and MTT method with antiviral drug ganciclovir as control. The results showed that forsythin, forsythin derivative, forsythin and phillygenin composition strongly inhibited human cytomegalovirus in vitro, with lower toxicity and higher therapeutic index than those of the control drug. It is suggested that forsythin, forsythin derivative, forsythin and phillygenin composition have potential clinical application prospects for the inhibition of cytomegalovirus.

**Exemplary Embodiments 49-52: Preparation of Panax Ginseng-forsythia Suspensa Composition (Original Powder of Crude Drug)**

Two crude drug powders of panax ginseng and forsythia suspensa are taken, and the two can be mixed according to the weight ratio in Table 2 to prepare the composition of panax ginseng and forsythia suspensa. Panax ginseng and forsythia suspensa are crude drugs meeting the standards of Chinese Pharmacopoeia.

Table 1. Raw Material Ratio Table of the Panax Ginseng and Forsythia Suspensa Composition of Exemplary Embodiments 49-52

| Exemplary Embodiments No. | Weight ratio | |
|---|---|---|
| | Panax ginseng | Forsythia suspensa (leaf) |
| Exemplary Embodiment 49 | 98 | 2 |
| Exemplary Embodiment 50 | 50 | 50 |
| Exemplary Embodiment 51 | 25 | 75 |
| Exemplary Embodiment 52 | 2 | 98 |

**Exemplary Embodiment 53: Preparation of Alcohol Extract of Panax Ginseng-forsythia Suspensa Composition**

1. First extraction treatment

Add pulverized panax ginseng (300 g) and forsythia suspensa (300 g) into a multi-functional extraction tank of traditional Chinese medicine, respectively, mix them with ethanol solution (3000 g) at the volume percentage concentration of 70%, soak for 30 min, turn on the power of the multi-functional extraction tank, heat, perform the first heating extraction treatment on panax ginseng and forsythia suspensa respectively, keep the temperature at 70°C for constant temperature extraction for 1 h, and then filter to obtain the first extract and the first residue; The weight ratio of panax ginseng and forsythia suspensa separately to the extracting solvent (ethanol solution) is 1:10.

In the present invention, in addition to the weight ratio of panax ginseng and forsythia suspensa separately to the extracting solvent of 1:10, and other ratios, such as 1:8-12, are applicable to the present invention. In addition to the soaking time of 30 min, other soaking times of ≥ 30 min are applicable to the present invention, preferably 30-60 min; In addition to the ethanol solution at the volume percentage concentration of 70%, other ethanol solutions with a volume percentage concentration of ≥ 50% are applicable to the invention, preferably the ethanol solution at a concentration of 50-75% and absolute ethanol. The extraction temperature of 60-80°C and the heating extraction time of 0.5-2h are applicable to the invent.

2. Second extraction treatment

Adding ethanol solution at the volume percentage concentration of 70% to the first residue, heat, perform the second heating extraction treatment on panax ginseng and forsythia suspensa, respectively, keep the temperature at 70°C for constant temperature extraction for 1 h, and then filter to obtain the second extract and the second residue. The weight ratio of panax ginseng and forsythia suspensa separately to the extracting solvent is 1:10.

3. Third extraction treatment

Add ethanol solution at the volume percentage concentration of 70% to the second residue, heat, perform the third heating extraction treatment on panax ginseng and forsythia suspensa, respectively, keep the temperature at 70°C for constant temperature extraction for 1 h, and then filter to obtain the third extract. The weight ratio of panax ginseng and forsythia suspensa to the extracting solvent is 1:10.

In the exemplary embodiment of the present invention, in addition to the ethanol solution at the volume percentage concentration of 70% for the extracting solvent used in the second and third extraction processes of the alcohol extract of panax ginseng and forsythia suspensa, other ethanol solutions at a volume percentage concentration of ≥ 50% are applicable to the present invention, preferably ethanol solution at a concentration of 50-75% and absolute alcohol. The extraction temperature of 60-80°C is applicable to the present invention; The heating extraction time of 0.5-2h is applicable to the present invention.

In the exemplary embodiment of the present invention, there are 3 heating extractions in the preparation process of alcohol extract of panax ginseng and forsythia suspensa, and extraction for 2-3 times is applicable to the present invention.

4. Concentration and drying treatment

Mix the first, second and third extracts obtained by 3 filtrations according to the weight ratio of 2: 98, then place it in a rotary evaporator for evaporation and concentration at the temperature of 80°C to obtain an extractum at the

relative density of 1.1, and then place it in an oven for drying to a constant weight at 85°C to obtain an alcohol extract of the panax ginseng and forsythia suspensa composition.

The alcohol extract of the panax ginseng and forsythia suspensa composition is a light brownish-yellow powder, has a special odor, and is soluble in ethanol.

In the process of concentration of the alcohol extract of the panax ginseng and forsythia suspensa composition in the present invention, the concentration temperature of 65-85°C and the relative density of the concentrated extractum of 1.05-1.15 are applicable to the present invention. The temperature of 70-90°C in the drying process is applicable to the present invention. The content of total ginsenoside in the alcohol extract of panax ginseng prepared by the method in the present invention is 2-5%.

### Exemplary Embodiment 54 Preparation of Water Extract of Panax Ginseng-forsythia Suspensa Composition

1. Soaking Treatment

Put the pulverized panax ginseng (300 g), forsythia suspensa (300 g) and extracting solvent tap water (3000 g) into the multi-function extraction tank of traditional Chinese medicine respectively, mix well, and soak for 30 min; The weight ratio of panax ginseng and forsythia suspensa separately to extracting solvent water is 1:10.

In the preparation process of the water extract of panax ginseng and forsythia suspensa in the present invention, a soaking time of ≥ 30 min is applicable to the present invention, preferably 30-60 min; in addition to the weight ratio of panax ginseng to extracting solvent water of 1:10, other ratios such as 1: 8-12 are applicable to the present invention.

2. First extraction treatment

After soaking for 30 min, turn on the power supply of the multi-function extraction tank, heat, decoct and extract panax ginseng and forsythia suspensa for the first time, keep the temperature at 95°C for constant temperature extraction for 1 h, and then filter to obtain the first extract and the first residue;

3. Second extraction treatment

Add tap water to the first residue, heat, decoct and extract panax ginseng and forsythia suspensa for the second time, keep the temperature at 90°C for constant temperature extraction for 1 h, and then filter to obtain the second extract and the second residue. The weight ratio of panax ginseng to extracting solvent is 1:10.

4. Third extraction treatment

Add tap water to the second residue, heat, decoct and extract panax ginseng and forsythia suspensa for the third time, keep the temperature at 90°C for constant temperature extraction for 1 h, and then filter to obtain the third extract. The weight ratio of panax ginseng and forsythia suspensa to extracting solvent is 1:10.

In the exemplary embodiment of the present invention, in addition to the weight ratio of 1:10 of panax ginseng to extracting solvent water, other ratios such as 1: 8-12 are applicable to the present invention in the first, second and third extraction processes of the water extract of panax ginseng and forsythia suspensa. The extraction temperature of 90-100°C is applicable to the present invention, preferably 90-95°C. The heating extraction time of 0.5 to 2h is applicable to the present invention, preferably 1h.

In the exemplary embodiment of the present invention, there are 3 heating extractions in the preparation process of water extract of panax ginseng and forsythia suspensa, and extraction for 2-3 times is applicable to the present invention.

5. Concentration and Drying Treatment

Mix the first, second and third extracts obtained by 3 filtrations according to the weight ratio of 2: 98, then place it in a rotary evaporator for evaporation and concentration at the temperature of 80°C to obtain an extractum at the relative density of 1.1, and then place it in an oven for drying to a constant weight at 85°C to obtain a water extract of panax ginseng.

The water extract of the panax ginseng and forsythia suspensa composition is a light brownish-yellow powder, has a special odor, and is well water-soluble.

In the process of concentration of the water extract of the panax ginseng and forsythia suspensa composition in the present invention, a concentration temperature of 70-95°C and the relative density of a concentrated extractum of 1.05-1.15 are applicable to the present invention; a drying temperature of 70-95°C is applicable to the present invention.

**Exemplary Embodiment 55: Preparation of Total Ginsenoside**

1. First extraction treatment: the same as in Exemplary Embodiment 5.
2. Second extraction treatment: the same as in Exemplary Embodiment 5.
3. Third extraction treatment: the same as in Exemplary Embodiment 5.
4. Concentration Treatment

Combine the first, second and third extracts obtained by 3 filtrations, and place it in a rotary evaporator at 80°C for concentration to obtain panax ginseng concentrate (120 mL), which is equivalent to 2.5 g crude drug/ml panax ginseng solution.

With respect to the concentration of panax ginseng concentrate in the process of concentrating treatment in the present invention, other concentrations of per milliliter of concentrate equivalent to 1-3.5 g crude drug (i.e. 1-3.5 g crude drug/ml) are applicable to the present invention in addition to the concentration of per milliliter of concentrate equivalent to 2.5 g crude drug.

5. Macroporous Resin Column Chromatography

Load the panax ginseng concentrate on a macroporous resin column, and perform the macroporous resin column separation treatment, wherein, the D101 macroporous adsorption resin is selected as the macroporous adsorption resin, and the ratio of the resin volume (300 mL) in the resin column to the weight (dry weight) of panax ginseng is 1: 1 (i.e., if the dry weight of panax ginseng is 1kg, the volume of macroporous resin is 1L; if the dry weight of crude drug is 1g, the volume of macroporous resin is 1 mL); after the concentrated supernatant completely flows into the resin column, wash with water of 4 times the column volume, and discard the eluate; Then elute with 50% (volume percentage) ethanol solution of 8 times column volume, collect the eluate, and obtain panax ginseng-macroporous resin eluate;

In the process of macroporous resin column chromatography for the panax ginseng concentrate in the present invention, the ratio of the weight of panax ginseng in the panax ginseng extract to the volume of the macroporous resin of 1: 0.8-2.5 is applicable to the present invention; In addition to D101, other macroporous adsorption resins X-5, AB-8, NK-2, NKA-2, NK-9, D3520, D101 and WLD are applicable to the present invention; In the process of water elution, the ratio of the amount of water to the column volume of the macroporous resin column of 2-4: 1 is applicable to the present invention. In addition to 50%, other volume percentage concentrations of ethanol solution of 30%-50% are applicable when the eluate is ethanol solution. The ratio of the amount of ethanol solution to the column volume of the macroporous resin column of 2-4: 1 is applicable to the present invention.

6. Concentration and Drying Treatment

Concentrate the panax ginseng-macroporous resin eluate under reduced pressure at 80°C in a rotary evaporator, recover the solvent, and dry the concentrated residue in a drying oven at 85°C to obtain 2.6 g of total ginsenoside.

Total ginsenoside is a light brownish-yellow powder with a special odor. The total ginsenoside content was measured using the method specified in Appendix B of GB/T19506-2009, and the total ginsenoside content was 32%.

In the process of concentration of the ginseng-macroporous resin eluate in the present invention, a concentrating temperature of 65-90°C is applicable to the present invention; A drying temperature of 75-95°C is applicable to the present invention. The content of total ginsenoside prepared by the method in the present invention is 20-50%.

**Exemplary Embodiment 56: Preparation of Panaxadiol Saponins**

1. First extraction treatment: the same as in Exemplary Embodiment 53.
2. Second extraction treatment: the same as in Exemplary Embodiment 53.
3. Third extraction treatment: the same as in Exemplary Embodiment 53.
4. Concentration treatment: the same as in Exemplary Embodiment 53.
5. First macroporous resin column chromatography

As in Exemplary Embodiment 53, obtain the first panax ginseng-macroporous resin eluate.

6. Second macroporous resin column chromatography

Put the first panax ginseng-macroporous resin eluate to a rotary evaporator for concentration at 80°C to obtain the first panax ginseng-macroporous resin concentrate (60 ml), which is equivalent to 5.0 g crude drug/ml panax ginseng solution;

Load the first panax ginseng-macroporous resin concentrate on a macroporous resin column, and perform the macroporous resin column separation treatment, wherein, the HPD-100 macroporous adsorption resin is selected as the macroporous adsorption resin, and the ratio of the resin volume (300 mL) in the resin column to the weight (dry weight) of panax ginseng is 1: 1 (i.e., if the dry weight of panax ginseng is 1kg, the volume of macroporous resin is 1L; if the dry weight of crude drug is 1g, the volume of macroporous resin is 1 mL); after the concentrated supernatant completely flows into the resin column, wash with ethanol solution at the volume percentage concentration of 60% of 4 times the column volume, and discard the eluate; Then elute with 80% (volume percentage) ethanol solution of 8 times column volume, collect the eluate, and obtain the second panax ginseng-macroporous resin eluate.

With respect to the concentration of panax ginseng concentrate in the process of concentrating treatment in the present invention, other concentrations of per milliliter of concentrate equivalent to 3.5-6 g crude drug (i.e. 3.5-6 g crude drug/ml) are applicable to the present invention in addition to the concentration of per milliliter of concentrate equivalent to 5.0 g crude drug.

In the process of macroporous resin column chromatography of the first panax ginseng-macroporous resin concentrate in the present invention, the ratio of the weight of panax ginseng in the first panax ginseng-macroporous resin concentrate to the volume of the macroporous resin of 1: 0.8-2.5 is applicable to the present invention; In addition to HPD-100, other macroporous adsorption resins such as HPD-200, D203 and XAD-4 are also applicable to the present invention. In the elution process with 60% ethanol solution, the ratio of the amount of 60% ethanol solution to the column volume of the macroporous resin column of 2-4: 1 is applicable to the present invention; In the elution process with 80% ethanol solution, the ratio of the amount of 80% ethanol solution to the column volume of the macroporous resin column of 2-4: 1 is applicable to the present invention.

7. Concentration and Drying Treatment

Concentrate the second panax ginseng-macroporous resin eluate under reduced pressure at 80°C in a rotary evaporator, recover the solvent, and dry the concentrated residue in a drying oven at 85°C to obtain 0.7 g of total panaxadiol saponins.

Panaxadiol saponins are light yellow powder with a special odor. It has good water solubility. The content of panaxadiol saponins prepared was determined using the high performance liquid chromatography in Appendix VI of Volume I, Chinese Pharmacopoeia (2010 Edition), and the content of panaxadiol saponins was 59%.

In the process of concentration of the panax ginseng-macroporous resin eluate, a concentration temperature of 65-95°C is applicable to the present invention; a drying temperature of 70-95°C is applicable to the present invention. The content of panaxadiol saponins prepared by the method in the present invention is 30-70%.

**Exemplary Embodiment 57: Preparation of Forsythin and Phillygenin Composition**

To 1 kg of dry forsythia suspensa leaves, add 10kg of 95% (m/m) ethanol, heat, reflux and extract 2 times (2 h each time), filter, concentrate the filtrate to 1/2 of the original volume under reduced pressure, place at 25°C for 1 h, and allow it to precipitate; Dissolve in methanol, recrystallize, and allow it to precipitate; recrystallize with methanol by repeating the above method, to obtain the amorphous powder of forsythin/phillygenin composition. The contents of forsythin and phillygenin were 98% and 2%, respectively as determined by HPLC.

**Exemplary Embodiment 58: Preparation of Forsythin and Phillygenin Composition**

To 1 kg of dry forsythia suspensa, add 10 kg of methanol, heat, reflux and extract 3 times (4 h each time), filter, concentrate the filtrate to 1/10 of the original volume under reduced pressure, place it at 20°C for 48h, and allow it to precipitate; Dissolve in ethanol, recrystallize, and allow it to precipitate; recrystallize with ethanol by repeating the above method, to obtain the amorphous powder of forsythin/phillygenin composition. The contents of forsythin and phillygenin were 95% and 4%, respectively.

**Exemplary Embodiment 59: Preparation of Forsythin and Phillygenin Composition**

To 1 kg of dry forsythia suspensa leaves, add 10 kg of 70% (m/m) methanol, heat, reflux and extract 3 times (3 h each time), filter, concentrate the filtrate to 1/3 of the original volume under reduced pressure, place at room

temperature for 2 h, and allow it to precipitate; Dissolve in 90% methanol, recrystallize, and allow it to precipitate; recrystallize with methanol by repeating the above method, to obtain the amorphous powder of forsythin/phillygenin composition. The contents of forsythin and phillygenin were 88% and 2%, respectively.

**Exemplary Embodiment 60: Preparation of Forsythin and Phillygenin Composition**

To 1 kg of dry forsythia suspensa, add 10 kg of absolute ethanol, heat, reflux and extract 2 times (4 h each time), filter, concentrate the filtrate to 1/4 of the original volume under reduced pressure, place it at room temperature for 24h, and allow it to precipitate. Dissolve in acetone, recrystallize, and allow it to precipitate; recrystallize with acetone by repeating the above method to obtain the amorphous powder of forsythin/phillygenin composition. The contents of forsythin and phillygenin were 90% and 6%, respectively.

**Exemplary Embodiment 61 Preparation of Forsythin and Phillygenin Composition**

To 1 kg of dry forsythia suspensa leaves, add 10kg of acetone, heat, reflux and extract 3 times (3 h each time), filter, concentrate the filtrate to 1/5 of the original volume under reduced pressure, place it at room temperature for 10 h, and allow it to precipitate; Dissolve in 70% ethanol, recrystallize, and allow it to precipitate; recrystallize with 70% ethanol by repeating the above method to obtain the amorphous powder of forsythin/phillygenin composition. The contents of forsythin and phillygenin were 80% and 5%, respectively.

**Exemplary Embodiment 62-66: Preparation of Composition of Total Ginsenoside- Forsythin and Phillygenin**

Mix the total ginsenoside prepared in Exemplary Embodiment 55 with the forsythin and phillygenin composition prepared in Exemplary Embodiments 57-61 in a weight ratio of 2:98, respectively, to obtain the composition of the total ginsenoside and forsythin.

**Exemplary Embodiments 67-71: Preparation of Panaxadiol Saponins- Forsythin and Phillygenin Composition**

Mix the panaxadiol saponins prepared in Exemplary Embodiment 56 with the forsythin and phillygenin composition prepared in Exemplary Embodiments 57-61 in a weight ratio of 2: 98, respectively, to obtain the composition of Total Ginsenoside and forsythin.

**Exemplary Embodiment 72: Preparation of Composition of Total Ginsenoside- Forsythin**

Mix the total ginsenoside prepared in Exemplary Embodiment 55 with forsythin in a weight ratio of 2: 98 to obtain the total ginsenoside-forsythin composition.

**Exemplary Embodiment 73: Preparation of Panaxadiol Saponins- Forsythin Composition**

Mix the panaxadiol saponins prepared in Exemplary Embodiment 55 with forsythin in a weight ratio of 2: 98 to obtain the panaxadiol saponins - forsythin composition.

**Exemplary Embodiment 74: Preparation of 20(R)-Ginsenoside Rg3- Forsythin Composition**

Mix 20(R)-ginsenoside Rg3 and forsythin in a weight ratio of 2: 98 to obtain the composition of 20(R)-ginsenoside Rg3 and forsythin.

**Exemplary Embodiments 75-88: Preparation of the Composition of Panax Ginseng-forsythia Suspensa Composition and Cyclodextrin**

Take the panax ginseng and forsythia suspensa composition prepared in Exemplary Embodiments 53, 54, 62-74, and prepare a composition according to the following method in the said ratio in Table 2: 1) directly add it to the cyclodextrin solution, or 2) directly add it to the cyclodextrin solution and stir thoroughly for 1-24h, 3) directly add it to the cyclodextrin solution and heat for 10-120 min, 4) directly add it to the cyclodextrin solution and sonicate for 10-120 min, 5) directly grind it with the cyclodextrin powder for 10-120 min, 6) mix the panax ginseng and forsythia suspensa composition with the said cyclodextrin powder well, and sieve; 7) directly add it to the cyclodextrin derivative solution, or 8) directly add it to the cyclodextrin derivative solution and stir thoroughly for 1-24h, 9) directly add it to the cyclodextrin derivative solution and heat for 10-120 min, 10) directly add it to the cyclodextrin derivative solution and sonicate for

10-120 min, 11) directly grind it with the cyclodextrin derivative powder for 10-120 min, 12) mix it well with the cyclodextrin derivative powder, and sieve.

Table 2 Raw Material Ratio and Preparation Method of Exemplary Embodiments 53, 54, 62-74

| Exemplary Embodiments No. | Panax ginseng component and forsythia suspensa component Grams of composition | Grams of cyclodextrin or cyclodextrin derivative | Preparation method |
|---|---|---|---|
| Exemplary Embodiment 75 | 100(2:98) | 100 | 1) Directly add |
| Exemplary Embodiment 76 | 100(2:98) | 10000 | 2) Stir for 1 h |
| Exemplary Embodiment 77 | 100(2:98) | 500 | (5) Heat for 10 min |
| Exemplary Embodiment 78 | 100(2:98) | 1000 | 4) Sonicate for 10 min |
| Exemplary Embodiment 79 | 100(2:98) | 2000 | 5) Grind for 10 min |
| Exemplary Embodiment 80 | 100(2:98) | 3000 | 6) Mix well and sieve for 10 min |
| Exemplary Embodiment 81 | 100(2:98) | 4000 | 1) Directly add |
| Exemplary Embodiment 82 | 100(2:98) | 3500 | 2) Stir for 12 h |
| Exemplary Embodiment 83 | 100(2:98) | 4500 | 3) Heat for 120 min |
| Exemplary Embodiment 84 | 100(2:98) | 1500 | 4) Sonicate for 120 min |
| Exemplary Embodiment 85 | 100(2:98) | 100 | (7) Directly add |
| Exemplary Embodiment 86 | 100(2:98) | 10000 | (8) Stir for 1h |
| Exemplary Embodiment 87 | 100(2:98) | 500 | (9) Heat for 10 min |
| Exemplary Embodiment 88 | 100(2:98) | 1000 | (10) Sonicate for 10 min |

The excipients in Exemplary Embodiments 75-88 are exemplified with selected β-cyclodextrin, and other cyclodextrins and cyclodextrin derivatives are applicable to the present invention. Exemplary Embodiments include 1) β-hydroxypropyl cyclodextrin, 2) hydroxyethyl-β-cyclodextrin, 3) 2,6-dimethyl-β-cyclodextrin, 4) 2,3,6-trimethyl-β-cyclodextrin, 5) 2,6-diethyl-β-cyclodextrin, 6) 2,3,6-triethyl-β-cyclodextrin, 7) maltosyl-β-cyclodextrin, 8) sulfobutylether β-cyclodextrin, 9) p-toluenesulfonyl chloride (p-TsCl) substituted β-cyclodextrin, 10) 6-position substituted β-CD p-toluenesulfonate (P-cyclodextrin-6-OTs), 11) 2-oxohydroxypropyl-β-cyclodextrin, 12) 2-position monosubstituted p-toluenesulfonate (2-β-cyclodextrin-2-OTs), 13) β-cyclodextrin p-toluenesulfonate (tosyl-β-CD) and 14) PCL-(Tos) 7-β-CD, the star-shaped macromolecule of β-cyclodextrin.

**Exemplary Embodiment 89: Preparation of Panax Ginseng-forsythia Suspensa Composition Tablets**

Prepare the tablets of the composition of panax ginseng and forsythia suspensa according to the following ratio:

Composition of panax ginseng and forsythia suspensa (weight ratio of 2: 98) 500 g

(continued)

| | |
|---|---|
| Starch | 480 g |
| Talc | 1%(10 g) |
| Magnesium stearate | 1%(10 g) |

According to the above ratio, mix the composition of panax ginseng and forsythia suspensa prepared in Exemplary Embodiments 53, 54, 62-74 with starch well, granulate, add talc and magnesium stearate and mix well, and compress to make 10000 tablets.

**Exemplary Embodiment 90 Preparation of Panax Ginseng-forsythia Suspensa Composition Granules**

Prepare the granules of the composition of panax ginseng and forsythia suspensa according to the following ratio:

| | |
|---|---|
| Composition of panax ginseng and forsythia suspensa (weight ratio of 2: 98) | 100 g |
| Microcrystalline cellulose | 10000 g |

According to the above ratio, mix the composition of panax ginseng and forsythia suspensa prepared in Exemplary Embodiments 53, 54, 62-74 with microcrystalline cellulose well, granulate, and dispense it into 10000 bags.

**Exemplary Embodiment 91: Preparation of Panax Ginseng-forsythia Suspensa Composition Capsules**

Prepare the capsules of the composition of panax ginseng and forsythia suspensa according to the following ratio:

| | |
|---|---|
| Composition of panax ginseng and forsythia suspensa (weight ratio of 2: 98) | 250 g |
| Starch | 2500 g |

According to the above ratio, mix the composition of panax ginseng and forsythia suspensa prepared in Exemplary Embodiments 53, 54, 62-74 with starch, and fill it into capsules to make 10000 capsules.

**Exemplary Embodiments 92-95: Preparation of Panax Ginseng-forsythia Suspensa Composition Capsules**

Mix the components of the panax ginseng and forsythia suspensa composition with starch well according to the weight ratio in Table 3, and fill it into capsules to make 10000 capsules.

Table 3

| Exemplary Embodiments No. | Raw material (Panax ginseng and forsythia suspensa composition, g) | Excipient (Starch, g) | Weight ratio of raw material to excipient |
|---|---|---|---|
| Exemplary Embodiment 92 | 500(2:98) | 500 | 1:1 |
| Exemplary Embodiment 93 | 50 (10:90) | 5000 | 1:100 |
| Exemplary Embodiment 94 | 250(25:75) | 2500 | 1:10 |
| Exemplary Embodiment 95 | 250(50:50) | 5000 | 1:20 |

The raw materials in the table may be the compositions of panax ginseng and forsythia suspensa in Exemplary Embodiments 53, 54, 62-74, and complexes of panax ginseng components, forsythia suspensa components and cyclodextrins.

**Exemplary Embodiments 96-99: Preparation of Panax Ginseng-forsythia Suspensa Granules**

Mix the components of the panax ginseng and forsythia suspensa composition with microcrystalline cellulose well according to the weight ratio in Table 4, granulate, and dispense it into 10000 bags.

Table 4

| Exemplary Embodiments No. | Raw material (Total ginsenoside and forsythin composition, g) | Excipient (Microcrystalline cellulose, g) | Weight ratio of raw material to excipient |
|---|---|---|---|
| Exemplary Embodiment 96 | 1000(2:98) | 1000 | 1:1 |
| Exemplary Embodiment 97 | 250 (10:90) | 25000 | 1:100 |
| Exemplary Embodiment 98 | 2500(25:75) | 25000 | 1:10 |
| Exemplary Embodiment 99 | 2500(50:50) | 50000 | 1:20 |

The raw materials in the table may be replaced with the compositions of panax ginseng and forsythia suspensa in Exemplary Embodiments 53, 54, 62-74, and complexes of panax ginseng components, forsythia suspensa components and cyclodextrins.

**Exemplary Embodiment 100: Preparation of Panax Ginseng-forsythia Suspensa Composition Tablets**

Prepare the tablets of the composition of panax ginseng and forsythia suspensa according to the following ratio:

| | |
|---|---|
| Composition of panax ginseng and forsythia suspensa (weight ratio of 2: 98) | 500 g |
| Starch | 380 g |
| Licorice extract | 100 g |
| Talc | 1%(10 g) |
| Magnesium stearate | 1%(10 g) |

According to the above ratio, mix the composition of panax ginseng and forsythia suspensa with the above extract powder well, then mix well with starch, granulate, add talc and magnesium stearate and mix well, and compress to make 10000 tablets. The composition of panax ginseng and forsythia suspensa in this exemplary embodiment may be replaced with the compositions of panax ginseng and forsythia suspensa in Exemplary Embodiments 53, 54, 62-74, and complexes of panax ginseng components, forsythia suspensa components and cyclodextrins.

**Exemplary Embodiment 101: Preparation of Panax Ginseng-forsythia Suspensa Composition Granules**

Prepare the granules of the composition of panax ginseng and forsythia suspensa according to the following ratio:

| | |
|---|---|
| Composition of panax ginseng and forsythia suspensa (weight ratio of 2: 98) | 250 g |
| Licorice extract | 250 g |
| Microcrystalline cellulose | 24500 g |

According to the above ratio, mix the composition of panax ginseng and forsythia suspensa with the above extract powder well, then mix well with microcrystalline cellulose, granulate, and dispense it into 10000 bags. The composition of panax ginseng and forsythia suspensa in this exemplary embodiment may be replaced with the compositions of panax ginseng and forsythia suspensa in Exemplary Embodiments 5, 6, 14-40, and complexes of panax ginseng components, forsythia suspensa components and cyclodextrins.

**Exemplary Embodiment 102: Preparation of Panax Ginseng-forsythia Suspensa Composition Capsules**

Prepare the capsules of the composition of panax ginseng and forsythia suspensa according to the following ratio:

| | |
|---|---|
| Composition of panax ginseng and forsythia suspensa (weight ratio of 2:98) | 250 g |
| Licorice extract | 250 g |
| Extract of fritillariae cirrhosae bulbus | 250 g |
| Extract of folium Ilicis latifoliae | 250 g |
| Starch | 1000 g |

According to the above ratio, mix the composition of panax ginseng and forsythia suspensa with the above extract powder well, then mix well with starch, granulate, and fill it into capsules to make 10000 capsules. The composition of panax ginseng and forsythia suspensa in this exemplary embodiment may be replaced with the compositions of panax ginseng and forsythia suspensa in Exemplary Embodiments 53, 54, 62-74, and complexes of panax ginseng components, forsythia suspensa components and cyclodextrins.

**Exemplary Embodiment 103: Preparation of Panax Ginseng-forsythia Suspensa Composition Tablets**

Prepare the tablets of the composition of panax ginseng and forsythia suspensa according to the following ratio:

| | |
|---|---|
| Composition of panax ginseng and forsythia suspensa (weight ratio of 2:98) | 500 g |
| Starch | 480 g |
| Extract of anemarrhenae rhizoma | 500 g |
| Talc | 1%(10 g) |
| Magnesium stearate | 1%(10 g) |

According to the above ratio, mix the composition of panax ginseng and forsythia suspensa with the above extract powder well, then mix well with starch, granulate, add talc and magnesium stearate and mix well, and compress to make 10000 tablets. The composition of panax ginseng and forsythia suspensa in this exemplary embodiment may be replaced with the compositions of panax ginseng and forsythia suspensa as prepared in Exemplary Embodiments 53, 54, 62-74.

**Exemplary Embodiment 104: Preparation of Panax Ginseng-forsythia Suspensa Composition Granules**

Prepare the granules of the composition of panax ginseng and forsythia suspensa according to the following ratio:

| | |
|---|---|
| Composition of panax ginseng and forsythia suspensa (weight ratio of 10: 90) | 1000 g |
| Extract of scrophulariae radix | 500 g |
| Extract lophatherum gracile | 500 g |
| Microcrystalline cellulose | 10000 g |

According to the above ratio, mix the composition of panax ginseng and forsythia suspensa with the above extract powder well, then mix well with microcrystalline cellulose, granulate, and dispense it into 10000 bags. The composition of panax ginseng and forsythia suspensa in this exemplary embodiment may be replaced with the compositions of panax ginseng and forsythia suspensa as prepared in Exemplary Embodiments 53, 54, 62-74.

**Exemplary Embodiment 105: Preparation of Panax Ginseng-forsythia Suspensa Composition Capsules**

Prepare the capsules of the composition of panax ginseng and forsythia suspensa according to the following ratio:

| | |
|---|---|
| Composition of panax ginseng and forsythia suspensa (weight ratio of 6:94) | 2000 g |
| Prunella vulgaris extract | 250 g |
| Houttuyniae Herba Extract | 500 g |

(continued)

| | |
|---|---|
| Phragmitis rhizoma extract | 250 g |
| Starch | 1000 g |

According to the above ratio, mix the composition of panax ginseng and forsythia suspensa with the above extract powder well, then mix well with starch, granulate, and fill it into capsules to make 10000 capsules. The composition of panax ginseng and forsythia suspensa in this exemplary embodiment may be replaced with the compositions of panax ginseng and forsythia suspensa as prepared in Exemplary Embodiments 53, 54, 62-74.

**Exemplary Embodiment 106: Antiviral Test of Panax Ginseng-forsythia Suspensa Composition**

**1 In Vitro Antiviral Test**

**1.1 Test Materials**

(1) Drug

Forsythin (content > 98%), white powder, is manufactured by Dalian Fusheng Natural Drugs Development Co., Ltd. Its purity was 99.5% as determined by HPLC with two detectors: UV detector and evaporative light scattering detector using the area normalization method. The content of forsythin was calibrated and confirmed to be 99.5% using the reference standard of forsythin for assay of pharmaceutical and biological products in China.

20(R)-ginsenoside Rg3 (content > 98%), white powder, is manufactured by Dalian Fusheng Natural Drugs Development Co., Ltd. Its purity was 99.0% as determined by HPLC with two detectors: UV detector and evaporative light scattering detector using the area normalization method. The content of forsythin was calibrated and confirmed to be 99.1% using the reference standard of ginsenoside Rg3 for the assay of pharmaceutical and biological products in China.

Panax ginseng and forsythia suspensa composition: Dalian Fusheng Natural Drugs Development Co., Ltd., calibrated it with the reference standard for assay of pharmaceutical and biological products in China, to determine the contents of ginsenoside Rg3 and forsythin.

Alcohol extract of panax ginseng-forsythia suspensa composition (A; Exemplary Embodiment 53), with the contents of ginsenoside Rg3 and forsythin of 0.15% and 18.6%, respectively;
Water extract of panax ginseng-forsythia suspensa composition (B; Exemplary Embodiment 54), with the contents of ginsenoside Rg3 and forsythin of 0.17% and 17.7%, respectively;
Total ginsenoside-forsythin and phillygenin composition (C; Exemplary Embodiment 62), with the contents of ginsenoside Rg3 and forsythin of 0.04% and 95.6%, respectively;
Panaxadiol saponin-forsythin and phillygenin composition (D; Exemplary Embodiment 67), with the contents of ginsenoside Rg3 and forsythin of 0.12% and 96.6%, respectively;
20(R)-ginsenoside Rg3-forsythin composition (E; Exemplary Embodiment 74): the content of ginsenoside Rg3 and forsythin is 2.0% and 96.6%, respectively.

Ribavirin Injection, colorless, transparent liquid, manufactured by Henan Runhong Pharmaceutical Co., Ltd., batch number: 1206261, CFDA Approval No.: H19993553, 100 mg/ml, as the positive control drug in this study.

Oseltamivir Phosphate, National Institutes for Food and Drug Control. Batch No.: 101096-200901, 100 mg/syringe, as the positive control drug in this study.

The above drugs were dissolved in corresponding reagents, filtered, sterilized and dispensed, and stored at 4°C for later use, and are the drugs to be tested in this study.

(2) Cell strain: Vero (African Green Monkey kidney cells) was preserved by the Basic Medical College of Jilin University.

(3) Virus strains: 1) Influenza virus, parainfluenza virus and respiratory syncytial virus (RSV) strains were purchased from Institute of Virology, Chinese Academy of Preventive Medicine; 2) Coxsackievirus $B_3$ ($CVB_3$) strain: from the United States, preserved by our teaching and research office; 3) Coxsackievirus A16 (CoxA16) and enterovirus 71 (EV71) were gifted by Sendai National Hospital and preserved by our teaching and research office. 4) Adenovirus (AdV): from the Department of Pediatrics, the First Hospital of Norman Bethune Health Science Center; 5) Herpes simplex virus type 1 (HSV-1): purchased from National Institute for the Control of Pharmaceutical and Biological Products, Ministry of Health.

(4) Main equipment and reagents:

Biosafety cabinet: BHC-1300 II A/B3, AIRTECH; $CO_2$ Incubator: MCO-18AIC, SANYO; Inverted microscope: CKX41, OLYMPUS; Electronic analytical balance: AR1140/C, DHAUS; Medium: DMEM, HyClone; Fetal bovine serum: HyClone; Trypsin: Gibco; MTT: Sigma; DMSO: Tianjin Beilian Fine Chemicals Development Co., Ltd.

### 1.2 Test Method

(1) Cell preparation

Vero cells were subcultured for 1-2 days until they reached confluency. When a clear boundary appeared and strong stereoscopic and refractive index were observed, they were digested with trypsin. When pinpoint-like holes appeared on the cell surface, the digestive fluid was aspirated completely. Then, the cells were split up by several milliliters of culture medium, counted, then diluted to about $5\times10^7$/L with culture medium (DMEM containing 10% fetal bovine serum), and inoculated into a 96-well culture plate to form a monolayer.

(2) Determination of drug toxicity

Cytotoxicity test: Dilute the drugs at the concentrations shown in Table 1-1 for cytotoxicity determination.

**Table 1-1 Reference Table for Drug Dilution (in g/L)**

| Drug \ Concentration gradient | Gradient 1 | Gradient 2 | Gradient 3 | Gradient 4 | Gradient 5 | Gradient 6 | Gradient 7 | Gradient 8 |
|---|---|---|---|---|---|---|---|---|
| Forsythin | 1 | 0.5 | 0.25 | 0.125 | 0.0625 | 0.03125 | 0.015625 | 0.078125 |
| 20(R)-Ginsenoside Rg3 | 1 | 0.5 | 0.25 | 0.125 | 0.0625 | 0.03125 | 0.015625 | 0.078125 |
| Composition A | 1 | 0.5 | 0.25 | 0.125 | 0.0625 | 0.03125 | 0.015625 | 0.078125 |
| Composition B | 1 | 0.5 | 0.25 | 0.125 | 0.0625 | 0.03125 | 0.015625 | 0.078125 |
| Composition C | 1 | 0.5 | 0.25 | 0.125 | 0.0625 | 0.03125 | 0.015625 | 0.078125 |
| Composition D | 1 | 0.5 | 0.25 | 0.125 | 0.0625 | 0.03125 | 0.015625 | 0.078125 |
| Composition E | 1 | 0.5 | 0.25 | 0.125 | 0.0625 | 0.03125 | 0.015625 | 0.078125 |
| Ribavirin | 5 | 2.5 | 1.25 | 0.625 | 0.3125 | 0.15625 | 0.078125 | 0.039063 |
| Oseltamivir Phosphate | 2 | 1 | 0.5 | 0.25 | 0.125 | 0.0625 | 0.03125 | 0.015625 |

The above drug that was diluted to different concentrations with the cell maintenance medium (DMEM containing 2% fetal bovine serum) was added dropwise to the monolayer of Vero cells, at 0.2 mL per well, in 9 replicate wells for each concentration. Additionally, prepare 9 wells of normal control (blank control group without drug added) and 9 wells of blank control (medium). Then, the cells were cultured in a 5% $CO_2$ incubator at 37°C. Observation for CPE was performed with an inverted microscope and recorded every day. After 72 h, 20 μL of MTT solution (5 mg/mL) was added to each well, and incubation was continued for 4 h; the medium in each well was aspirated and discarded, and 100 μL of DMSO was added into each well. The plate was shaken for 5 min, the OD value was measured at 492 nm, and the cell survival rate was calculated. Probit regression analysis was performed on the cell viability in the SPSS 18.0 statistical software, and the maximum non-toxic concentration ($TC_0$) and half toxic concentration ($TC_{50}$) of the drug to Vero cells were calculated.

(3) Determination of $TCID_{50}$ of various viruses

The viruses were diluted on a 10-fold basis to different dilutions of $10^{-1}$, $10^{-2}$, $10^{-3}$, $10^{-4}$, $10^{-5}$ and $10^{-6}$ and inoculated in sequence into the monolayer of Vero cells in the 96-well culture plate, at 100 μL per well, in 10 wells for each dilution. Additionally, a normal cell control group was prepared. The plate was incubated in 5% $CO_2$ at 37°C for 2 h, and the virus suspension was discarded. Then, 100 μL of cell maintenance medium was added to each well, and the

plate was incubated in 5% $CO_2$ at 37°C. Observation for cytopathic effect was performed with a microscope from Day 3, and the results were interpreted and recorded on Days 7-8. The highest dilution that could cause 50% of the cell wells to show positive cytopathic effect was used as the endpoint, and the virus titer was calculated by Spearman-karber method.

$$\text{Formula: } \mathrm{LogTCID}_{50} = \mathrm{XM} + \frac{1}{2}\,\mathrm{d}\text{-}\mathrm{d}\,\frac{\sum Pi}{100}$$

($TCID_{50}$: 50% tissue cell infective dose; XM: logarithm of the dilution of the highest dilution of virus concentration; d: logarithm of dilution factor (fold); Σpi: sum of percent cytopathy at each dilution.)

(4) Effect of drugs on viral cytopathic effect

The culture plate covered with cell monolayers was taken, and the medium was aspirated and discarded. The cells were inoculated at the virus challenge amount corresponding to 100TCID50, and cell adhesion was allowed in a 5% $CO_2$ incubator at 37°C for 2 h. Drug solutions of specific concentrations (maximum non-toxic concentration) were added, and incubation was performed in 10 replicate wells for each concentration at 200 μL/well. Ribavirin Injection and Oseltamivir Phosphate were set as positive drug control groups. Meanwhile, a blank control group (without virus and drug added) and virus control group (with virus added but without drug added) were wet up. Observations were made on the effect of drugs on viral CPE. After 72h, the OD value was measured by MTT colorimetric assay at the wavelength of 492 nm, and the effective antiviral rate (ER%) was calculated. In the SPSS 18.0 statistical software, the significant differences were analyzed on effective antiviral rate among drugs by ANOVA.

ER% = (mean OD value of drug treatment group - mean OD value of virus control group)/(mean OD value of cell control group - mean OD value of virus control group) × 100%

**1.3 Test Results**

(1) $TCID_{50}$ of various viruses

Parainfluenza virus:

$$\mathrm{LogTCID}_{50} = -2 + 0.5 - \frac{100+100+50}{100} = -4$$

Influenza virus:

$$\mathrm{LogTCID}_{50} = -2 + 0.5 - \frac{100+100+50}{100} = -4$$

$CVB_3$:

$$\mathrm{LogTCID}_{50} = -2 + 0.5 - \frac{100+100+100+50}{100} = -5$$

HSV-1:

$$\mathrm{LogTCID}_{50} = -2 + 0.5 - \frac{100+100+100+30}{100} = -4.8$$

AdV:

$$\text{LogTCID}_{50}=-2+0.5-\frac{100+100+50}{100}=-4$$

RSV:

$$\text{LogTCID}_{50}=-2+0.5-\frac{100+100+100+50}{100}=-5$$

CoxA16:

$$\text{LogTCID}_{50}=-2+0.5-\frac{100+100+100+50}{100}=-5$$

EV71:

$$\text{LogTCID}_{50}=-2+0.5-\frac{100+100+100+50}{100}=-5$$

**(2) Determination of drug toxicity**

1) Determination of cytotoxicity of drugs

The maximum non-toxic concentration ($TC_0$), half toxic concentration ($TC_{50}$) of each drug on Vero cells and the concentration used for the antiviral test are shown in Table 1-2.

**Table 1-2 Drug Cytotoxicity Test Results (in g/L)**

| Drug / Virus | Forsythin | 20(R)-Ginsenoside Rg3 | Composition A | Composition B |
|---|---|---|---|---|
| Maximum non-toxic concentration | 0.0066 | 0.0048 | 0.026 | 0.018 |
| Half toxic concentration | 0.55 | 0.31 | 0.65 | 0.71 |

| Drug / Virus | Composition C | Composition D | Composition E | Ribavirin | Oseltamivir Phosphate |
|---|---|---|---|---|---|
| Maximum non-toxic concentration | 0.013 | 0.011 | 0.016 | 0.065 | 0.28 |
| Half toxic concentration | 0.71 | 0.73 | 0.75 | 1.392 | 0.832 |

2) Results of drug protection against the viral cytopathic effect

The effective rates of drugs against viruses and the results of one-way analysis of variance by ANOVA method are shown in Table 1-3 and Table 1-4.

**Table 1-3 Effective Antiviral Rates (ER%)-Table 1**

| Drug / Virus | Forsythin | 20(R)-Ginsenoside Rg3 | Composition A | Composition B |
|---|---|---|---|---|
| Influenza virus | 75.38** | 74.06** | 86.82***##▲▲△△● | 89.00***##▲▲△△● |
| Parainfluenza virus | 84.96** | 82.21** | 89.64***#▲ | 89.00***#▲ |
| CoxA16 | 75.08** | 52.33 | 88.24***##▲▲△△△●●● | 83.68***##▲▲△△△●●● |
| RSV | 80.40** | 79.42** | 86.41***#▲△△●●● | 84.32***#▲△△●●● |
| HSV-I | 85.00** | 83.61** | 88.67***#▲△△●● | 86.64***#▲△△●● |
| ADV | 75.14** | 51.07 | 87.51***##▲▲△△△●●● | 89.00***##▲▲△△△●●● |
| EV71 | 84.85** | 74.66** | 88.34***#▲▲△△△●●● | 89.00***#▲▲△△△●●● |
| $CVB_3$ | 75.27** | 53.75 | 81.28**##▲▲△△△●●● | 85.67**##▲▲△△△●●● |

**Table 1-4 Effective Antiviral Rates (ER%)-Table 2**

| Drug / Virus | Composition C | Composition D | Composition E | Ribavirin | Oseltamivir Phosphate |
|---|---|---|---|---|---|
| Influenza virus | 90.00***##▲▲△△● | 100.00***##▲▲△△● | 96.82***##▲▲△△● | 57.49** | 81.76** |
| Parainfluenza virus | 90.00***#▲ | 100.00***#▲ | 99.64***#▲ | 91.56** | 94.52** |
| CoxA16 | 88.68***##▲▲△△△●●● | 97.68***##▲▲△△△●●● | 98.24***##▲▲△△△●●● | 0.70 | 2.95 |
| RSV | 86.32***#▲△△●●● | 95.32***#▲△△●●● | 96.41***#▲△△●●● | 50.08* | 37.60 |
| HSV-I | 92.64***#▲△△●● | 99.64***#▲△△●● | 98.67***#▲△△●● | 62.92** | 66.56** |
| ADV | 90.00***##▲▲△△△●●● | 100.00***##▲▲△△△●●● | 97.51***##▲▲△△△●●● | 0.43 | 10.31 |
| EV71 | 90.00***#▲▲△△△●●● | 100.00***#▲▲△△△●●● | 98.34***#▲▲△△△●●● | 4.25 | 51.86 |
| $CVB_3$ | 86.67**##▲▲△△△●●● | 92.67**##▲▲△△△●●● | 91.28**##▲▲△△△●●● | 13.44 | 1.64 |

Note: * P < 0.05, **P < 0.01 compared with the virus control group; #P < 0.05, ##P < 0.01 for the comparison between panax ginseng-forsythia suspensa composition groups and forsythin; ▲P < 0.05, ▲▲P < 0.01 for the comparison between panax ginseng-forsythia suspensa composition groups and ginsenoside Rg3; △P <0.05, △△P <0.01, △△△P <0.001 for the comparison between panax ginseng-forsythia suspensa composition groups and ribavirin; •P < 0.05, ••P < 0.01, •••P < 0.001 for the comparison between panax ginseng-forsythia suspensa composition groups vs. oseltamivir phosphate.

Tables 1-3 and 1-4 show that the panax ginseng-forsythia suspensa composition groups significantly inhibited 8 kinds of viruses (P < 0.01 or P < 0.001). The effective rates of influenza virus, parainfluenza virus, herpes simplex virus type I (HSV-I), enterovirus 71 and adenovirus (ADV) reached 100%, and the curative effect was significantly better than that of forsythin and ginsenoside Rg3, indicating that the panax ginseng-forsythia suspensa composition groups had a synergistic effect. In addition, the inhibitory effects of panax ginseng-forsythia suspensa composition groups on influenza, coxsackievirus A16 (CoxA16), respiratory syncytial virus (RSV), herpes simplex virus type I (HSV-I), adenovirus (ADV), enterovirus 71 (EV71) and coxsackievirus $B_3$ ($CVB_3$) were significantly better than positive drug ribavirin (P < 0.01 or P < 0.001). The inhibitory effects on influenza, coxsackievirus A16 (CoxA16), respiratory syncytial virus (RSV), herpes simplex virus type I (HSV-I), adenovirus (ADV), enterovirus 71 and coxsackievirus $B_3$ ($CVB_3$) were significantly better than oseltamivir phosphate (P < 0.05 or P < 0.01, P < 0.001).

## 2. In Vivo Antiviral Test

### 2.1 Experimental Materials

#### (1) Experimental animals

Kunming mice were provided by Laboratory Animal Center, Norman Bethune Health Science Center, Jilin University, YDZ No. 10-5219.

#### (2) Test instruments and reagents

| Name of the Instrument | Model | Manufacturer |
|---|---|---|
| Quantitative PCR instrument | 7300 | ABI |
| PCR instrument | ES-60J | Shenyang Longteng Electronic Weighing Instrument Co., Ltd. |
| Electronic analytical balance | FA1004 | Shenyang Longteng Co., Ltd. |
| $CO_2$ incubator | HG303-5 | Nanjing Laboratory Instrument Factory |
| Ultra-clean workbench | SW-CJ-IF | Suzhou Antai Air Technology Co., Ltd. |
| Inverted microscope | CKX41 | Olympus Instrument |
| -80°C ultra-low temperature freezer | TECON-5082 | Australia |
| Water bath oscillator | HZS-H | Harbin Donglian Co., Ltd. |
| Plate reader | TECAN A-5082 | Australia |
| Spectrophotometer | Model 7550 | Japan |

### 2.2 Experimental Method

#### (1) Determination of median lethal dose of influenza virus and parainfluenza virus in mice

Influenza virus and parainfluenza virus (cell lysate) were serially diluted at 10-fold to the concentrations of $10^{-1}$, $10^{-2}$, $10^{-3}$, $10^{-4}$ and $10^{-5}$. One hundred and twenty Kunming mice were divided into the influenza virus group (n=60) and parainfluenza virus group (n=60), each of which was divided into 6 groups randomly. The mice were lightly anesthetized with ether and intranasally infected with a virus solution of different dilutions at 0.03 mL/animal. Meanwhile, a blank control was set up, where the virus suspension was replaced with normal saline. Observation for mortality and survival was performed daily until 14 days after infection. The death within 24 h of infection was non-specific death, which was not counted. The $LD_{50}$ of virus solution was calculated by Karber's method. Calculation formula: $\mathrm{LogLD_{50}} = \mathrm{XM} + \frac{1}{2}\mathrm{d} - \mathrm{d}\frac{\sum Pi}{100}$ [where: $LD_{50}$: median lethal dose; XM: logarithm of the highest dilution of virus concentration; d: logarithm of dilution factor (fold); and $\Sigma$pi: sum of percent cytopathy at each dilution].

#### (2) Study on the effect of panax ginseng-forsythia suspensa composition against the pulmonitis induced by influenza virus and parainfluenza virus infection

##### 1) Test animals and grouping

Five hundred and forty-four-week-old Kunming mice were selected for two tests.

Firstly, 270 mice were randomly divided into 27 groups, with 10 mice in each group, and used to determine the lung index and lung index inhibition rate of panax ginseng-forsythia suspensa composition on influenza and parainfluenza virus-infected mice. Ninety mice were selected for each test, and the test was repeated 3 times. Another 270 mice were randomly divided into 27 groups, with 10 mice in each group, which were used to determine the virus hemagglutination titers in lung suspension of panax ginseng-forsythia suspensa composition groups. Ninety mice were selected for each test, and the test was repeated 3 times.

**2) Method of infection**

A mass of absorbent cotton was put in a 200-300 mL beaker, then an appropriate amount of ether was poured (to wet the absorbent cotton), the beaker loaded with absorbent cotton was inverted, and a mouse was placed in the beaker for anesthesia. When the mouse appeared to be extremely excited and then obviously weak, the mouse was placed in a supine position and infected intranasally with 15$LD_{50}$ influenza virus and parainfluenza virus at 0.03ml/nostril. For the blank control group, the virus suspension was replaced with normal saline.

**3) Method of administration and dosage**

The panax ginseng-forsythia suspensa composition groups, ginsenoside Rg3 group, forsythin group, ribavirin and oseltamivir phosphate control group were administrated by gavage on the day before infection, respectively. Panax ginseng-forsythia suspensa composition groups were divided into high, middle and low dose groups, and the dosage was 13.0, 6.5 and 3.25 mg/kg, respectively. The dosage was 13mg/kg for the forsythin group, 13mg/kg for the ginsenoside Rg3 group, 58.5 mg/kg for the ribavirin positive drug group and 19.5 mg/kg for the oseltamivir phosphate group, once daily for 5 days. The blank control and virus control groups were administrated by gavage with the same volume of normal saline.

**4) Observation indicators**

**1) Determination of lung index**

On the 5th day after drug administration, the mice were fasted for 8 h, weighed and sacrificed by eyeball removal and exsanguination. The thorax was opened, and the whole lungs were removed, washed twice with normal saline, and the surface was dried with filter paper, and the lungs were weighed by electronic balance. The lung index and lung index inhibition rate were calculated according to the following formulas:

$$\text{Lung index} = (\text{mouse lung weight}/\text{mouse body weight}) \times 100\%$$

$$\text{Lung index inhibition rate} = (\text{mean lung index of infection model group - mean lung index of test group})/\text{mean lung index of infection model group} \times 100\%$$

**2) Determination of virus hemagglutination titer in lung suspension**

The lungs of mice in each group were removed on the 5th day after treatment and ground into homogenate with a homogenizer at a low temperature. The homogenate was diluted into 10% lung tissue suspension with normal saline, which was then centrifuged, and the supernatant was taken and diluted on a fold basis. The diluted supernatant was dropped into a titration plate at 0.2 ml/well, and 0.2 ml of 1% chicken red blood cell suspension was added into each well, and the mixture was mixed well and kept at room temperature for 30 min. The hemagglutination titer was observed and recorded. The endpoint was the agglutination of the red blood cells (++), and the titer was expressed by the dilution factor of the suspension.

**2.3 Test Results and Analysis**

**(1) Determination results of the median lethal dose of influenza virus and parainfluenza virus in mice**

Kunming mice in the test group were intranasally infected with 30 $\mu$L of influenza virus and parainfluenza virus at different concentrations, respectively. The first 3 groups (virus concentrations of $10^{-1}$, $10^{-2}$ and $10^{-3}$) showed symptoms of varying degrees on day 3 of infection: piloerection, trembling, and reduced food consumption, etc.; On day 5, the mice appeared to wobble while walking. Death was observed in the mice in the highest virus concentration group on day 6, and deaths in the other groups were observed successively on day 7 after infection. At the end of 14-day observation, the number of dead mice in each group was counted, and the results are shown in Tables 1-4 and 1-5 below. The $LD_{50}$ was calculated as a dilution of $10^{-2.9}$ for the influenza virus and a dilution of $10^{-2.5}$ for the parainfluenza virus.

**Table 1-4 Summary of Median Lethal Dose Test Results of Influenza Virus**

| Influenza virus group | Cumulative deaths | Cumulative survival | Cumulative death rate |
|---|---|---|---|
| Group 10-1 | 9 | 1 | 90% |
| Group 10-2 | 7 | 3 | 70% |
| Group 10-3 | 4 | 6 | 40% |
| Group 10-4 | 3 | 7 | 30% |
| Group 10-5 | 1 | 9 | 10% |
| Blank control group | 0 | 10 | 0% |

The $LD_{50}$ of virus was calculated by the Karber method. The $LogLD_{50}$ of influenza virus is as follows:

$$LogLD_{50}=XM+\frac{1}{2}d-d\frac{\sum Pi}{100}=-1+0.5-(80\%+60\%+40\%+20\%+0\%+0\%)=-2.9$$

**Table 1-5 Summary of Median Lethal Dose Test Results of Parainfluenza Virus**

| Parainfluenza virus group | Cumulative deaths | Cumulative survival | Cumulative death rate |
|---|---|---|---|
| Group 10-1 | 8 | 2 | 80% |
| Group 10-2 | 6 | 4 | 60% |
| Group 10-3 | 4 | 6 | 40% |
| Group 10-4 | 2 | 8 | 20% |
| Group 10-5 | 0 | 10 | 0% |
| Blank control group | 0 | 10 | 0% |

The $LD_{50}$ of virus was calculated by the Karber method. $LogLD_{50}$ of parainfluenza virus is as follows:

$$LogLD_{50} = XM + \frac{1}{2}d-d\frac{\sum Pi}{100} =-1 +0.5-(90\% +70\% +40\% +30\% +10\% +0\%) =-2.5$$

**(2) Results of the effect of panax ginseng-forsythia suspensa composition against the pulmonitis induced by influenza virus and parainfluenza virus infection**

**1) Determination of lung index**

The mean lung index of mice infected with influenza virus and parainfluenza virus showed that compared with the infection model group, the lung index of the blank control group, forsythin group (13.0 mg/kg/d), ginsenoside Rg3 group (16.0 mg/kg/d), panax ginseng-forsythia suspensa composition groups (low dose group 3.25 mg/kg/d, middle dose group 6.5 mg/kg/d, high dose group 13.0 mg/kg/d), ribavirin group and oseltamivir phosphate group decreased significantly ($P < 0.05$ or $P < 0.01$). The panax ginseng-forsythia suspensa composition groups had a significant protective effect within the concentration range of 3.25-13.0 mg/kg/d, with significantly reduced lung index and significantly better inhibitory rate on lung tissue lesion index than that of the forsythin group and ginsenoside Rg3 group ($P < 0.01$ or $P < 0.05$). See Tables 1-6 and 1-7 for the results.

**Table 1-6 Inhibitory Rate of Panax Ginseng-forsythia Suspensa Composition on Lung Index of Mice Infected with Influenza Virus (n=3)**

| Group | Drug dose (mg/kg/day) | Lung index ($\bar{X}$ ±S) | Lung index Inhibitory rate (%) | P-value | P-value |
|---|---|---|---|---|---|
| Blank control group | 0 | 1.274±0.102 | - | | |
| Virus control group | 0 | 1.488±0.084 | - | | |
| Ribavirin | 58.5 | 1.281±0.061 | 13.90 | *<0.05 | |

(continued)

| Group | | Drug dose (mg/kg/day) | Lung index ($\overline{X}$ ±S) | Lung index Inhibitory rate (%) | P-value | P-value |
|---|---|---|---|---|---|---|
| Oseltamivir Phosphate | | 19.5 | 1.178±0.066 | 19.84 | **<0.01 | |
| Forsythin | | 13.0 | 1.280±0.040 | 14.00 | *<0.05 | |
| 20(R)-Ginsenoside Rg3 | | 13.0 | 1.302±0.046 | 12.51 | *<0.05 | |
| Composition A | High dose group | 13.0 | 1.078±0.042 | 28.10 | **<0.01 | ##▲▲<0.01 |
| | Medium dose group | 6.5 | 1.143±0.033 | 24.00 | **<0.01 | ▲#<0.05 |
| | Low dose group | 3.25 | 1.191±0.025 | 19.56 | **<0.01 | ▲#<0.05 |
| Composition B | High dose group | 13.0 | 1.071±0.046 | 29.52 | **<0.01 | ##▲▲<0.01 |
| | Medium dose group | 6.5 | 1.133±0.021 | 24.15 | **<0.01 | ▲#<0.05 |
| | Low dose group | 3.25 | 1.142±0.027 | 20.40 | **<0.01 | ▲#<0.05 |
| Composition C | High dose group | 13.0 | 1.074±0.048 | 29.16 | **<0.01 | ##▲▲<0.01 |
| | Medium dose group | 6.5 | 1.124±0.031 | 25.14 | **<0.01 | ▲#<0.05 |
| | Low dose group | 3.25 | 1.174±0.029 | 20.56 | **<0.01 | ▲#<0.05 |
| Composition D | High dose group | 13.0 | 1.049±0.056 | 29.52 | **<0.01 | ##▲▲<0.01 |
| | Medium dose group | 6.5 | 1.129±0.041 | 24.15 | **<0.01 | ▲#<0.05 |
| | Low dose group | 3.25 | 1.184±0.039 | 20.40 | **<0.01 | ▲#<0.05 |
| Composition E | High dose group | 13.0 | 1.070±0.056 | 28.10 | **<0.01 | ##▲▲<0.01 |
| | Medium dose group | 6.5 | 1.131±0.041 | 24.00 | **<0.01 | ▲#<0.05 |

(continued)

| Group | | Drug dose (mg/kg/day) | Lung index ($\bar{X}$ ±S) | Lung index Inhibitory rate (%) | P-value | P-value |
|---|---|---|---|---|---|---|
| | Low dose group | 3.25 | 1.197±0.039 | 19.56 | **<0.01 | ▲#<0.05 |

*P < 0.05, **P < 0.01 for the comparison between each test group and the virus control group; #P < 0.05, ##P < 0.01 for the comparison between panax ginseng-forsythia suspensa composition and forsythin; ▲P < 0.05, ▲▲P < 0.01 for the comparison between panax ginseng-forsythia suspensa composition and ginsenoside Rg3.

**Table 1-7 Inhibitory Rate of Panax Ginseng-forsythia Suspensa Composition on Lung Index of Mice Infected with Parainfluenza Virus (n=3)**

| Group | | Drug dose (mg/kg/day) | Lung index ($\bar{X}$ ±S) | Lung index Inhibitory rate (%) | P-value | P-value |
|---|---|---|---|---|---|---|
| Blank control group | | 0 | 1.305±0.031 | - | | |
| Virus control group | | 0 | 1.591±0.062 | - | | |
| Ribavirin | | 58.5 | 1.340±0.065 | 15.76 | *<0.05 | |
| Oseltamivir Phosphate | | 19.5 | 1.243±0.054 | 21.85 | *<0.01 | |
| Forsythin | | 13.0 | 1.335±0.062 | 16.10 | *<0.01 | |
| 20(R)-Ginsenoside Rg3 | | 13.0 | 1.357±0.050 | 14.69 | *<0.01 | |
| | High dose group | 13.0 | 1.086±0.023 | 26.74 | *<0.01 | ##▲▲<0.01 |
| Composition A | Medium dose group | 6.5 | 1.136±0.047 | 25.37 | *<0.01 | #▲▲<0.05 |
| | Low dose group | 3.25 | 1.174±0.036 | 23.29 | *<0.01 | #▲<0.05 |
| | High dose group | 13.0 | 1.094±0.067 | 26.87 | *<0.01 | ##▲▲<0.01 |
| Composition B | Medium dose group | 6.5 | 1.163±0.045 | 25.43 | *<0.01 | #▲▲<0.05 |
| | Low dose group | 3.25 | 1.181±0.051 | 22.98 | *<0.01 | #▲<0.05 |
| | High dose group | 13.0 | 1.127±0.033 | 29.21 | *<0.01 | ##▲▲<0.01 |
| Composition C | Medium dose group | 6.5 | 1.142±0.034 | 26.81 | *<0.01 | #▲▲<0.05 |
| | Low dose group | 3.25 | 1.197±0.052 | 23.62 | *<0.01 | #▲<0.05 |
| | High dose group | 13.0 | 1.068±0.058 | 32.87 | *<0.01 | ##▲▲<0.01 |

(continued)

| Group | | Drug dose (mg/kg/day) | Lung index ($\overline{X}$ ±S) | Lung index Inhibitory rate (%) | P-value | P-value |
|---|---|---|---|---|---|---|
| Composition D | Medium dose group | 6.5 | 1.143±0.065 | 28.13 | *<0.01 | #▲▲<0.05 |
| | Low dose group | 3.25 | 1.177±0.044 | 26.01 | *<0.01 | #▲<0.05 |
| Composition E | High dose group | 13.0 | 1.101±0.058 | 30.79 | *<0.01 | ##▲▲<0.01 |
| | Medium dose group | 6.5 | 1.158±0.065 | 27.22 | *<0.01 | #▲▲<0.05 |
| | Low dose group | 3.25 | 1.188±0.044 | 25.30 | *<0.01 | #▲<0.05 |

*P < 0.05, **P < 0.01 for the comparison between each test group and the virus control group; #P < 0.05, ##P < 0.01 for the comparison between panax ginseng-forsythia suspensa composition and forsythin, ▲P < 0.05 for the comparison between panax ginseng-forsythia suspensa composition and ginsenoside Rg3.

**2) Determination of virus hemagglutination titer in lung suspension**

The hemagglutination titers (InX) of the virus in lung tissues in the infection model group were 32.40 and 33.11, respectively, after infection with influenza virus and parainfluenza virus in mice. The hemagglutination titers of virus in lung tissues were decreased in the panax ginseng-forsythia suspensa composition groups at different concentrations after 5 days of treatment, and the difference was significant when compared with the infection model group (P < 0.01). The hemagglutination titers of influenza virus and parainfluenza virus with different doses of panax ginseng-forsythia suspensa composition groups were significantly lower than those in the forsythin group and ginsenoside Rg3 group (P < 0.05 to P < 0.001), indicating that the composition had a synergistic effect. The inhibitory rates of the panax ginseng-forsythia suspensa composition groups against virus proliferation were significantly higher than those of the forsythin group and ginsenoside Rg3 group (P < 0.05 to P < 0.001). The inhibitory rates of the high, middle and low dose groups of panax ginseng-forsythia suspensa composition against the hemagglutination titer of the lung suspension of mice infected with influenza virus were significantly higher than those of the Forsythin group and ginsenoside Rg3 group (P < 0.01 to P < 0.001). The results of the above tests are shown in Tables 1-8 and 1-9.

**Table 1-8 Effect of Panax Ginseng-forsythia Suspensa Composition on Hemagglutination Titer of Lung Suspension of Mice Infected with Influenza Virus (n=3)**

| Group | | Drug dose (mg/kg/day) | Hemagglutinin titer (InX) | Inhibitory rate (%) | P-value | P-value |
|---|---|---|---|---|---|---|
| Blank control group | | 0 | 0 | | | |
| Virus control group | | 0 | 32.40±1.105 | | | |
| Ribavirin | | 58.5 | 21.91±1.050 | 32.39 | *<0.01 | |
| Oseltamivir Phosphate | | 19.5 | 20.50±1.123 | 36.73 | *<0.01 | |
| Forsythin | | 13.0 | 22.06±1.120 | 31.90 | *<0.01 | |
| 20(R)-Ginsenoside Rg3 | | 13.0 | 22.61±1.059 | 30.22 | *<0.01 | |
| Composition A | High dose group | 13.0 | 18.70±0.428 | 43.93 | *<0.01 | ###▲▲▲<0.001 |
| | Medium dose group | 6.5 | 20.53±0.577 | 40.02 | *<0.01 | ##▲▲<0.01 |

(continued)

| Group | | Drug dose (mg/kg/day) | Hemagglutinin titer (lnX) | Inhibitory rate (%) | P-value | P-value |
|---|---|---|---|---|---|---|
| | Low dose group | 3.25 | 21.03±1.460 | 32.58 | *<0.01 | #▲<0.05 |
| | High dose group | 13.0 | 18.52±0.572 | 43.57 | *<0.01 | ###▲▲▲<0.001 |
| Composition B | Medium dose group | 6.5 | 20.75±0.289 | 40.31 | *<0.01 | ##▲▲<0.01 |
| | Low dose group | 3.25 | 21.21±1.398 | 32.67 | *<0.01 | #▲<0.05 |
| | High dose group | 13.0 | 18.84±0.364 | 44.67 | *<0.01 | ###▲▲▲<0.001 |
| Composition C | Medium dose group | 6.5 | 20.37±0.484 | 37.25 | *<0.01 | ##▲▲<0.01 |
| | Low dose group | 3.25 | 21.71±1.217 | 33.41 | *<0.01 | #▲<0.05 |
| | High dose group | 13.0 | 17.86±0.491 | 43.75 | *<0.01 | ###▲▲▲<0.001 |
| Composition D | Medium dose group | 6.5 | 18.35±0.397 | 41.33 | *<0.01 | ##▲▲<0.01 |
| | Low dose group | 3.25 | 21.03±1.227 | 36.59 | *<0.01 | #▲<0.05 |
| | High dose group | 13.0 | 17.70±0.618 | 45.36 | *<0.01 | ###▲▲▲<0.001 |
| Composition E | Medium dose group | 6.5 | 19.21±0.450 | 40.72 | *<0.01 | ##▲▲<0.01 |
| | Low dose group | 3.25 | 20.63±1.439 | 36.08 | *<0.01 | #▲<0.05 |

**Table 1-9 Effect of Panax Ginseng-forsythia Suspensa Composition on Hemagglutination Titer of Lung Suspension of Mice Infected with Parainfluenza Virus (n=3)**

| Group | | Drug dose (mg/kg/day) | Hemagglutinin titer (lnX) | Inhibitory rate (%) | P-value | P-value |
|---|---|---|---|---|---|---|
| Blank control group | | 0 | 0 | | | |
| Virus control group | | 0 | 33.11±1.210 | | | |
| Ribavirin | | 58.5 | 23.22±1.091 | 29.86 | *<0.05 | |
| Oseltamivir Phosphate | | 19.5 | 22.05±1.055 | 33.40 | *<0.01 | #<0.05 |
| Forsythin | | 13.0 | 23.17±1.059 | 30.01 | *<0.01 | #<0.05 |
| 20(R)-Ginsenoside Rg3 | | 13.0 | 23.79±1.072 | 28.15 | *<0.01 | #>0.05 |
| | High dose group | 13.0 | 20.11±0.151 | 43.38 | *<0.01 | ###▲▲▲<0.001 |
| Composition A | Medium dose group | 6.5 | 21.85±0.550 | 38.41 | *<0.01 | ##▲▲▲<0.01 |
| | Low dose group | 3.25 | 22.81±0.547 | 35.67 | *<0.01 | ##▲▲<0.01 |
| | High dose group | 13.0 | 19.23±0.480 | 43.17 | *<0.01 | ###▲▲▲<0.001 |

(continued)

| Group | | Drug dose (mg/kg/day) | Hemagglutinin titer (lnX) | Inhibitory rate (%) | P-value | P-value |
|---|---|---|---|---|---|---|
| Composition B | Medium dose group | 6.5 | 20.97±0.543 | 40.80 | *<0.01 | ##▲▲▲<0.01 |
| | Low dose group | 3.25 | 22.04±0.457 | 35.98 | *<0.01 | ##▲▲<0.01 |
| Composition C | High dose group | 13.0 | 18.97±0.261 | 43.98 | *<0.01 | ###▲▲▲<0.001 |
| | Medium dose group | 6.5 | 20.80±0.274 | 39.87 | *<0.01 | ##▲▲▲<0.01 |
| | Low dose group | 3.25 | 21.07±0.613 | 36.11 | *<0.01 | ##▲▲<0.01 |
| Composition D | High dose group | 13.0 | 17.38±0.955 | 47.50 | *<0.01 | ###▲▲▲<0.001 |
| | Medium dose group | 6.5 | 19.04±0.501 | 42.49 | *<0.01 | ##▲▲▲<0.01 |
| | Low dose group | 3.25 | 20.36±0.824 | 38.52 | *<0.01 | ##▲▲<0.01 |
| Composition E | High dose group | 13.0 | 17.97±0.955 | 45.73 | *<0.01 | ###▲▲▲<0.001 |
| | Medium dose group | 6.5 | 19.69±0.501 | 40.52 | *<0.01 | ##▲▲▲<0.01 |
| | Low dose group | 3.25 | 20.81±0.824 | 37.15 | *<0.01 | ##▲▲<0.01 |

In Tables 1-8 and 1-9, *P < 0.05, **P < 0.01 for the comparison between each test group and the virus control group; #P < 0.05, ##P < 0.01, ###P < 0.001 for the comparison between panax ginseng-forsythia suspensa composition and forsythin; ▲P < 0.05, ▲▲P < 0.01, ▲▲▲P < 0.001 for the comparison between panax ginseng-forsythia suspensa composition groups and ginsenoside Rg3.

**Exemplary Embodiment 107 Antipyretic and Anti-inflammatory Test of Panax Ginseng and Forsythia Suspensa composition**

**1.1 Test Materials**

**(1) Test animals** Wistar rats, weighing 120-250 g, male and female, certificate No.: YDZ No. 13-1225; Japanese big-ear white rabbits, male, weighing 1.5 ~ 2.0 kg. Certificate No.: YDZ No. 10-5115, all provided by Changchun High-tech Medicine Animal Experimental Research Centre. Animal feed was provided by Laboratory Animal Department of Jilin University.

**(2) Test Drugs**

Forsythin (content > 98%), white powder, is manufactured by Dalian Fusheng Natural Drugs Development Co., Ltd. Its purity was 99.5% as determined by HPLC with two detectors: UV detector and evaporative light scattering detector using the area normalization method. The content of forsythin was calibrated and confirmed to be 99.5% using the reference standard of forsythin for the assay of pharmaceutical and biological products in China.

20(R)-ginsenoside Rg3 (content > 98%), white powder, is manufactured by Dalian Fusheng Natural Drugs Development Co., Ltd. Its purity was 99.0% as determined by HPLC with two detectors: UV detector and evaporative light scattering detector using the area normalization method. The content of forsythin was calibrated and confirmed to be 99.1% using the reference standard of ginsenoside Rg3 for the assay of pharmaceutical and biological products in China.

Panax ginseng and forsythia suspensa composition: Dalian Fusheng Natural Drugs Development Co., Ltd., calibrated with the reference standard for assay of pharmaceutical and biological products in China, to determine the contents of ginsenoside Rg3 and forsythin.

Alcohol extract of panax ginseng-forsythia suspensa composition (A; Exemplary Embodiment 53), with the contents of ginsenoside Rg3 and forsythin of 0.15% and 18.6%, respectively;
Water extract of panax ginseng-forsythia suspensa composition (B; Exemplary Embodiment 54), with the contents of ginsenoside Rg3 and forsythin of 0.17% and 17.7%, respectively;
Total ginsenoside-forsythin and phillygenin composition (C; Exemplary Embodiment 62), with the contents of ginsenoside Rg3 and forsythin of 0.04% and 95.6%, respectively;
Panaxadiol saponin-forsythin and phillygenin composition (D; Exemplary Embodiment 67), with the contents of ginsenoside Rg3 and forsythin of 0.12% and 96.6%, respectively;
20(R)-ginsenoside Rg3-forsythin composition (E; Exemplary Embodiment 74): the content of ginsenoside Rg3 and forsythin is 2.0% and 96.6%, respectively.

### 1.2 Main Instruments and Reagents

YLS-7A rat toe swelling measuring instrument: Equipment Station of Shandong Academy of Medical Sciences; 722 visible spectrophotometer: manufactured by Shanghai Spectrum Instruments Co., Ltd.; Portable digital thermometer: model WSC-411P, Shanghai Pudong No.3 Factory; Pilocarpine: Tianjin People's Pharmaceutical Factory, batch No.: 20130112; Histamine: Shanghai Institute of Biochemistry, batch No.: 20130115; 5-Hydroxytryptamine: Shanghai Institute of Biochemistry, batch No.: 20130623; Evans Blue: Shanghai Chemical Reagent Purchasing and Supply Station, batch No.: 20130217; Chlorpheniramine Tablets: Changchun Economic Development Zone Pharmaceutical Co., Ltd., batch No.: 20130801; Carrageenan: Jilin Institute of Medicine, batch No.: 20130502; Paracetamol Tablets: Liaoyuan Baikang Pharmaceutical Co., Ltd., batch No.: 20130512; Aspirin Tablets: Baicheng Wanda Pharmaceutical Co., Ltd., batch No.: 20130305; Beer yeast: Beijing Aoboxing Biotech Co., Ltd., batch No.: 2013020; Typhoid and paratyphoid vaccines: Changchun Institute of Biological Products, batch No.: 20130216.

### 1.3 Statistical Treatment

Rank sum test, $X^2$ test and t test were used for statistical analysis.

### 2.1 Study on the Effect of the Panax Ginseng and Forsythia Suspensa Composition on Sweat Secretion of Rat Paw (Staining Method)

#### (1) Materials and Methods

There are sweat glands on the plantar skin of rats, so the changes in sweat secretion can be observed by the purple reaction between the iodine-starch mixture and the sweat.

Five hundred Wistar rats, half male and half female, weighing 120-150 g, were taken in the study. They were randomly divided into 50 groups according to body weight and sex, namely the control (0.5% carboxymethylcellulose) group, forsythin group, ginsenoside Rg3 group, low, middle and high dose groups of panax ginseng-forsythia suspensa composition (2.5, 5 and 10 mg/kg, respectively) and positive drug pilocarpine (35 mg/kg) group, with 10 rats in each group, and 10 groups for each test, for totally 5 time periods of testing (1, 5, 10, 15 and 20 min). The rats were placed in a homemade rat-fixing bag to expose both hind limbs. The dirt on the right paw was gently scrubbed with a cotton swab dipped in absolute ethanol. Except for the pilocarpine solution, which was given by subcutaneous injection, other groups were administered by gavage. One hour after administration (30 min after administration in the pilocarpine group), the sweat secreted from the right paw of rats in each group was gently cleaned with a dry cotton swab before the study and during struggling. The right paw was coated with Wotan-Takagaki's reagent A solution (dissolve 2g of iodine in 100 mL of absolute ethanol) and then coated with Wotan-Takagaki's reagent B solution (mix 50 g of soluble starch and 100 mL of Ricinus communis oil well) after reagent A was fully absorbed. The color and number of dark purple spots (i.e. sweat spots) were observed carefully with a magnifying glass at 1, 5, 10, 15 and 20 min, respectively, after applying solution B. At the end of the test, a rank-sum test was used to analyze the differences among the groups.

#### (2) Results

Compared with the control group, the middle and high dose groups (5 and 10 mg/kg) of panax ginseng-forsythia suspensa composition could significantly promote the sweat secretion of rat paw at 10, 15 and 20 min after applying solution B ($p < 0.05$), and the 2.5 mg/kg group of panax ginseng-forsythia suspensa composition could significantly promote the sweat secretion of rat paw at 15 and 20 min after application of solution B ($p < 0.05$). The sweating effect was comparable with the positive drug pilocarpine, and the panax ginseng-forsythia suspensa composition could slowly promote the rat paw's sweat secretion. The high dose group of the panax ginseng-forsythia suspensa composition was

significantly superior to forsythin and ginsenoside Rg3 ($p < 0.05$) in promoting sweat secretion of rat paw following 10, 15 and 20 min after applying solution B. The middle dose group of the panax ginseng-forsythia suspensa composition was significantly superior to forsythin and ginsenoside Rg3 ($p < 0.05$) in promoting sweat secretion of rat paw following 10 and 15 after application of solution B. The low dose group of the panax ginseng-forsythia suspensa composition was significantly superior to forsythin and ginsenoside Rg3 ($p < 0.05$) in promoting sweat secretion of rat paw following 15min after application of B solution. The above test results show that the effect of panax ginseng-forsythia suspensa composition groups was significantly superior to that of forsythin and ginsenoside Rg3 in promoting sweat secretion of rat paw. Please see 2-1, 2-2, 2-3, 2-4, and 2-5 for details.

**Table 2-1 Effect of the Panax Ginseng and Forsythia Suspensa Composition on Sweat Secretion of Paw of Normal Rats (Staining Method)**

| Group | Animal number (animals) | Number of animals with sweat spots of each grade at lmin after application of solution B (animals) | | | | | P-value |
|---|---|---|---|---|---|---|---|
| | | - | + | ++ | +++ | +++ | |
| Control group | 10 | 2 | 2 | 3 | 2 | 1 | |
| Forsythin | 10 | 0 | 2 | 2 | 2 | 4 | >0.05 |
| Ginsenoside Rg3 | 10 | 0 | 1 | 3 | 3 | 3 | >0.05 |
| Pilocarpine | | | | | | | |
| 35.0 mg/kg | 10 | 0 | 1 | 3 | 1 | 5 | >0.05 |
| Composition A | | | | | | | |
| 2.5 mg/kg | 10 | 0 | 3 | 2 | 0 | 5 | >0.05 |
| 5.0 mg/kg | 10 | 0 | 3 | 1 | 1 | 5 | >0.05 |
| 10.0 mg/kg | 10 | 0 | 3 | 1 | 1 | 5 | >0.05 |
| Composition B | | | | | | | |
| 2.5 mg/kg | 10 | 0 | 3 | 2 | 0 | 5 | >0.05 |
| 5.0 mg/kg | 10 | 0 | 3 | 1 | 1 | 5 | >0.05 |
| 10.0 mg/kg | 10 | 0 | 3 | 1 | 1 | 5 | >0.05 |
| Composition C | | | | | | | |
| 2.5 mg/kg | 10 | 0 | 3 | 2 | 0 | 5 | >0.05 |
| 5.0 mg/kg | 10 | 0 | 3 | 1 | 1 | 5 | >0.05 |
| 10.0 mg/kg | 10 | 0 | 3 | 1 | 1 | 5 | >0.05 |
| 2.5 mg/kg | 10 | 0 | 3 | 2 | 0 | 5 | >0.05 |
| 5.0 mg/kg | 10 | 0 | 3 | 1 | 1 | 5 | >0.05 |
| 10.0 mg/kg | 10 | 0 | 3 | 1 | 1 | 5 | >0.05 |
| Composition E | | | | | | | |
| 2.5 mg/kg | 10 | 0 | 3 | 2 | 0 | 5 | >0.05 |
| 5.0 mg/kg | 10 | 0 | 3 | 1 | 1 | 5 | >0.05 |
| 10.0 mg/kg | 10 | 0 | 3 | 1 | 1 | 5 | >0.05 |

**Table 2-2 Effect of the Panax Ginseng and Forsythia Suspensa Composition on Sweat Secretion of Paw of Normal Rats (Staining Method)**

| 1. Group | 2. Animal number 3. (animals) | 4. Number of animals with sweat spots of each grade at 5min after application of solution B (animals) | | | | | 5. P-value |
|---|---|---|---|---|---|---|---|
| | | - | + | ++ | +++ | +++ | |
| Control group | 10 | 0 | 4 | 1 | 4 | 1 | |
| Forsythin | 10 | 0 | 1 | 2 | 2 | 5 | >0.05 |
| Ginsenoside Rg3 | 10 | 0 | 1 | 3 | 2 | 4 | >0.05 |
| Pilocarpine | | | | | | | |
| 35.0 mg/kg | 10 | 0 | 1 | 2 | 1 | 6 | <0.05* |

(continued)

| | | | | | | | |
|---|---|---|---|---|---|---|---|
| Composition A | | | | | | | |
| 2.5 mg/kg | 10 | 0 | 2 | 2 | 1 | 5 | >0.05 |
| 5.0 mg/kg | 10 | 0 | 1 | 2 | 2 | 5 | >0.05 |
| 10.0 mg/kg | 10 | 0 | 1 | 3 | 2 | 5 | >0.05 |
| Composition B | | | | | | | |
| 2.5 mg/kg | 10 | 0 | 3 | 2 | 0 | 5 | >0.05 |
| 5.0 mg/kg | 10 | 0 | 3 | 1 | 1 | 5 | >0.05 |
| 10.0 mg/kg | 10 | 0 | 3 | 1 | 1 | 5 | >0.05 |
| Composition C | | | | | | | |
| 2.5 mg/kg | 10 | 0 | 3 | 2 | 0 | 5 | >0.05 |
| 5.0 mg/kg | 10 | 0 | 3 | 1 | 1 | 5 | >0.05 |
| 10.0 mg/kg | 10 | 0 | 3 | 1 | 1 | 5 | >0.05 |
| Composition D | | | | | | | |
| 2.5 mg/kg | 10 | 0 | 3 | 2 | 0 | 5 | >0.05 |
| 5.0 mg/kg | 10 | 0 | 3 | 1 | 1 | 5 | >0.05 |
| 10.0 mg/kg | 10 | 0 | 3 | 1 | 1 | 5 | >0.05 |
| Composition E | | | | | | | |
| 2.5 mg/kg | 10 | 0 | 3 | 2 | 0 | 5 | >0.05 |
| 5.0 mg/kg | 10 | 0 | 3 | 1 | 1 | 5 | >0.05 |
| 10.0 mg/kg | 10 | 0 | 3 | 1 | 1 | 5 | >0.05 |

**Table 2-3 Effect of the Panax Ginseng and Forsythia Suspensa Composition on Sweat Secretion of Paw of Normal Rats (Staining Method)**

| Group | Animal number (animals) | Number of animals with sweat spots of each grade at 10 min after application of solution B (animals) | | | | | P-value |
|---|---|---|---|---|---|---|---|
| | | - | + | ++ | +++ | +++ | |
| Control group | 10 | 0 | 3 | 2 | 4 | 1 | |
| Forsythin | 10 | 0 | 1 | 1 | 3 | 5 | >0.05 |
| Ginsenoside Rg3 | 10 | 0 | 1 | 3 | 1 | 5 | >0.05 |
| Pilocarpine | | | | | | | |
| 35.0 mg/kg | 10 | 0 | 0 | 2 | 1 | 6 | <0.05* |
| Composition A | | | | | | | |
| 2.5 mg/kg | 10 | 0 | 0 | 2 | 3 | 5 | >0.05 |
| 5.0 mg/kg | 10 | 0 | 3 | 0 | 1 | 6 | <0.05*#▲ |
| 10.0 mg/kg | 10 | 0 | 1 | 1 | 1 | 6 | <0.05*#▲ |
| Composition B | | | | | | | |
| 2.5 mg/kg | 10 | 0 | 0 | 2 | 3 | 5 | >0.05 |
| 5.0 mg/kg | 10 | 0 | 3 | 0 | 1 | 6 | <0.05*#▲ |
| 10.0 mg/kg | 10 | 0 | 1 | 1 | 1 | 6 | <0.05*#▲ |
| Composition C | | | | | | | |
| 2.5 mg/kg | 10 | 0 | 0 | 2 | 3 | 5 | >0.05 |
| 5.0 mg/kg | 10 | 0 | 3 | 0 | 1 | 6 | <0.05*#▲ |
| 10.0 mg/kg | 10 | 0 | 1 | 1 | 1 | 6 | <0.05*#▲ |
| Composition D | | | | | | | |
| 2.5 mg/kg | 10 | 0 | 0 | 2 | 3 | 5 | >0.05 |

(continued)

| | | | | | | | |
|---|---|---|---|---|---|---|---|
| Composition D | | | | | | | |
| 5.0 mg/kg | 10 | 0 | 3 | 0 | 1 | 6 | <0.05*#▲ |
| 10.0 mg/kg | 10 | 0 | 1 | 1 | 1 | 6 | <0.05*#▲ |
| Composition E | | | | | | | |
| 2.5 mg/kg | 10 | 0 | 0 | 2 | 3 | 5 | >0.05 |
| 5.0 mg/kg | 10 | 0 | 3 | 0 | 1 | 6 | <0.05*#▲ |
| 10.0 mg/kg | 10 | 0 | 1 | 1 | 1 | 6 | <0.05*#▲ |

**Table 2-4 Effect of the Panax Ginseng and Forsythia Suspensa Composition on Sweat Secretion of Paw of Normal Rats (Staining Method)**

| Group | Animal number (animals) | Number of animals with sweat spots of each grade at 15min after application of solution B (animals) | | | | | P-value |
|---|---|---|---|---|---|---|---|
| | | - | + | ++ | +++ | +++ | |
| Control group | 10 | 0 | 3 | 2 | 4 | 1 | |
| Forsythin | 10 | 0 | 1 | 2 | 4 | 5 | <0.05* |
| Ginsenoside Rg3 | 10 | 0 | 1 | 1 | 3 | 5 | >0.05 |
| Pilocarpine | | | | | | | |
| 35.0 mg/kg | 9 | 0 | 0 | 2 | 1 | 6 | <0.05* |
| Composition A | | | | | | | |
| 2.5 mg/kg | 10 | 0 | 3 | 0 | 1 | 6 | <0.05*▲ |
| 5.0 mg/kg | 10 | 0 | 2 | 1 | 1 | 6 | <0.05*▲ |
| 10.0 mg/kg | 10 | 0 | 1 | 2 | 1 | 6 | <0.05*#▲ |
| Composition B | | | | | | | |
| 2.5 mg/kg | 10 | 0 | 3 | 0 | 1 | 6 | <0.05*▲ |
| 5.0 mg/kg | 10 | 0 | 2 | 1 | 1 | 6 | <0.05*▲ |
| 10.0 mg/kg | 10 | 0 | 1 | 2 | 1 | 6 | <0.05*#▲ |
| Composition C | | | | | | | |
| 2.5 mg/kg | 10 | 0 | 3 | 0 | 1 | 6 | <0.05*▲ |
| 5.0 mg/kg | 10 | 0 | 2 | 1 | 1 | 6 | <0.05*▲ |
| 10.0 mg/kg | 10 | 0 | 1 | 2 | 1 | 6 | <0.05*#▲ |
| Composition D | | | | | | | |
| 2.5 mg/kg | 10 | 0 | 3 | 0 | 1 | 6 | <0.05*▲ |
| 5.0 mg/kg | 10 | 0 | 2 | 1 | 1 | 6 | <0.05*▲ |
| 10.0 mg/kg | 10 | 0 | 1 | 2 | 1 | 6 | <0.05*#▲ |
| Composition E | | | | | | | |
| 2.5 mg/kg | 10 | 0 | 3 | 0 | 1 | 6 | <0.05*▲ |
| 5.0 mg/kg | 10 | 0 | 2 | 1 | 1 | 6 | <0.05*▲ |
| 10.0 mg/kg | 10 | 0 | 1 | 2 | 1 | 6 | <0.05*#▲ |

**Table 2-5 Effect of the Panax Ginseng and Forsythia Suspensa Composition on Sweat Secretion of Paw of Normal Rats (Staining Method)**

| Group | Animal number (animals) | Number of animals with sweat spots of each grade at 20 min after application of solution B (animals) | | | | | P-value |
|---|---|---|---|---|---|---|---|
| | | - | + | ++ | +++ | +++ | |
| Control group | 10 | 0 | 3 | 2 | 4 | 1 | |

(continued)

| Group | Animal number (animals) | Number of animals with sweat spots of each grade at 20 min after application of solution B (animals) | | | | | P-value |
|---|---|---|---|---|---|---|---|
| | | - | + | ++ | +++ | +++ | |
| Forsythin | 10 | 0 | 0 | 4 | 0 | 6 | <0.05* |
| Ginsenoside Rg3 | 10 | 0 | 0 | 1 | 4 | 5 | <0.05* |
| Pilocarpine 35.0 mg/kg | 9 | 0 | 0 | 2 | 1 | 6 | <0.05* |
| Composition A | | | | | | | |
| 2.5 mg/kg | 10 | 0 | 2 | 1 | 1 | 6 | <0.05* |
| 5.0 mg/kg | 10 | 0 | 1 | 2 | 1 | 6 | <0.05* |
| 10.0 mg/kg | 10 | 0 | 0 | 2 | 2 | 6 | <0.05*#▲ |
| Composition B | | | | | | | |
| 2.5 mg/kg | 10 | 0 | 2 | 1 | 1 | 6 | <0.05* |
| 5.0 mg/kg | 10 | 0 | 1 | 2 | 1 | 6 | <0.05* |
| 10.0 mg/kg | 10 | 0 | 0 | 2 | 2 | 6 | <0.05*#▲ |
| Composition C | | | | | | | |
| 2.5 mg/kg | 10 | 0 | 2 | 1 | 1 | 6 | <0.05* |
| 5.0 mg/kg | 10 | 0 | 1 | 2 | 1 | 6 | <0.05* |
| 10.0 mg/kg | 10 | 0 | 0 | 2 | 2 | 6 | <0.05*#▲ |
| Composition D | | | | | | | |
| 2.5 mg/kg | 10 | 0 | 2 | 1 | 1 | 6 | <0.05* |
| 5.0 mg/kg | 10 | 0 | 1 | 2 | 1 | 6 | <0.05* |
| 10.0 mg/kg | 10 | 0 | 0 | 2 | 2 | 6 | <0.05*#▲ |
| Composition E | | | | | | | |
| 2.5 mg/kg | 10 | 0 | 2 | 1 | 1 | 6 | <0.05* |
| 5.0 mg/kg | 10 | 0 | 1 | 2 | 1 | 6 | <0.05* |
| 10.0 mg/kg | 10 | 0 | 0 | 2 | 2 | 6 | <0.05*#▲ |

**Evaluation standard for sweat spot grade:** "-": there is no sweat spot on the surface of plantar pad of the rat; "+": sweat spots are occasionally found on the surface of the plantar pad of the rat, and the area of sweat spots was less than 10% of the plantar surface; "++": sweat spots are scattered on the surface of the plantar pad of the rat, and the area of sweat spots is about 11-40% of the plantar surface; "+++": sweat spots are distributed on multiple areas of the surface of the plantar pad of the rat, and the area of sweat spots is about 41-70% of the plantar surface; "++++": sweat spots are distributed evenly on the surface of the plantar pad of the rat, and the area of sweat spots is about more than 71% of the plantar surface

*P < 0.05 for the comparison between each test group and the virus control group; $^{\#}$P < 0.05 for the comparison between panax ginseng-forsythia suspensa composition and forsythin; $^{▲}$P < 0.05 for the comparison between panax ginseng-forsythia suspensa composition and ginsenoside Rg3.

### 2.2 Effect of the Panax Ginseng and Forsythia Suspensa Composition on Sweat Secretion of Rat Paw (Histomorphology Observation Method)

#### (1) Materials and Methods

After sweat glands activation in rats, besides the increase of sweat secretion, the morphology of the epithelial cells of the sweat glands changes accordingly. Under the optical microscope, the increase and enlargement of vacuoles in the epithelial cells of the sweat glands are observed. Under an electron microscope, the enlarged vacuoles appear to be due to swelling and rupture of mitochondria, enlargement of fusion and secretory vesicles in the epithelial cells of sweat glands. The secretory activity of sweat glands could be understood by observing the histomorphology of the epithelial cells of plantar sweat glands of rats.

Three hundred Wistar rats, half male and half female, weighing 120-160 g, were taken in the study. They were randomly divided into 30 groups according to body weight and sex, namely the control group (0.5% carboxymethylcellulose), ginsenoside Rg3, forsythin, low, middle and high dose groups of panax ginseng-forsythia suspensa composition (2.5, 5, 10 mg/kg) and positive drug pilocarpine group (35 mg/kg), with 10 rats in each group, and 3 tests for each group. Except for the pilocarpine solution given by subcutaneous injection, other groups were administered by gavage. One hour after the control group was given 0.5% carboxymethyl cellulose, 30 min after the positive drug group was given pilocarpine, and 1 h after the administration of forsythin, ginsenoside Rg3 and the panax ginseng-forsythia suspensa composition, the right hind limb was immediately cut off at the level of the ankle joint. Then, the right plantar pad was removed, placed in 10% formaldehyde solution, fixed, dehydrated, embedded, sectioned and stained with HE. Observations were made under an optical microscope for changes in the epithelial cells of the plantar sweat glands of rats in each group, mainly the incidence of vacuoles. The statistical processing was performed by $X^2$ test, and differences among the groups were compared. The above study was repeated for 3 times.

$$\text{Percentage of vacuolation} = \text{number of vacuolar sweat glands/number of observed sweat glands} \times 100\%$$

**(2) Results**

Compared with the control group, the 2.5 mg/kg, 5 mg/kg, 10 mg/kg groups of panax ginseng-forsythia suspensa composition very significantly promoted the sweat secretion of rat paw ($p < 0.001$). The low, middle and high dose groups (2.5, 5 and 10 mg/kg) had significantly better efficacy than forsythin and ginsenoside Rg3 ($p < 0.001$ or $p < 0.01$), indicating that the compositions of panax ginseng and forsythia suspensa have a synergistic effect. The test results are shown in Tables 2-6;

**Table 2-6 Effect of the Panax Ginseng and Forsythia Suspensa Composition on Sweat Secretion of Rat Paw (Histomorphology Observation Method, n=3)**

| Group | Animal number (animals) | Number of observed sweat glands (number) | Number of vacuolar sweat glands (number) | Vacuolation rate (%) |
|---|---|---|---|---|
| Control group | 10 | 242 | 14 | 5.78 |
| Forsythin | 10 | 209 | 86 | 22.15** |
| Ginsenoside Rg3 | 10 | 212 | 79 | 20.26** |
| Pilocarpine | | | | |
| 35.0 mg/kg | 10 | 208 | 57 | 27.40*** |
| Composition A | | | | |
| 2.5 mg/kg | 10 | 221 | 66 | 29.86***##▲▲ |
| 5.0 mg/kg | 10 | 215 | 73 | 33.95***###▲▲▲ |
| 10.0 mg/kg | 10 | 207 | 82 | 39.61***###▲▲▲ |
| Composition B | | | | |
| 2.5 mg/kg | 10 | 221 | 68 | 30.76***##▲▲ |
| 5.0 mg/kg | 10 | 218 | 71 | 32.56***###▲▲▲ |
| 10.0 mg/kg | 10 | 209 | 87 | 41.62***###▲▲▲ |
| Composition C | | | | |
| 2.5 mg/kg | 10 | 222 | 65 | 29.27***##▲▲ |
| 5.0 mg/kg | 10 | 217 | 72 | 33.17***###▲▲▲ |
| 10.0 mg/kg | 10 | 208 | 83 | 39.03***###▲▲▲ |
| Composition D | | | | |
| 2.5 mg/kg | 10 | 221 | 25 | 30.31***##▲▲ |
| 5.0 mg/kg | 10 | 213 | 73 | 34.27***###▲▲▲ |

(continued)

| | | | | |
|---|---|---|---|---|
| Composition D | | | | |
| 10.0 mg/kg | 10 | 207 | 85 | 41.63***###▲▲▲ |
| Composition E | | | | |
| 2.5 mg/kg | 10 | 225 | 66 | 29.30***##▲▲ |
| 5.0 mg/kg | 10 | 219 | 69 | 31.51***###▲▲▲ |
| 10.0 mg/kg | 10 | 208 | 87 | 41.82***###▲▲▲ |
| **p<0.01, ***p<0.001 compared with the control group; ##P < 0.01, ###P < 0.001 for the comparison between panax ginseng-forsythia suspensa composition and forsythin; ▲▲P < 0.01, ▲▲▲P < 0.001 for the comparison between panax ginseng-forsythia suspensa composition groups and ginsenoside Rg3. | | | | |

**2.3 Effect of the Panax Ginseng-forsythia Suspensa Composition on the Fever Induced by Beer Yeast in Rats**

**(1) Materials and Methods**

Male Wistar rats: weighing 180-200 g. Before the test, the normal rectal temperature was measured twice with WSC-411P portable digital thermometer (at a certain interval), and the mean value of the two measurements was taken as the normal body temperature of the rats. Then, 300 rats with a body temperature of 36.5 ~ 38°C were randomly divided into 30 groups: the model group (0.5% carboxymethylcellulose), low, middle and high dose groups of panax ginseng-forsythia suspensa composition (2.5, 5, 10 mg/kg), forsythin group (13mg/kg), ginsenoside Rg3 group (13mg/kg) and positive drug paracetamol group (100 mg/kg), with 10 rats in each group, and 3 replicate tests for each group. The rats in each group were subcutaneously injected with 10% fresh beer yeast suspension at 10 mL/kg on the back to induce fever. Six point zero hours after administration of 10% fresh beer yeast suspension, the composition of ginsenoside Rg3 and forsythin and paracetamol were given by gavage, and the model group was given an equal volume of 0.5% carboxymethylcellulose by gavage. Rectal temperature was measured at 1, 2, 3 and 4 h after administration. Observations were made on changes in body temperature, and the differences among the groups were compared by t-test of antipyretic percentage. The above study was repeated 3 times.

$$\text{Antipyretic percentage} = \frac{\text{Body temperature at a certain time after administration - body temperature 6h after fever induction}}{\text{Body temperature at 6h after fever induction}} \times 100\%$$

**(2) Results**

Six hours after subcutaneous injection of 10% fresh beer yeast suspension to rats in each group, the body temperature increased by about 1.5°C, which was significantly different from that before fever induction (p < 0.001), indicating that the rat fever model induced by beer yeast was established successfully. Compared with the model group, a significant temperature-lowering effect on the rat fever induced by beer yeast suspension was noted in the middle and high dose groups of panax ginseng-forsythia suspensa composition at 1, 2, 3 and 4h after drug administration and in the low dose group of panax ginseng-forsythia suspensa composition at 2, 3 and 4h after drug administration (p<0.05 to p<0.001). Meanwhile, the different doses of panax ginseng-forsythia suspensa composition were significantly superior to the forsythin group and ginsenoside Rg3 group (p < 0.001 or p < 0.01) in lowering the temperature, indicating that they had a significant synergistic effect. The results of the above study are shown in Tables 2-7.

**2.4 Effect of the Panax Ginseng-forsythia Suspensa Composition on Carrageenan-induced Paw Swelling in Rats**

**(1) Materials and Methods**

Two hundred and twenty male Wistar rats weighing 120-150 g were randomly divided into 22 groups, namely the control group (0.5% sodium carboxymethylcellulose), low, middle and high dose groups of panax ginseng-forsythia suspensa composition (2.5, 5, 10 mg/kg), forsythin group, 20(R)-ginsenoside Rg3 group and positive drug aspirin group (200 mg/kg), with 10 rats in each group. Each group was administered by sublingual intravenous injection. Before the study, the normal volume of the right hind limb was measured by the capillary magnification method. To avoid errors,

the measurement position should be fixed and performed by one person before and after administration. The average of the two measurements was taken as the normal volume of the right hind limb of the rats prior to administration. Immediately after administration, 0.1 ml of 1% carrageenan was subcutaneously injected into the right hind paw of rats to induce inflammation. The right hind plantar volume was measured at 15, 30, 60, 120, 180, 240, 300 and 360 min after inflammation induction. The inter-group t-test was performed by the difference percentage (swelling rate) of the plantar volume before and after the inflammation induction, and the differences among the groups were compared.

$$\text{Swelling rate (\%)} = \frac{\text{Right hind plantar volume after inflammation induction - right hind plantar volume before administration}}{\text{Right plantar volume before administration}} \times 100\%$$

**Results**

Compared with the blank control group, the rat paw swelling induced by carrageenan was significantly inhibited ($p < 0.05$ or $p < 0.01$) in the high dose group (10 mg/kg) of panax ginseng-forsythia suspensa composition within 15min to 360 min after administration, the middle dose group (5 mg/kg) and the low dose group (2.5 mg/kg) within 30 min to 360 min after administration, and the efficacy was significantly better than that of the forsythin 10 mg/kg group and 20(R)-ginsenoside Rg3 10 mg/kg group ($p < 0.05$ or $p < 0.01$). The efficacy of the above-mentioned dose groups of the composition was very significantly better than that of the 20(R)-ginsenoside Rg3 group at 60 min and 240 min after administration ($p < 0.01$). The above study results showed a significant synergistic effect when forsythin in the panax ginseng-forsythia suspensa composition was combined with 20(R)-ginsenoside Rg3. See Table 2-8 for details.

**Table 2-7 Effect of the Panax Ginseng-forsythia Suspensa Composition on the Fever Induced by Beer Yeast in Rats ($\overline{\chi}\pm$ s, n=3)**

| Group | Normal | 6h after fever induction | Body temperature (°C) | | | |
|---|---|---|---|---|---|---|
| | | | Time post-dose (h) | | | |
| | | | 1 | 2 | 3 | 4 |
| Model control group (%) | 37.72±0.90 | 39.30±0.54<br>4.22±2.11### | 39.44±0.58<br>0.37±1.66 | 39.42±0.47<br>0.31±1.77 | 38.88±0.46<br>-1.07±1.54 | 38.57±0.49<br>-1.86±1.20 |
| Paracetamol 100 mg/kg(%) | 37.55±0.70 | 39.48±0.62<br>5.14±1.42### | 38.66±0.59<br>-2.07±0.54** | 38.19±0.59<br>-3.27±0.77*** | 37.98±0.19<br>-3.80±1.43*** | 37.84±0.32<br>-4.15±1.59*** |
| Forsythin 10 mg/kg (%) | 37.58±0.59 | 39.53±0.63<br>5.18±1.52### | 39.13±0.52<br>-0.60±0.39 | 38.77±0.42<br>-0.92±0.93** | 38.63±0.40<br>-1.29±1.18* | 38.46±0.31<br>-1.70±1.23* |
| 20(R)-Ginsenoside Rg3 10 mg/kg (%) | 37.50±0.59 | 39.44±0.47<br>5.17±1.37### | 39.31±0.48<br>-0.33±0.41 | 39.04±0.45<br>-0.69±0.93* | 38.91±0.40<br>-1.03±1.18* | 38.70±0.31<br>-1.55±1.23* |
| Composition A 2.5 mg/kg(%) | 37.46±0.34 | 39.57±0.55<br>5.14±1.38### | 39.22±0.49<br>-0.62±1.04▲ | 38.94±0.42<br>-1.88±1.04▲ | 38.47±0.41<br>-2.38±1.17**△▲ | 38.27±0.49<br>-3.17±1.74***△▲ |
| 5.0 mg/kg(%) | 37.52±0.36 | 39.48±0.52<br>5.36±1.18### | 38.79±0.52<br>-1.77±0.48*△▲▲ | 38.37±0.25<br>-2.15±0.45**△▲ | 38.28±0.33<br>-2.52±0.39**△▲ | 37.97±0.41<br>-3.62±0.54***△△▲▲ |

(continued)

| | | | | | | |
|---|---|---|---|---|---|---|
| 20(R)-Ginsenoside Rg3 | | | | | | |
| 10.0 mg/kg(%) | 37.38±0.67 | 39.52±0.49<br>5.21±1.44### | 38.69±0.38<br>-2.03±0.46** △△▲▲ | 38.34±0.47<br>-2.46±0.65** △▲▲ | 38.21±0.73<br>-2.94±1.08** △△▲▲ | 37.87±0.28<br>-3.78±1.12** *△△▲▲ |
| Composition B | | | | | | |
| 2.5 mg/kg(%) | 37.57±0.41 | 39.49±0.36<br>5.45±1.28### | 39.21±0.48<br>-0.67±1.26▲ | 38.94±0.42<br>-1.23±1.54▲ | 38.47±0.54<br>-1.95±1.08** △▲ | 38.31±0.48<br>-2.87±1.73** *△▲ |
| 5.0 mg/kg(%) | 37.52±0.43 | 39.56±0.38<br>5.29±1.16### | 38.79±0.51<br>-1.76±0.47* △▲▲ | 38.43±0.42<br>-2.18±0.54** △▲ | 38.28±0.37<br>-2.84±0.31** △▲ | 37.93±0.47<br>-3.74±0.38** *△△▲▲ |
| 10.0 mg/kg(%) | 37.67±0.29 | 39.48±0.53<br>5.09±1.27### | 38.72±0.37<br>-1.63±0.45** △△▲▲ | 38.27±0.65<br>-2.37±0.62** △▲▲ | 38.18±0.50<br>-3.15±1.10** *△△▲▲ | 37.87±0.42<br>-4.10±1.32** *△△▲▲ |
| Composition C | | | | | | |
| 2.5 mg/kg(%) | 37.64±0.42 | 39.60±0.63<br>5.21±1.40### | 39.26±0.47<br>-0.87±1.12▲ | 38.97±0.41<br>-1.59±1.18▲ | 38.59±0.35<br>-2.54±1.32** △▲ | 38.30±0.50<br>-3.29±1.81** *△▲ |
| 5.0 mg/kg(%) | 37.47±0.63 | 39.47±0.43<br>5.33±1.27### | 38.76±0.56<br>-1.80±0.50* △▲▲ | 38.57±0.52<br>-2.27±0.50** △▲ | 38.35±0.40<br>-2.84±0.31** △▲ | 37.95±0.38<br>-3.85±0.44** *△△▲▲ |
| 10.0 mg/kg(%) | 37.55±0.76 | 39.48±0.67<br>5.13±1.50### | 38.71±0.49<br>-1.95±0.58** △△▲▲ | 38.39±0.48<br>-2.76±0.8** △▲▲ | 38.24±0.37<br>-3.15±1.10** *△△▲▲ | 37.90±0.31<br>-4.00±1.12** *△△▲▲ |
| Composition D | | | | | | |
| 2.5 mg/kg(%) | 37.33±0.51 | 39.23±0.63<br>5.10±1.45### | 38.86±0.47<br>-0.95±1.02▲ | 38.20±0.44<br>-1.70±1.11▲ | 37.85±0.52<br>-2.61±1.65** △▲ | 38.52±0.59<br>-3.45±1.88** △▲ |
| 5.0 mg/kg(%) | 37.41±0.55 | 39.37±0.41<br>5.25±1.25### | 38.61±0.52<br>-1.92±0.42* △▲▲ | 38.46±0.55<br>-2.31±0.57** △▲ | 38.22±0.32<br>-2.93±0.36** △▲ | 37.84±0.41<br>-3.93±0.50** *△△▲▲ |
| 10.0 mg/kg(%) | 37.53±0.59 | 39.54±0.62<br>5.36±1.52### | 38.69±0.57<br>-2.16±0.51** △△▲▲ | 37.72±0.40<br>-2.85±0.93** △▲▲ | 37.27±0.42<br>-3.66±1.18** *△△▲▲ | 37.08±0.35<br>-4.16±1.23** *△△▲▲ |
| Composition E | | | | | | |
| 2.5 mg/kg(%) | 37.58±0.47 | 39.68±0.36<br>5.17±1.28### | 39.25±0.33<br>-0.43±1.09▲ | 38.46±0.57<br>-1.07±1.05▲ | 38.11±0.47<br>-2.06±1.28** △▲ | 38.30±0.50<br>-3.29±1.81** *△▲ |
| 5.0 mg/kg(%) | 37.63±0.67 | 39.56±0.48<br>5.21±1.36### | 38.67±0.46<br>-1.69±0.62* △▲▲ | 38.13±0.46<br>-2.15±0.48** △▲ | 37.85±0.46<br>-2.32±0.44** △▲ | 37.95±0.38<br>-3.85±0.44** *△△▲▲ |

(continued)

| | | | | | | |
|---|---|---|---|---|---|---|
| 20(R)-Ginsenoside Rg3 | | | | | | |
| 10.0 mg/kg(%) | 37.55±0.52 | 39.62±0.53<br>-5.25±1.44### | 38.59±0.55<br>-1.95±0.58** △△▲▲ | 37.95±0.48<br>-2.68±0.58** △▲▲ | 37.82±0.33<br>-3.36±1.04** *△△▲▲ | 37.63±0.29<br>-4.13±1.24** *△△▲▲ |

*p < 0.05, **p < 0.01, ***p < 0.001 compared with the model control group;
###p < 0.001 compared with normal (before fever induction). Δp < 0.05; △△p<0.01; △△△p<0.001 for the comparison of antipyretic percentage between panax ginseng-forsythia suspensa composition and forsythin; ▲p < 0.05; ▲▲p < 0.01; ▲▲▲p < 0.001 for the comparison of antipyretic percentage between panax ginseng-forsythia suspensa composition and ginsenoside Rg3.

**Table 2-8 Inhibitory Effect of the Panax Ginseng-forsythia Suspensa Composition on Carrageenan-induced Paw Swelling in Rats ($\bar{x}\pm$ s, n=10)**

| Group | | Swelling rate (%) | | | | | | | |
|---|---|---|---|---|---|---|---|---|---|
| | | 15min | 30 min | 60 min | 120 min | 180 min | 240 min | 300 min | 360 min |
| Control group | | 18.7±12.6 | 28.9±14.6 | 33.8±10.5 | 46.5±18.2 | 46.7±15.3 | 48.8±21.9 | 49.1±14.6 | 47.4±15.5 |
| Aspirin | 100 mg/kg | 8.90±6.70* | 15.6±12.2* | 20.3±10.9* | 21.6±12.1** | 26.1±21.1** | 26.1±16.3** | 34.9±14.6* | 31.9±12.2* |
| Forsythin | 10.0 mg/kg | 12.9 ±8.41 | 19.9±9.55 | 21.8±11.8* | 28.9±14.0* | 30.8±15.4* | 29.9±11.1* | 33.5±10.0* | 32.2±11.9* |
| 20 (R)-Ginsenoside Rg3 | 10.0 mg/kg | 14.9 ±6.13 | 21.9±9.72 | 27.1±13.5 | 30.7±10.2* | 33.8±12.1* | 31.0±11.3* | 35.8±13.5* | 36.4±10.2 |
| Composition A | 2.5 mg/kg | 14.8±8.74 | 18.9±12.1 | 23.5±13.3*▲▲ | 28.9±14.2**△▲ | 31.8±14.8**△▲ | 33.1±18.3**△▲ | 34.9±19.6**△▲ | 37.8±18.3*▲▲ |
| | 5.0 mg/kg | 12.7 ±8.66 | 15.1±7.15*△▲ | 17.5±8.43*▲▲ | 19.2±10.4**△▲ | 22.6±12.9**△▲ | 24.6±13.7**△▲ | 27.3±11.7**△▲ | 30.3±16.8**△▲▲ |
| | 10.0 mg/kg | 10.4±8.12*△▲ | 12.4±9.52*△▲ | 14.3±11.9*△▲▲ | 16.6±10.7**△▲ | 19.6±12.7**△▲ | 21.5±11.6**△▲ | 24.2±12.7**△▲ | 28.9±15.1**△▲▲ |
| Composition B | 2.5 mg/kg | 14.5 ±9.11 | 17.4±16.8 | 22.1±17.3*▲▲ | 27.8±13.5**△▲ | 30.9±16.5**△▲ | 33.4±17.3**△▲ | 33.6±15.8**△▲ | 36.9±21.0*▲▲ |
| | 5.0 mg/kg | 13.1 ±8.97 | 16.7±7.62*△▲ | 18.5±10.35*▲▲ | 21.6±13.7**△▲ | 23.9±14.7**△▲ | 26.8±12.8**△▲ | 28.9±10.6**△▲ | 32.7±13.9**△▲▲ |
| | 10.0 mg/kg | 10.3±7.65*△▲ | 12.4±9.88*△▲ | 15.1±13.8**△▲▲ | 17.8±12.9**△▲ | 20.1±13.7**△▲ | 23.5±13.6**△▲ | 26.2±15.1**△▲ | 29.4±14.6**△▲▲ |
| Composition C | 2.5 mg/kg | 13.5 ±8.52 | 20.9±16.0 | 21.1±14.1*▲▲ | 23.1±16.5**△▲ | 27.4±15.6**△▲ | 27.7±23.7**△▲ | 30.4±16.9**△▲ | 33.1±22.8**△▲▲ |
| | 5.0 mg/kg | 12.4 ±8.71 | 16.0±6.15*△▲ | 19.5±9.55*▲▲ | 20.2±11.6**△▲ | 23.2±15.2**△▲ | 25.6±11.6**△▲ | 28.89±12.7**△▲ | 31.6±15.5**△▲▲ |
| | 10.0 mg/kg | 9.9 ±7.80*△▲ | 13.9±9.61*△▲ | 16.9±12.2**△▲▲ | 18.2±14.8**△▲ | 20.5±15.9**△▲ | 23.8±12.0**△▲ | 26.1±11.3**△▲ | 29.8±11.9**△▲▲ |
| Composition D | 2.5 mg/kg | 13.6 ±8.40 | 24.2±16.3 | 20.1±14.8*▲▲ | 22.0±16.0**△▲ | 26.8±16.1**△▲ | 26.8±23.3**△▲ | 29.5±16.1**△▲ | 32.4±23.4*▲▲ |
| | 5.0 mg/kg | 11.4 ±8.90 | 15.2±6.20*△▲ | 18.0±9.00*▲▲ | 19.7±12.1**△▲ | 22.0±14.9**△▲ | 24.2±12.0**△▲ | 27.8±13.1**△▲ | 30.0±16.0**△▲▲ |
| | 10.0 mg/kg | 9.0 ±7.90*△▲ | 13.2±9.50*△▲ | 15.8±11.6**△▲▲ | 17.9±14.3**△▲ | 19.7±15.2**△▲ | 22.5±11.8**△▲ | 25.4±10.6**△▲ | 28.7±11.4**△▲▲ |

(continued)

| Group | | Swelling rate (%) | | | | | | | |
|---|---|---|---|---|---|---|---|---|---|
| | | 15min | 30 min | 60 min | 120 min | 180 min | 240 min | 300 min | 360 min |
| Composition E | 2.5 mg/kg | 12.7 ±8.26 | 19.5±14.6 | 20.9±13.7*▲▲ | 22.9±14.2** △▲ | 24.6±12.4** △▲ | 26.8±16.7** △▲ | 29.5±13.7**△▲ | 32.1±18.7*▲ ▲ |
| | 5.0 mg/kg | 11.3 ±7.92 | 14.3±7.33*△ ▲ | 16.5±8.62*▲▲ | 18.7±13.9** △▲ | 22.7±14.2** △▲ | 24.3±14.1** △▲ | 26.1±19.3**△▲ | 29.6±16.1** △▲ |
| | 10.0 mg/kg | 9.6 ±7.45*△▲ | 12.6±9.54*△ ▲A | 15.8±11.3**△▲▲ | 17.4±12.6** △▲ | 19.1±12.8** △▲ | 21.8±16.3** △▲ | 24.7±16.3**△▲ | 27.8±15.4** △▲▲ |

*p < 0.05, **p < 0.01 compared with the control group;
△p < 0.05; △△p<0.01 for the comparison of antipyretic percentage between panax ginseng-forsythia suspensa composition and forsythin; ▲p < 0.05, ▲▲p < 0.01 for the comparison of antipyretic percentage between panax ginseng-forsythia suspensa composition and ginsenoside Rg3.

**Exemplary Embodiment 108 Exploration on the Effect of the Panax Ginseng-forsythia Suspensa Composition on Enhancing Immune Function in Mice**

**1. Experimental Materials**

1.1 Drugs and Reagents

Forsythin (content > 98%), white powder, is manufactured by Dalian Fusheng Natural Drugs Development Co., Ltd. Its purity was 99.5% as determined by HPLC with two detectors: UV detector and evaporative light scattering detector using the area normalization method. The content of forsythin was calibrated and confirmed to be 99.5% using the reference standard of forsythin for the assay of pharmaceutical and biological products in China.

20(R)-ginsenoside Rg3 (content > 98%), white powder, is manufactured by Dalian Fusheng Natural Drugs Development Co., Ltd. Its purity was 99.0% as determined by HPLC with two detectors: UV detector and evaporative light scattering detector using the area normalization method. The content of forsythin was calibrated and confirmed to be 99.1% using the reference standard of ginsenoside Rg3 for the assay of pharmaceutical and biological products in China.

Panax ginseng and forsythia suspensa composition: Dalian Fusheng Natural Drugs Development Co., Ltd., calibrated with the reference standard for assay of pharmaceutical and biological products in China, to determine the contents of ginsenoside Rg3 and forsythin.

Alcohol extract of panax ginseng-forsythia suspensa composition (A; Exemplary Embodiment 53), with the contents of ginsenoside Rg3 and forsythin of 0.15% and 18.6%, respectively;
Water extract of panax ginseng-forsythia suspensa composition (B; Exemplary Embodiment 54), with the contents of ginsenoside Rg3 and forsythin of 0.17% and 17.7%, respectively;
Total ginsenoside-forsythin and phillygenin composition (C; Exemplary Embodiment 62), with the contents of ginsenoside Rg3 and forsythin of 0.04% and 95.6%, respectively;
Panaxadiol saponin-forsythin and phillygenin composition (D; Exemplary Embodiment 67), with the contents of ginsenoside Rg3 and forsythin of 0.12% and 96.6%, respectively;
20(R)-ginsenoside Rg3-forsythin composition (E; Exemplary Embodiment 74): the content of ginsenoside Rg3 and forsythin is 2.0% and 96.6%, respectively.
Positive control drug: Pidotimod Oral Solution (Suzhou Pharmaceutical Factory of Jiangsu Wuzhong Pharmaceutical Group Corporation, strength: 10 mL: 400 mg, batch number: 2014091211);

1.2 Experimental Animals

Kunming mice, aged 6-8 weeks, weighing 18-22 g, were purchased from Experimental Animal Center of Dalian Medical University, with quality certificate No.: SCXK (13) 2013-0003.

**2. Experimental Method**

2.1 Grouping and Administration

One hundred and fifty-two healthy male mice were subjected to acclimation for 4 days before they were randomly divided into 19 groups: the negative control group, positive control group, forsythin group, 20(R)-ginsenoside Rg3 group, high, middle and low dose groups of panax ginseng-forsythia suspensa composition. The positive control groups were given pidotimod (50 mg/kg), forsythin (144mg/kg), and 20(R)-ginsenoside Rg3 (144mg/kg); the panax ginseng-forsythia suspensa composition groups were given low dose (36mg/kg), middle dose (72mg/kg) and high dose (144mg/kg). The drugs were given by gavage, once daily for 30 days, and the negative control group was given the same volume of water.

2.2 Experiments and Results

2.2.1 ConA-induced Mouse Splenic Lymphocyte Transformation Test

At 1 h after the last dose, the spleens of animals of each group were aseptically excised to prepare splenocyte suspension. After diluting the splenocyte suspension to a concentration of $3\times10^6$ cells/mL, the splenocyte suspension was divided into 2 aliquots.Each of the aliquot was transferred into two 24-well culture plates at a volume of 1 mL/well followed by addition of 75 $\mu$L of ConA solution (a) was added to one well, and the other well was used as control (b). The wells were cultured at 37°C for 72 h. Then, thiazolyl blue (MTT) was added 4 h before the end of the culture. After

incubation, acidic isopropanol was added, and the absorbance (ABS) of each solution was measured at 570 nm after it was mixed well. The proliferativity was calculated using the formula: proliferativity = ABSa-ABSb. Each dose group of the test sample was compared with the negative control group. The experimental results are shown in Table 1.

2.2.2 Determination of NK Cell Viability

One hour after the last dose, the spleens of animals of each group were aseptically excised to prepare splenocyte suspension. After the red blood cells were lysed with sterile water for injection, the cell suspension was diluted with 1% glacial acetic acid. Then, the concentration of splenocyte suspension was adjusted to $2\times10^7$ cells/mL, and the splenocyte suspension was added into a 96-well plate for culturing. For each animal, the wells were divided into reaction wells (splenocyte suspension and YAC-1 cell suspension 100 $\mu$L each, effector-to-target ratio 50:1), natural release wells (YAC-1 cell suspension and culture medium 100 $\mu$L each) and maximum release wells (YAC-1 cell suspension and 1% NP40 100 $\mu$L each). A triplicate set of tubes was prepared for each of the above items. The 96-well plate was incubated at 37°C in a 5% $CO_2$ incubator for 4 h. After adding LDH matrix solution and 1 moL/L HCl, the solutions in each parallel well were combined, and the absorbance (ABS) was measured at 490 nm. The NK cell viability was calculated: NK cell viability (%) = (ABS reaction well -ABS natural release well)/(ABS maximum release well -ABS natural release well). The experimental results are shown in Table 1.

**Table 1 Effect of the Panax Ginseng-forsythia Suspensa Composition on NK Cell Viability and ConA-induced Lymphocyte Proliferation Ability ($x \pm s$)**

| Group | | Dose administered (mg/kg) | NK cell viability (%) | Proliferation ability ($\times10^{-2}$) |
|---|---|---|---|---|
| Negative control group | | -- | 34.14±5.68 | 9.04±3.81 |
| Positive control group | | 50 | 42.57±7.31 | 27.97±11.45 |
| Forsythin | | 144 | 41.86±7.24 | 28.66±12.63 |
| 20(R)-Ginsenoside Rg3 | | 144 | 37.34±7.21 | 23.9±11.70 |
| Compos ition A | Low dose group | 36 | 38.77±9.15 | 20.65±8.03 |
| | Medium dose | 72 | 41.52±10.53 | 25.61±10.12 |
| | group | | | |
| | High dose group | 144 | 45.77±8.76 | 33.89±16.87 |
| Compos ition B | Low dose group | 36 | 38.38±6.84 | 19.94±10.60 |
| | Medium dose group | 72 | 40.31±11.29 | 26.08±11.74 |
| | High dose group | 144 | 44.82±7.97 | 31.84±13.61 |
| Compos ition C | Low dose group | 36 | 39.64±7.15 | 20.89±7.84 |
| | Medium dose group | 72 | 43.21±10.18 | 28.03±12.02 |
| | High dose group | 144 | 45.62±11.74 | 35.54±13.97 |
| Compos ition D | Low dose group | 36 | 40.13±9.25 | 21.96±7.38 |
| | Medium dose group | 72 | 44.33±9.51 | 30.55±10.91 |
| | High dose group | 144 | 47.43±8.27 | 37.85±12.93 |
| Compos ition E | Low dose group | 36 | 40.75±7.51 | 20.93±9.11 |
| | Medium dose group | 72 | 44.79±11.64 | 29.49±12.52 |
| | High dose group | 144 | 47.92±8.94 | 37.13±17.03 |

### 3. Test Results

Following the stimulation of T lymphocytes by ConA, the blast cells will have proliferative responses. The mitochondrial hydrolase in the viable cells, especially the proliferating cells, decomposes MTT into blue-purple crystals. Increased optical density value, if any, indicates that the cell proliferation ability is enhanced. As shown in Table 1, the optical density differences of the high, middle and low dose groups of the panax ginseng-forsythia suspensa composition were higher than those of the negative control group, indicating that this sample had the effect of promoting the proliferation of splenocytes.

After the cells are killed by the NK cells, the LDH in the cytoplasm of viable cells will be released out of the cells, dehydrogenate lithium lactate and reduce NAD to NADH, and NADH is further reduced to iodonitro-tetrazolium chloride (INT) through hydrogen transmitter phenazine methosulfate (PMS). After receiving H+, the INT is reduced to a purple formazan compound, and the optical density value is determined using a microplate reader. As shown in Table 1, the NK cell viability of the high, middle and low dose groups of the panax ginseng-forsythia suspensa composition was significantly higher than that of the negative control group, indicating that the sample could increase the viability of the NK cells.

The above study results showed that different doses of the panax ginseng-forsythia suspensa composition could promote the proliferation of spleen cells and enhance the activity of NK cells. Moreover, the composition of panax ginseng and forsythia suspensa had a synergistic effect, and could significantly promote the proliferation of spleen cells, enhance the activity of NK cells and improve immune function.

## Claims

1. A composition for achieving the antiviral, antipyretic, anti-inflammatory and/or immunity boosting effects, which includes the forsythia suspensa components and preferably the panax ginseng components.

2. In the said composition in Claim 1, the panax ginseng components comprise the medicinal material of panax ginseng, the extract of panax ginseng, total ginsenoside, panaxadiol saponins, ginsenoside Rg3 or 20 (R)-ginsenoside Rg3; and the forsythia suspensa components include the medicinal material of forsythia suspensa leaf, the extract of forsythia suspensa leaf, forsythin and phillygenin and/or forsythin derivatives.

3. In the said composition in Claim 1 or 2, wherein the extract is an alcoholic or aqueous extract.

4. In the said composition in Claim 1 or 2, wherein the weight ratio of panax ginseng components to forsythia suspensa components is 2-98: 98-2.

5. The said composition in Claim 1 further includes one or more of the following components: panax notoginseng, aloe vera, glycyrrhiza uralensis, fallopia multiflora, ginkgo biloba, black sesame extract, ginger extract, grape seed extract, pomegranate seed extract, plant essential oil, arbutin, vitamin C and its derivatives or vitamin E and its derivatives; or, it also includes one or more of the following components: sophora tonkinensis extract, xanthium sibiricum extract, scutellaria barbata extract, Sophora flavescens extract, dandelion extract, honeysuckle extract, zingiber officinale rhizome extract, grape seed extract, pomegranate seed extract, vitamin C and its derivatives or vitamin E and its derivatives.

6. The said composition in Claim 1 or 5 does not include a panax ginseng component, wherein the forsythia component is forsythin and its derivatives, or the composition of forsythin and phillygenin.

7. The said composition in Claim 6, wherein the forsythin derivatives include 33-hydroxy phillygenin-8-O-β-D-glucuronide, 9-hydroxy phillygenin-8-O-β-D-glucuronide, 33, 34-methylenedioxy phillygenin-8-O-β-D-glucuronide, (2R,3R,4R,5S)-methyl 6-(5-((1R,4S)-4-(3,4-dimethoxyphenyl) hexahydrofuro[3,4-c] furan-1-yl)-2-methoxyphenoxy)-3,4,5-trihydroxyte trahydro-2H-pyran-2-carboxylate, sodium (2R,3R,4R,5S)-6-(5- ((1R,4S) -4- (3,4-dimethoxyphenyl) hexahydrofuro [3,4-c]furan-1-yl) -2-methoxyphenoxy)-3,4,5-trihydroxytetrahydro -2H - pyran-2-carboxylate, potassium (2R,3R,4R,5S)-6-(5-((1R,4S)-4- (3,4-dimethoxyphenyl) hexahydrofuro[3,4-c]furan-1-yl)-2-methoxyphenoxy)-3,4,5-trihydroxytetrahydro- 2H- pyran-2-carboxylate or (2R,3R, 4R,5S)-6-(5-((1R,4S)-4-(3,4-dimethoxyphenyl) hexahydrofuro [3,4-c]furan-1-yl)-2-methoxyphenoxy)-3,4,5 -trihydroxytetrahydro-2H-pyran-2-carboxylic acid; preferably 33-hydroxy phillygenin-8-O-β-D-glucuronide, 9-hydroxy phillygenin-8-O-β-D-glucuronide, 33, 34-methylenedioxy phillygenin-8-O-β-D-glucuronide, (2R,3R,4R,5 S)-methyl 6-(5-((1R,4S)-4-(3,4-dimethoxyphenyl) hexahydrofuro [3,4-c] furan-1-yl)-2-methoxyphenoxy)-3,4,5 -trihydroxyte trahydro-2H-pyran-2-carboxylate, sodium

(2R,3R,4R,5S)-6-(5- ((1R,4S) -4- (3,4-dimethoxyphenyl) hexahydrofuro [3,4-c]furan-1-yl) -2-methoxyphenoxy)-3,4,5-trihydroxytetrahydro -2H - pyran-2-carboxylate and potassium (2R,3R,4R,5S)-6-(5-((1R,4S)-4- (3,4-dimethoxyphenyl) hexahydrofuro [3,4-c]furan-1-yl)-2-methoxyphenoxy)-3,4,5 -trihydroxytetrahydro- 2H- pyran-2-carboxylate.

**8.** The said composition in Claim 6, wherein the composition of forsythin and phillygenin consists of forsythin and phillygenin, the weight ratio of forsythin to phillygenin is 2-99.99: 0.01-98, preferably at 80-99:1-20.

**9.** The preparation for achieving the antiviral, antipyretic, anti-inflammatory and/or immunity-boosting effects comprise a said composition in one of the Claims 1-8 and excipients that are acceptable in the industries of pharmaceutics, health products or foods.

**10.** In the said preparation in Claim 9, the weight ratio of the said composition in one of the Claims 1-8 and the carrier acceptable in the industries of pharmaceutics, health products or foods is 1:1 to 1:100, or the weight ratio of the said composition in one of Claims 6-8 and the carrier acceptable in the industries of pharmaceutics, health products or foods is 0.01-50:100.

**11.** In the said preparation in Claim 9, the carrier acceptable in the industries of pharmaceutics, health products or foods is a cyclodextrin, such as $\alpha$-, $\beta$-or $\gamma$-cyclodextrin or its derivatives.

**12.** The said preparation in Claim 9 is a drug product, which may exist in the preferable dosage forms of tablets, capsules, pills, powder, granules, syrup, solution, emulsion, injection, spray, aerosol, gel, cream, cataplasm, adhesive plaster or emplastrum.

**13.** In the said preparation in Claim 9, wherein the preparation is a health product or food, which may exist in the preferable dosage forms of tablets, capsules, pills, powder, granules, syrup, solution, emulsion, spray, aerosol, gel, cream, cataplasm, adhesive plaster or emplastrums, or in the forms of foods such as dairy products, confectionery, beverages, biscuits, tea leaves and related products, wine and the like.

**14.** The method for preparing a said composition in one of Claims 1-5 comprises a step of blending the panax ginseng component and forsythia suspensa component.

**15.** The method for preparing a said composition in one of Claims 1-5 comprises a step of using the solvent heating extraction method to extract the panax ginseng and forsythia suspensa and optional Chinese medicinal materials.

**16.** The method for preparing a said composition in one of Claims 9-13 comprises a step of blending the said composition in one of the Claims 1-8 and a carrier acceptable in the industries of pharmaceutics, health products or foods.

**17.** The method for preparing a said composition in Claim 16 comprises a step of blending the said composition in one of the Claims 1-8 with a cyclodextrin or its derivative (e.g., $\alpha$-, $\beta$-or $\gamma$-cyclodextrin or a derivative thereof).

**18.** The method for preparing a said composition in Claim 16 comprises a step of using physical or chemical methods to treat a said composition in Claims 1-8 with a cyclodextrin or a derivative thereof (e.g., $\alpha$-, $\beta$-or $\gamma$-cyclodextrin or a derivative thereof) to form a complex.

**19.** The use of a composition in one of the Claims 1-8 in manufacturing drugs, health products or foods for achieving the antiviral, antipyretic, anti-inflammatory and/or immunity-boosting effects.

**20.** The method for achieving the antiviral, antipyretic, anti-inflammatory and/or immunity-boosting effects comprises administering an effective amount of a said composition in one of the Claims 1-8 to an individual who need the said composition.

**21.** The use of a said composition in one of the Claims 6-8 in the manufacturing of drugs, health products or foods for the anti-cytomegalovirus effects or the prevention or treatment of disorders caused by cytomegalovirus infection.

**22.** The method for achieving the anti-cytomegalovirus effect or for the prevention or treatment of disorders caused by cytomegalovirus infection comprises administering an effective amount of a said composition in one of the Claims 6-8 to an individual who need the said composition.

**23.** In the said application in Claim 21 or the said method in Claim 22, the said cytomegalovirus is human cytomegalovirus.

**24.** In the said application in Claim 21 or the said method in Claim 22, the said condition comprises a simple human cytomegalovirus infection condition, such as simple human cytomegalovirus infection or patients infected by human cytomegalovirus during the treatment of cardiovascular and cerebrovascular diseases, tumors, burns or AIDS or during the process of organ transplantation or during the perinatal period.

## INTERNATIONAL SEARCH REPORT

International application No.
**PCT/CN2021/115584**

**A. CLASSIFICATION OF SUBJECT MATTER**

A61K 36/634(2006.01)i; A61K 36/25(2006.01)i; A61P 31/12(2006.01)i; A61P 29/00(2006.01)i; A61P 37/04(2006.01)i

According to International Patent Classification (IPC) or to both national classification and IPC

**B. FIELDS SEARCHED**

Minimum documentation searched (classification system followed by classification symbols)

A61K, A61P

Documentation searched other than minimum documentation to the extent that such documents are included in the fields searched

Electronic data base consulted during the international search (name of data base and, where practicable, search terms used)

CNABS, CNTXT, CNKI, DWPI, VEN, 中国药物专利数据库, E药全库, 读秀, 百度学术搜索: 连翘, 连翘苷, 连翘脂素, 人参, 抗病毒, 巨细胞病毒, 免疫力, 抗炎, 环糊精, fructus forsythiae, forsythin, phillyrin, forsythiaside, phillygenin, ginseng, antiviral, cytomegalovirus, immunity, anti-inflammatory, cyclodextrin

**C. DOCUMENTS CONSIDERED TO BE RELEVANT**

| Category* | Citation of document, with indication, where appropriate, of the relevant passages | Relevant to claim No. |
|---|---|---|
| X | CN 105796861 A (FU, Li) 27 July 2016 (2016-07-27)<br>claims 1-10, description, paragraphs 9-33, 64-67 | 1-20 |
| X | CN 106063797 A (FU, Li) 02 November 2016 (2016-11-02)<br>claims 1-10, description, paragraphs 12-36, 59-62 | 1-20 |
| X | CN 108066350 A (FU, Li) 25 May 2018 (2018-05-25)<br>claims 1-10, description, paragraphs 9-33, 65-68 | 1-20 |
| X | CN 106063790 A (FU, Li) 02 November 2016 (2016-11-02)<br>claims 1-10, description, paragraphs 13-37, 133-136 | 1-20 |
| X | CN 106063794 A (FU, Li) 02 November 2016 (2016-11-02)<br>claims 1-10, description, paragraphs 13-45, 208-211 | 1-20 |
| Y | 张丹丹 等 (ZHANG, Dandan et al.). "连翘及其主要有效成分槲皮素体外抗人巨细胞病毒的实验研究 (An In Vitro Study of Anti-human Cytomegalovirus Effect of Forsythia Suspensa and Its Main Active Ingredient Quercetin)"<br>*中国中药杂志 (China Journal of Chinese Materia Medica),*<br>Vol. 35, No. 8, 30 April 2010 (2010-04-30),<br>pp. 1055-1059 | 21-24 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 November 2021** | **01 December 2021** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2021/115584**

**C. DOCUMENTS CONSIDERED TO BE RELEVANT**

| Category* | Citation of document, with indication, where appropriate, of the relevant passages | Relevant to claim No. |
|---|---|---|
| Y | GR 1002799 B (AXARLIS STAVROS et al.) 04 November 1997 (1997-11-04) abstract | 21-24 |
| Y | CN 105796861 A (FU, Li) 27 July 2016 (2016-07-27) claims 1-10, description, paragraphs 9-33, 64-67 | 21-24 |

Form PCT/ISA/210 (second sheet) (January 2015)

**INTERNATIONAL SEARCH REPORT**

International application No.
**PCT/CN2021/115584**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **20、22-24**
because they relate to subject matter not required to be searched by this Authority, namely:

[1] Claims 20 and 22-24 relate to a method for treating diseases, do not comply with PCT Rule 39.1(iv), and thus the search or written opinion is provided on the basis of the claims being reasonably expected to be corresponding pharmaceutical uses.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/115584**

| Patent document cited in search report | | | Publication date (day/month/year) | Patent family member(s) | | | Publication date (day/month/year) |
|---|---|---|---|---|---|---|---|
| CN | 105796861 | A | 27 July 2016 | CN | 105796861 | B | 11 February 2020 |
| CN | 106063797 | A | 02 November 2016 | | None | | |
| CN | 108066350 | A | 25 May 2018 | CN | 108066350 | B | 26 June 2020 |
| CN | 106063790 | A | 02 November 2016 | | None | | |
| CN | 106063794 | A | 02 November 2016 | CN | 106063794 | B | 30 July 2019 |
| GR | 1002799 | B | 04 November 1997 | | None | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 201510319303 A
- WO 201510164294 A
- WO 201510320579 A
- WO 201410825547 A


### Non-patent literature cited in the description


- Handbook of Pharmaceutical Excipients. American Pharmaceutical Association, Washington and The Pharmaceutical Press, 1994
- Appendix VI. Chinese Pharmacopoeia. 2010, vol. I